# FASCICULE DE BREVET EUROPEEN

(11) **EP 0 776 325 B1**
(45) Date de publication et mention de la délivrance du brevet: **20.11.2002**
(21) Numéro de dépôt: 95927004.2
(22) Date de dépôt: 01.08.1995
(51) Int. Cl.: C07D 405/06, A61K 31/415

(54) **NOUVELLE UTILISATION ET NOUVEAUX DERIVES DE L'IMIDAZOLE, LEUR PROCEDE DE PREPARATION, LES NOUVEAUX INTERMEDIAIRES OBTENUS, LEUR APPLICATION A TITRE DE MEDICAMENTS ET LES COMPOSITIONS PHARMACEUTIQUES LES RENFERMANT**
NEUE VERWENDUNG UND NEUE DERIVATE VON IMIDAZOL, IHRER HERSTELLUNG, ZWISCHENPRODUKTE, UND VERWENDUNG ALS ARZNEIMITTEL
NOVEL USE AND NOVEL DERIVATIVES OF IMIDAZOLE, METHOD FOR PREPARING SAME, NOVEL INTERMEDIATES THUS OBTAINED, MEDICINAL APPLICATIONS OF SAME AND PHARMACEUTICAL COMPOSITIONS CONTAINING SAME

(30) Priorité: 02.08.1994 FR 9409566
(43) Date de publication de la demande: 04.06.1997
(73) Titulaire: Aventis Pharma S.A., 92160 Antony (FR)
(72) Inventeur: HECKMANN, Bertrand, F-94230 Cachan (FR); VEVERT, Jean-Paul, F-93500 Pantin (FR); ZHANG, Jidong, F-75013 Paris (FR)
(86) Numéro de dépôt international: FR9501034
(87) Numéro de publication internationale: WO96004276

(56) Documents cités:
- WO-A-94/02474
- FR-A- 2 416 012
- CHEMICAL ABSTRACTS, vol. 103, no. 15, 14 Octobre 1985, Columbus, Ohio, US; abstract no. 116118u, G. STEFANCICH ET AL 'Studies on new psychotropic agents. I. Synthesis and pharmacological activity of derivatives of 5H-imidazo(2,1-c)(1,4)benzodiazepine' page 68 ; & FARMACO, EDIZIONE SCIENTIFICA, vol.40, no.6, 1985, PAVIA IT pages 429 - 441
- JOURNAL OF MEDICINAL CHEMISTRY., vol.18, no.8, 1975, WASHINGTON US pages 833 - 836 K. H. BAGGALEY ET AL 'Hypolipidemic imidazoles'

## Description

La présente invention concerne la nouvelle utilisation de dérivés de l'imidazole, des nouveaux dérivés de l'imidazole, leur procédé de préparation, les nouveaux intermédiaires obtenus, leur application à titre de médicaments et les compositions pharmaceutiques les renfermant.

La présente invention a pour objet les produits répondant à la formule (I) : 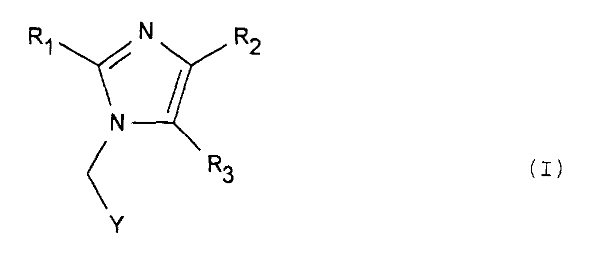 dans laquelle :
R₁ est choisi parmi les radicaux méthyle, éthyle, propyle, butyle linéaires ou ramifiés éventuellement substitués par un radical hydroxy ou alcoxy, et les radicaux alkylthio renfermant jusqu'à 6 atomes de carbone, dioxolanne et dioxane,
R₂ est choisi parmi les radicaux :
   a) 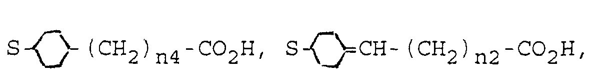 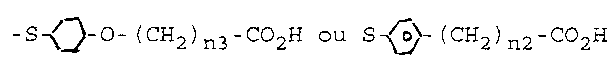 dans lequel n₄ représente le nombre 2 ou 3, n₂ représente un nombre entre 0 et 3 et n₃ représente un nombre entre 1 et 3.
   b) les radicaux alkylthio, alkénylthio, cycloalkylthio, cycloalkyle, cycloalkylalkyle, cycloalkylalkényle, phényle, phénoxy, phénylthio, benzyle, benzoyle, phénylthioalkyle, radicaux dans lesquels les radicaux alkyle et alkényle renferment au plus 4 atomes de carbone, les radicaux cycloalkyle et cycloalkényle renferment au plus 6 atomes de carbone et les radicaux alkyle, alkényle, cycloalkyle et phényle sont éventuellement substitués par un ou plusieurs radicaux choisis parmi les radicaux hydroxy, carboxy, alkyle, alkényle et alcoxy, ces trois derniers radicaux étant eux-mêmes éventuellement substitués par un radical carboxy et les radicaux phényle étant de plus éventuellement substitués sur deux carbones adjacents par un radical dioxole,
R₃ est choisi parmi les radicaux carboxy libre ou estérifié, tétrazolyle ou sulfonylurée,
Y représente un radical phényle substitué par deux radicaux formant ensemble un radical dioxole et éventuellement par un ou plusieurs radicaux choisis parmi les atomes d'halogène et les radicaux hydroxy, alcoxy et carboxy,
l'atome de soufre étant éventuellement oxydé sous forme de sulfoxyde ou de sulfone, ainsi que les produits suivants :
   - acide 2-butyl 1-((6-chloro 1,3-benzodioxol-5-yl) méthyl) 4-((4-méthoxyphényl) méthylthio) 1H-imidazole 5-carboxylique
   - acide 1-((6-chloro 1,3-benzodioxol-5-yl) méthyl) 2,4-bis (((4-méthoxyphényl) méthyl) thio) 1H-imidazole 5-carboxylique
   - acide 1-((6-chloro 1,3-benzodioxol-5-yl) méthyl) 4-(2-phényléthyl) 2-propyl 1H-imidazol-5-carboxylique,
lesdits produits de formule (I) et les produits définis ci-dessus étant sous toutes les formes isomères possibles racémiques, énantiomères et diastéréoisomères, ainsi que les sels d'addition avec les acides minéraux et organiques ou avec les bases minérales et organiques desdits produits de formule (I) et des produits définis ci-dessus .

La présente invention a pour objet l'utilisation pour la préparation de compositions pharmaceutiques destinées au traitement d'affections résultant d'une stimulation anormale des récepteurs à l'endothéline, des produits de formule (I) telle que définie ci-dessus.

La présente invention a notamment pour objet l'utilisation des produits de formule (I) telle que définie ci-dessus, pour la préparation de compositions pharmaceutiques destinées au traitement de l'hypertension induite par l'endothéline, de tous les spasmes vasculaires et au traitement des post-hémorragies cérébrales et également pour la préparation de compositions pharmaceutiques destinées au traitement de l'infarctus du myocarde et à la prévention des resténoses post-angioplastie.

Dans les produits de formule (I) et dans ce qui suit :
- le terme radical alkyle linéaire ou ramifié désigne de préférence les radicaux méthyle, éthyle, propyle, isopropyle, butyle, isobutyle, sec-butyle et tert-butyle mais peut également représenter un radical pentyle ou hexyle et particulièrement isopentyle et isohexyle,
- le terme radical alkényle linéaire ou ramifié désigne de préférence un radical vinyle, allyle, 1-propényle, butényle et particulièrement 1-butényle, ou pentényle,
- le terme atome d'halogène désigne de préférence l'atome de chlore, mais peut aussi représenter un atome de fluor, de brome ou d'iode,
- le terme radical alcoxy linéaire ou ramifié désigne de préférence les radicaux méthoxy, éthoxy, propoxy ou isopropoxy, mais peut aussi représenter un radical butoxy linéaire, secondaire ou tertiaire,
- le terme radical acyle désigne de préférence un radical ayant de 1 à 6 atomes de carbone tel que par exemple le radical formyle, acétyle, propionyle, butyryle ou benzoyle, mais également le radical pentanoyle, hexanoyle, acryloyle, crotonoyle ou carbamoyle, ainsi que les dérivés de radicaux carbonyle tels que notamment les radicaux cycloalkylcarbonyle,
- le terme radical acyloxy désigne par exemple un radical
dans lequel le radical acyle a les valeurs indiquées ci-dessus et désigne de préférence un radical formyloxy, acétyloxy, propionyloxy, butyryloxy ou benzoyloxy,
- le terme radical cycloalkyle éventuellement interrompu par un ou plusieurs hétéroatomes désigne de préférence les radicaux cyclopropyle, cyclobutyle et tout particulièrement les radicaux cyclopentyle et cyclohexyle ou bien un radical carbocyclique interrompu par un ou plusieurs atomes choisis parmi les atomes d'oxygène, d'azote ou de soufre et tout particulièrement un radical dioxolane ou dioxane
- le terme radical alkylthio désigne de préférence les radicaux dans lesquels le radical alkyle est tel que défini ci-dessus comme par exemple dans méthylthio, éthylthio, propylthio, isoprcpylthio, butylthio, sec-butylthio, tert-butylthio, isopentylthio ou isohexylthio, éventuellement substitué comme par exemple dans hydroxyméthylthio ou aminoéthylthio,
- le radical phénylalkylthio ou alkylthio substitué par phényle représente par exemple le radical benzylthio ou phénéthylthio.
- les termes radical alkylsulfinyle et alkylsulfonyle désignent les radicaux alkylthio dans lesquels le radical alkyle linéaire ou ramifié peut représenter, par exemple, les valeurs indiquées ci-dessus pour le radical alkyle et dans lesquels le radical thio est oxydé en radical sulfinyle ou sulfonyle. On peut citer par exemple les radicaux méthylsulfinyle, éthylsulfinyle, méthylsulfonyle ou éthylsulfonyle,

Les radicaux carbamoyle et amino que peuvent représenter ou porter l'un ou plusieurs des éventuels substituants des radicaux définis dans les produits de formule (I) et dans ce qui suit, désignent des radicaux dans lesquels à l'atome d'azote sont liés deux radicaux, identiques ou différents, choisis parmi l'atome d'hydrogène pour donner le radical amino ; les radicaux alkyle tels que définis ci-dessus pour donner les radicaux monoalkyl- ou dialkylamino dans lesquels les radicaux alkyles linéaires ou ramifiés renferment de 1 à 6 atomes de carbone, tous ces radicaux étant éventuellement substitués ainsi qu'il est indiqué ci-dessus et ci-après.

De même, dans les produits de formule (I), les radicaux carbamoyle ou amino sont tels que les radicaux portés par l'atome d'azote sont identiques ou différents, peuvent représenter des chaînes aliphatiques ou cyclisées ou peuvent former avec l'atome d'azote auquel ils sont liés un hétérocycle.

Les radicaux amino éventuellement substitué et carbamoyle éventuellement substitué désignent respectivement les radicaux dans lesquels l'un ou les deux atomes d'hydrogène liés à l'atome d'azote peut être substitué par un ou deux radicaux choisis parmi les radicaux tels que définis précédemment.

Le radical amino éventuellement substitué désigne ainsi le radical amino éventuellement substitué par un ou deux radicaux alkyle choisis parmi les radicaux alkyle tels que définis ci-dessus comme par exemple pour monoalkylamino dans méthylamino ou éthylamino ou isopropylamino ou par exemple pour dialkylamino dans diméthylamino, diéthylamino ou encore méthyléthylamino, ces radicaux alkyles étant éventuellement substitués ainsi qu'il est indiqué ci-dessus, comme par exemple les radicaux méthoxyméthyle, méthoxyéthyle, éthoxyéthyle.

A titre d'exemple et de façon non exhaustive, le terme radical carbamoyle éventuellement substitué désigne les radicaux carbamoyle éventuellement substitué sur l'atome d'azote par un ou deux radicaux alkyle éventuellement substitués ainsi qu'il est défini ci-dessus, pour former notamment un groupe N-monoalkyl carbamoyle tel que N-méthylcarbamoyle, N-éthylcarbamoyle ou un groupe N,N-dialkyl carbamoyle, tel que N,N-diméthylcarbamoyle, N,N-diéthylcarbamoyle ; un groupe N-(hydroxyalkyl carbamoyle, tel que N-(hydroxyméthyl) carbamoyle, N-(hydroxyéthyl) carbamoyle, phénylcarbamoyle ; pyridylcarbamoyle ; benzylcarbamoyle ; N-méthyl N-phénylcarbamoyle ; pyridylméthylcarbamoyle. Par ailleurs, parmi les radicaux alkyles substitués, on peut citer également les radicaux alkyles substitués par un radical carbamoyle tel que défini ci-dessus, pour former un groupe carbamoylalkyle tel que carbamoylméthyle ou carbamoyléthyle.

Le radical amino peut être un radical alcoxycarbonylamino, ce radical étant alors de préférence le radical tert-butyloxycarbonylamino ou le radical benzyloxycarbonylamino.

Les radicaux amino et carbamoyle peuvent encore notamment être substitués par un ou deux acides aminés choisis parmi les 20 acides aminés naturels tels que notamment la proline ou par exemple la glycine, l'alanine, la leucine, l'isoleucine, la valine ou la phénylalanine ou l'un des autres acides aminés naturels connus de l'homme de métier.

Le ou les radicaux carboxy des produits de formule (I) peuvent être salifiés ou estérifiés par les groupements divers connus de l'homme du métier parmi lesquels on peut citer, par exemple :
- parmi les composés de salification, des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine,
- parmi les composés d'estérification, les radicaux alkyle pour former des groupes alcoxy carbonyle tel que, par exemple, méthoxycarbonyle, éthoxycarbonyle, tert-butoxycarbonyle ou benzyloxycarbonyle, ces radicaux alkyles pouvant être substitués par des radicaux choisis par exemple parmi les atomes d'halogène, les radicaux hydroxyle, alcoxy, acyle, acyloxy, alkylthio, amino ou aryle comme, par exemple, dans les groupements chlorométhyle, hydroxypropyle, méthoxyméthyle, propionyloxyméthyle, méthylthiométhyle, diméthylaminoéthyle, benzyle ou phénéthyle.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

Lorsque R₂ et R₃ représentent tous deux un groupement soufré, R₂ et R₃ étant identiques ou différents, dans les produits préférés de l'invention, ces groupements soufrés n'ont pas nécessairement le même degré d'oxydation.

L'invention a plus particulièrement pour objet les produits de formule (I) telle que définie ci-dessus, dans laquelle Y représente un radical benzofuranyl ou un radical phényle substitué par deux radicaux formant ensemble un radical dioxol et éventuellement substitué par un ou plusieurs substituants choisis parmi les valeurs de R₂ et R₃, telles que définies ci-dessus, lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

Parmi les produits objet de l'invention, peuvent être cités tout particulièrement les produits de formule (I) telle que définie ci-dessus, répondant aux formules suivantes :
- acide 1-((6-chloro 1,3-benzodioxol-5-yl) méthyl) 4-((4-méthoxyphényl) méthylthio) 1H-imidazole 5-carboxylique
- acide 2-butyl 1-((6-chloro 1,3-benzodioxol-5-yl) méthyl) 5-((4-méthoxyphényl) thio) 1H-imidazole 4-carboxylique
- acide 1-((6-chloro 1,3-benzodioxol-5-yl) méthyl) 2,4-bis (((4-méthoxyphényl) méthyl) thio) 1H-imidazole 5-carboxylique
- acide 1-((6-chloro 1,3-benzodioxol-5-yl) méthyl) 4-(4-(méthoxyphényl) thio) 2-(propylthio) 1H-imidazole 5-carboxylique,
- acide 1-((6-chloro 1,3-benzodioxol-5-yl) méthyl) 4-((3,4-diméthoxyphényl) thio) 2-propyl 1H-imidazole 5-carboxylique,
- acide 2-butyl 1-((6-chloro 1,3-benzodioxol-5-yl) méthyl) 4-(cyclohexylthio) 1H-imidazole 5-carboxylique,
- acide 2-butyl 4-((3-carboxypropyl) thio) 1-((6-chloro 1,3-benzodioxol-5-yl) méthyl) 1H-imidazole 5-carboxylique,
- acide 4-(((4-(2-carboxyéthyl) phényl) thio) méthyl) 1-((6-chloro 1,3-benzodioxol-5-yl) méthyl) 2-propyl 1H-imidazol 5-carboxylique,
- acide 4-((3-carboxypropyl) thio) 1-((6-chloro 1,3-benzodioxol-5-yl) méthyl) 2-propyl 1H-imidazol 5-carboxylique,
- acide 4-((7-carboxyheptyl) trio) 1-((6-chloro 1,3-benzodioxol-5-yl) méthyl) 2-propyl 1H-imidazol 5-carboxylique.

Certains produits de formule (I) peuvent être préparés notamment ainsi qu'il est indiqué dans les demandes de brevet européennes EP 0465368 cu EP 0503162.

L'invention a ainsi également pour objet un procédé de préparation des produics de formule (I), celle que définie ci-dessus, caractérisé en ce que :
soit l'on soumet un composé de formule (II) : 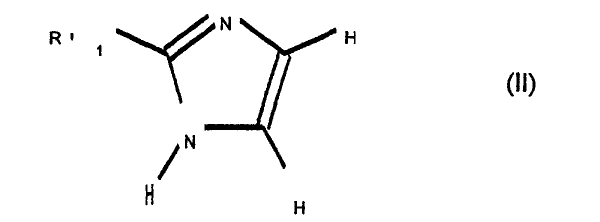 dans laquelle R'₁ a la signification indiquée ci-dessus pour R₁, dans laquelle les éventuelles fonctions réactives sont éventuellement protégées par des groupements protecteurs, à une réaction avec un composé de formule (III) : 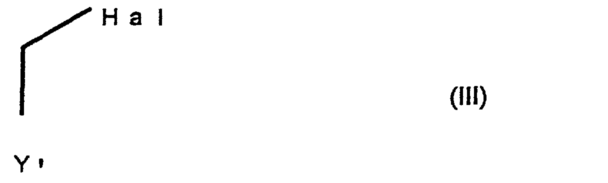 dans laquelle Hal représente un atome d'halogène, et
   Y' a la signification indiquée ci-dessus pour Y, dans laquelle les éventuelles fonctions réactives sont éventuellement protégées par des groupements protecteurs,
   pour obtenir le produit de formule (IV₁) : 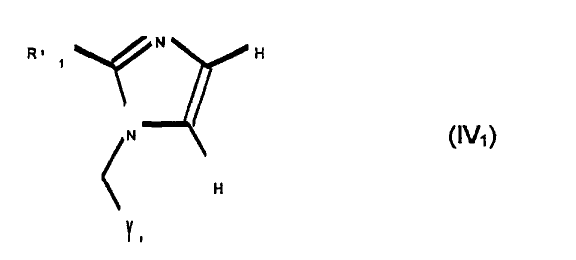 dans laquelle R'₁ et Y' ont les significations indiquées ci-dessus, produit de formule (IV₁) que l'on peut soumettre à une réaction d'halogénation, pour obtenir le produit de formule (IV₂) : 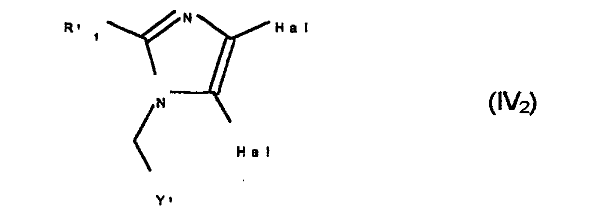 dans laquelle R'₁, Hal et Y' ont les significations indiquées ci-dessus,
soit l'on soumet un composé de formule (VIII) : 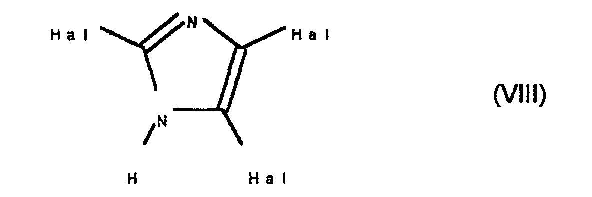 dans laquelle Hal a la signification indiquée ci-dessus, soit à une réaction avec le composé de formule (III) telle que définie ci-dessus, soit à l'action d'un groupement protecteur P, pour obtenir un produit de formule (IX) : 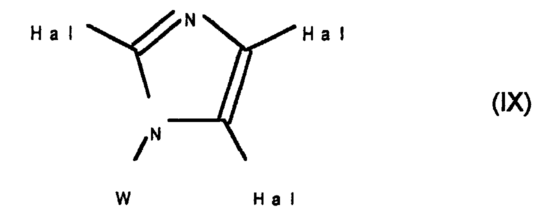 dans laquelle Hal a la signification indiquée ci-dessus et W représente soit -CH₂-Y' avec Y' tel que défini ci-dessus, soit P qui représente un groupement protecteur de l'atome d'azote, produit de formule (IX) que l'on soumet à une réaction d'échange halogène-métal puis à une réaction avec un électrophile de formule (X), (X'), (XI), (XII) ou (XII') :

   L₁-CHO (X)

   L₁-CHO-Cl (X')

   (-S-L₁)₂ (XI)

   L₁SO₂-S-L'₁ (XII)

   L₁SO₂-Cl (XII')

   dans lesquelles L₁ et L'₁ identiques ou différents, représentent un radical alkyle ou alkényle, tels que définis ci-dessus, pour obtenir un produit de formule (XIII) : 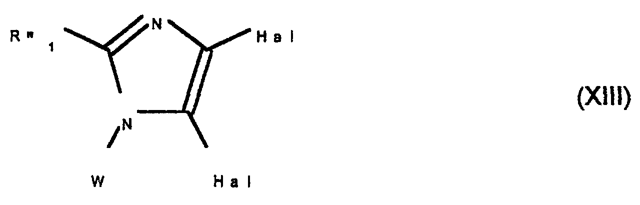 dans laquelle Hal et W ont les significations indiquées ci-dessus, R"₁ représente un radical alkyl-carbonyle, alkyl-hydroxyalkyle ou alkylthio dans lesquels le radical alkyle a la signification indiquée ci-dessus et dans lesquels les éventuelles fonctions réactives sont éventuellement protégées par des groupements protecteurs, produits de formules (IV₂) et (XIII) que l'on peut soumettre à une réaction d'échange halogène-métal sur l'un des atomes d'halogène puis à une réaction avec CO₂ ou DMF ou un composé électrophile de formule (Vₐ), (V_{b}), (VIₐ), (VI_{b}) ou (VI_{c}) :

   (-S-Z₁)₂ (Vₐ) ou MeSO₂SZ₁ (V_{b})

   ou 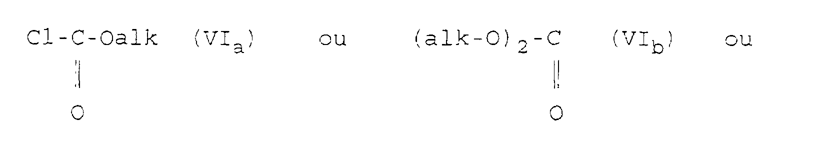 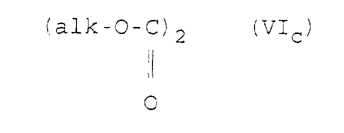 dans lesquelles Z₁ représente un radical alkyle, alkényle ou phényle, éventuellement substitués, dans lesquelles les éventuelles fonctions réactives sont éventuellement protégées par des groupements protecteurs, et alk représente un radical alkyle renfermant au plus 4 atomes de carbone,
   pour obtenir respectivement le composé de formule (I₁) : 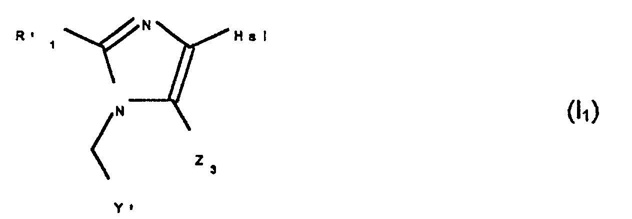 ou le composé de formule (XIV) : 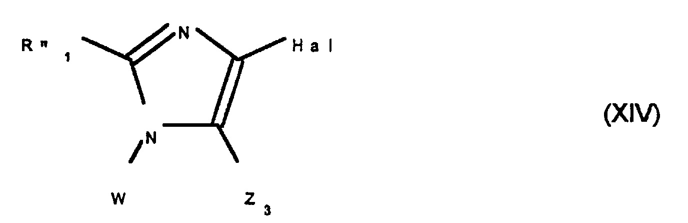 dans lesquelles R'₁, R"₁, Hal, Y' et W ont les significations indiquées ci-dessus et Z₃ représente le radical carboxy, formyle, S-Z₁ tel que défini ci-dessus, ou le radical K-O-alk dans lequel K représence le radical 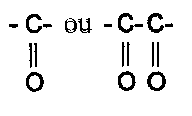 et alk a la signification indiquée ci-dessus,
   composés de formule (I₁) ou (XIV) que lorsque Z₃ représente un radical formyle ou carboxy estérifié, l'on peut soumettre à une réaction avec un composé de formule (VII) :

   Z₂-S-M (VII)

   dans laquelle S représente un atome de soufre, M représente un métal tel que sodium, potassium ou cuivre et Z₂ représente un radical alkyle, alkényle ou phényle, éventuellement substitué, dans lesquelles les éventuelles fonctions réactives sont éventuellement protégées par des groupements protecteurs pour obtenir respectivement le composé de formule (I₂) : 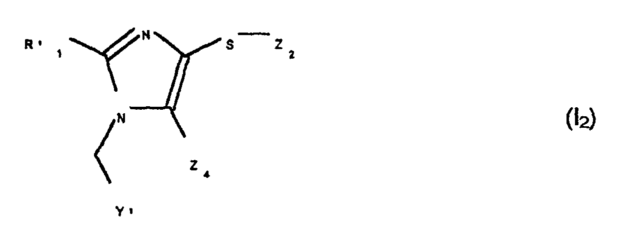 ou le composé de formule (XV) : 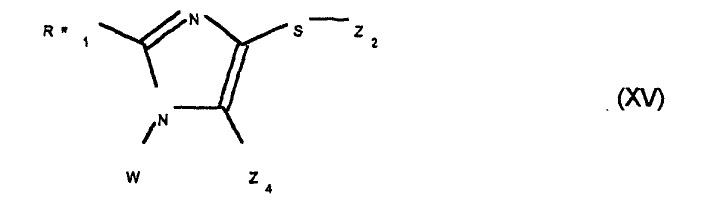 dans lesquelles R'₁, R"₁, Y', Z₂ et W ont les significations indiquées ci-dessus, et Z₄ représente le radical formyle ou carboxy estérifié, produits de formules (I₂) et (XV) que lorsque Z₄ représente le radical formyle, l'on peut soumettre à une réaction d'oxydation ou de réduction pour obtenir respectivement un produit de formule (I₃) : 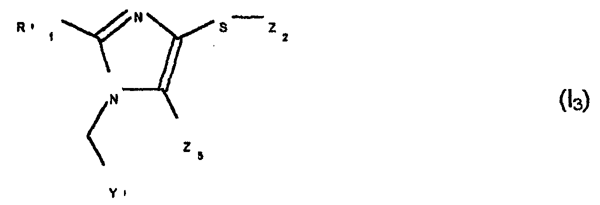 ou un produit de formule (I₄) : 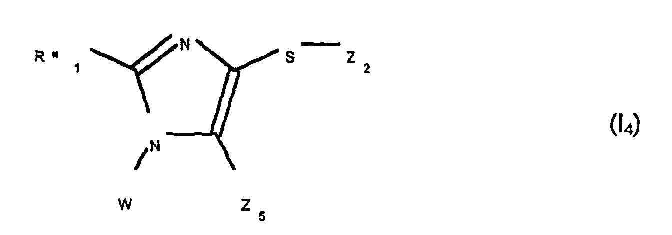 dans lesquelles R'₁, R"₁, Z₂, Y' et W ont les significations indiquées ci-dessus, et Z₅ représente le radical CH₂OH ou le radical carboxy libre ou estérifié par un radical alkyle linéaire ou ramifié renfermant au plus 6 atomes de carbone, produits de formule (XV) ou (I₄) que, dans le cas où W représente P tel que défini ci-dessus et après libération de la fonction amine bloquée par P tel que défini ci-dessus, l'on fait réagir avec le composé de formule (III) telle que définie ci-dessus, pour obtenir un produit de formule (I₅) : 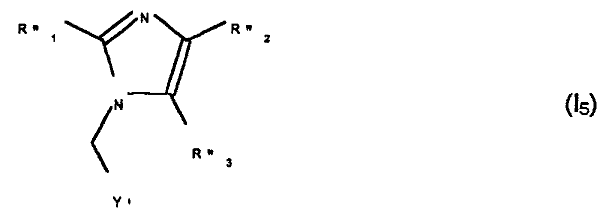 dans laquelle R"₁ et Y' ont les significations indiquées ci-dessus, et R"₂ et R"₃ représentent indifféremment l'un Z₄ ou Z₅ tels que définis ci-dessus et l'autre S-Z₂ tel que défini ci-dessus,
soit l'on soumet un composé de formule (XVI) 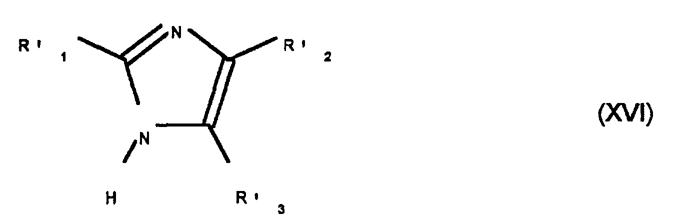 dans laquelle R'₁ a la signification indiquée ci-dessus et R'₂ et R'₃ ont les significations indiquées ci-dessus respectivement pour R₂ et R₃ dans lesquelles les éventuelles fonctions réactives sont éventuellement protégées par des groupements protecteurs, à une réaction avec le composé de formule (III) telle que définie ci-dessus, pour obtenir un produit de formule (I') : 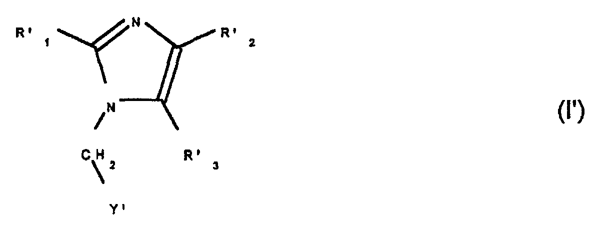 dans laquelle R'₁, R'₂, R'₃ et Y' ont les définitions indiquées ci-dessus,
soit l'on soumet un composé de formule (XVII) : 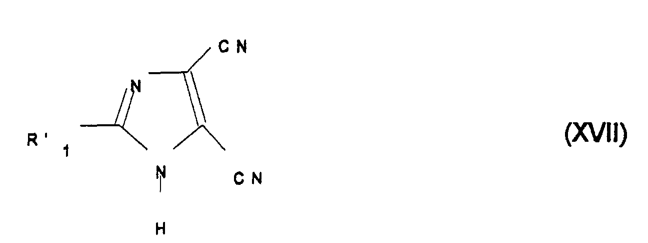 dans laquelle R'₁ a la signification indiquée précédemment, à une réaction d'oxydation pour obtenir le produit de formule (XVIII) : 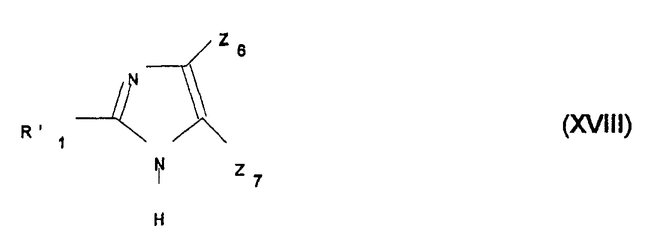 dans laquelle R'₁ a la signification indiquée précédemment et l'un ou les deux de Z₆ et Z₇ représentent un radical carboxy libre ou estérifié et le cas échéant, l'autre de Z₆ et Z₇ conservant la valeur CN, produit de formule (XVIII) que l'on fait réagir avec le composé de formule (III) telle que définie ci-dessus pour obtenir le produit de formule (I₆) : 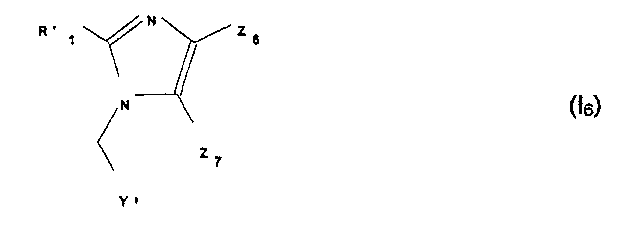 dans laquelle R'₁, Y', Z₆ et Z₇ ont les significations indiquées précédemment,
   produits de formules (I₁), (I₂), (I₃), (I₄), (XIV), (XV), (I₅), (I₆) et (I') qui peuvent être des produits de formule (I) et que, pour obtenir des ou d'autres produits de formule (I), l'on peut soumettre, si désiré et si nécessaire, à l'une ou plusieurs des réactions de transformations suivantes, dans un ordre quelconque :
   a) une réaction d'estérification de fonction acide,
   b) une réaction de saponification de fonction ester en fonction acide,
   c) une réaction de transformation de fonction ester en fonction acyle,
   d) une réaction de transformation de la fonction cyano en fonction acide,
   e) une réaction de transformation de fonction acide en fonction amide, puis éventuellement en fonction thioamide,
   f) une réaction de réduction de la fonction carboxy en fonction alcool,
   g) une réaction de transformation de fonction alcoxy en fonction hydroxyle, ou encore de fonction hydroxyle en fonction alcoxy,
   h) une réaction d'oxydation de fonction alcool en fonction aldéhyde, acide ou cétone,
   i) une réaction de transformation du radical formyle en radical carbamoyle,
   j) une réaction de transformation du radical carbamoyle en radical nitrile,
   k) une réaction de transformation de radical nitrile en tétrazolyle,
   l) une réaction d'oxydation de groupement alkylthio ou arylthio en sulfoxyde ou sulfone correspondant,
   m) une réaction de transformation de fonction sulfure, sulfoxyde ou sulfone en fonction sulfoximine correspondante,
   n) une réaction de transformation de fonction oxo en fonction thioxo,
   o) une réaction de transformation du radical 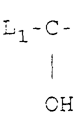 en radical 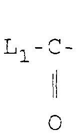 puis si nécessaire de nouveau en radical 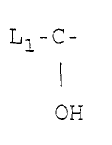 avec L₁ tel que défini ci-dessus,
   p) une réaction de transformation de fonction acide en fonction 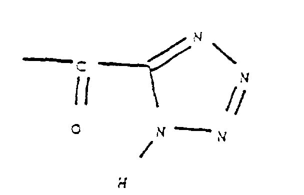
   q) une réaction de transformation de la fonction β-cétosulfoxyde en fonction α-céto thio ester,
   r) une réaction de transformation d'un carbamate en urée et notamment d'un sulfonylcarbamate en sulfonylurée,
   s) une réaction de transformation d'une fonction halogénée en fonction formyle ou carboxy estérifié,
   t) une réaction de transformation d'un radical formyle en fonction CH₂-CO₂alk ou CH=CH-CO₂alk dans laquelle alk représente un radical alkyle renfermant de 1 à 4 atomes de carbone, puis le cas échéant, transformation en acide correspondant,
   u) une réaction de transformation d'un radical formyle en radical -CH=CH- puis le cas échéant en -CH₂-CH₂- et -CH₂-CH₂- ,
   v) une réaction de transformation d'un radical formyle en radical CH₂-OH ou 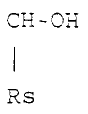 puis le cas échéant en CH₂-Rs ou transformation de 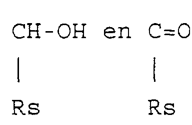 pour obtenir les radicaux alkyle ou acyle éventuellement substitués tels que définis ci-dessus pour R'₂ et R'₃,
   w) une réaction d'oxydation du radical S-alk en 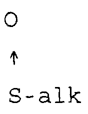 puis transformation en fonction SH et le cas échéant en S-Z₂ dans lequel alk et Z₂ ont la signification indiquée précédemment,
   x) une réaction d'élimination des groupements protecteurs que peuvent porter les fonctions réactives protégées,
   y) une réaction de salification par un acide minéral ou organique ou par une base pour obtenir le sel correspondant,
   z) une réaction de dédoublement des formes racémiques en produits dédoublés,
lesdits produits de formule (I) ainsi obtenus étant sous toutes les formes isomères possibles racémiques, énantiomères et diastéréoisomères.

On peut noter que de telles réactions de transformation de substituants en d'autres substituants peuvent également être effectuées sur les produits de départ ainsi que sur les intermédiaires tels que définis ci-dessus avant de poursuivre la synthèse selon les réactions indiquées dans le procédé décrit ci-dessus.

Dans des conditions préférentielles de mise en oeuvre de l'invention, le procédé décrit ci-dessus peut-être réalisé de la façon suivante. Dans le produit de formule (III), l'atome d'halogène représente de préférence un atome de brome mais peut également représenter un atome de chlore ou d'iode. La réaction de condensation des imidazoles de formules telles que définies ci-dessus (II), (VIII), (XVI), (XV) et (I₄) (dans le cas des produits de formules (XV) et (I₄), lorsque W représente P et après déprotection de l'atome d'azote), avec le composé de formule (III) telle que définie ci-dessus, pour obtenir respectivement les produits de formules (IV₁), (IX) lorsque W représente Y', (I₅) et (I') telles que définies ci-dessus peut être réalisée dans un solvant tel que par exemple le diméthylformamide ou encore le diméthylacétamide, le tétrahydrofuranne, le diméthoxyéthane ou le diméthylsulfoxyde au reflux du solvant ou à la température ambiante, de préférence sous agitation ; la réaction est réalisée de préférence en présence d'une base telle que par exemple l'hydrure de sodium ou de potassium ou encore du carbonate de sodium ou de potassium, du méthylate ou éthylate ou tert-butylate de sodium ou de potassium.

La réaction d'halogénation du composé de formule (IV₁) telle que définie ci-dessus en composé de formule (IV₂) telle que définie ci-dessus, peut être réalisée dans les conditions usuelles de l'homme du métier et notamment par bromation à l'aide de NBS dans CH₂Cl₂ ou encore Br₂ dans l'acide acétique.

Les composés de formules (IV₂), (IX) et (XIII) telles que définies ci-dessus peuvent être soumis à une réaction d'échange halogène métal sur l'atome d'halogène par réaction avec un composé organo métallique tel que le nBuli ou EtMgBr dans un solvant tel que le THF à une température d'environ - 78°C pour le Buli et température ambiante pour EtMgBr.

Les réactions de carboxylation par CO₂ et de formylation par DMF des composés de formules (IV2) et (XIII) respectivement en composés de formules coll et (XIV) peuvent être réalisées selon les conditions usuelles de l'homme de métier soit par exemple dans le tétrahydrofuranne à température ambiante.

Z₁ et Z₂, identiques ou différents, représentent un radical alkyle, alkényle ou aryle de telle manière que Z₁-S- et Z₂-S- représentent les valeurs correspondantes définies ci-dessus par R₂ et R₃ dans lesquelles les éventuelles fonctions réactives sont éventuellement protégées par des groupements protecteurs.

L₁ et L₁,, identiques ou différents, représentent un radical alkyle ou alkényle de telle manière que R_{1"} représente les valeurs correspondantes choisies parmi les valeurs de R₁ telles que définies ci-dessus dans lesquelles les éventuelles fonctions réactives sont éventuellement protégées par des groupements protecteurs.

La réaction du composé de formule (IV₂) ou (XIII) telles que définies ci-dessus avec le composé de formule (VIₐ), (VI_{b}) ou (VI_{c}), telles que définies ci-dessus, pour obtenir le composé correspondant de formule respective (I₁) ou (XIV) telles que définies ci-dessus peut être réalisée d'une manière identique en utilisant le EtMgBr comme agent de métallation dans le tétrahydrofuranne à température ambiante.

La réaction des composés de formules (IV₂) et (XIII) avec les composés de formule (Vₐ) ou (V_{b}) peuvent être réalisées selon les conditions usuelles de l'homme du métier soit par exemple dans le tétrahydrofuranne à température ambiante.

La réaction du composé de formule (IX) avec les composés de formules (X), (XI), (XII) et (XII') peut être réalisée selon les conditions usuelles de l'homme de métier soit par exemple dans le tétrahydrofuranne à température ambiante.

La fonction amine des composés de formules (XV) et (I₄) telle que définie ci-dessus, protégée par P tel que défini ci-dessus, peut être libérée dans les conditions usuelles connues de l'homme du métier et notamment lorsque P représente le radical -CH₂-O-(CH₂)₂-Si(CH₃)₃, l'atome d'hydrogène peut être libéré dans du TFA ou encore en présence d'ion fluorure.

La réaction de saponification peut être réalisée selon les méthodes usuelles connues de l'homme du métier, telles que par exemple dans un solvant tel que le méthanol ou l'éthanol, le dioxane ou le diméthoxyéthane, en présence de soude ou de potasse ou encore de carbonate de cesium.

Les réactions de réduction ou oxydation des produits de formules (I₂) et (XV) respectivement en produits de formules (I₃) et (I₄) peuvent être réalisées selon les méthodes usuelles connues de l'homme du métier.

Selon les valeurs de R'₁, R"₁, R'₂, R"₂, R'₃, R"₃, les produits de formules (I₁), (I₂), (I₃), (I₄), (I₅), (I₆), (XIV), (IV₂), (XV) et (I') constituent ou non des produits de formule (I) et peuvent donner des produits de formule (I), ou être transformés en d'autres produits de formule (I) en étant soumis à une ou plusieurs des réactions a) à z) indiquées ci-dessus.

Ainsi les diverses fonctions réactives que peuvent porter certains composés des réactions définies ci-dessus peuvent, si nécessaire, être protégées : il s'agit par exemple des radicaux hydroxyle, acyle, carboxy libres ou encore amino et monoalkylamino qui peuvent être protégés par les groupements protecteurs appropriés.

La liste suivante, non exhaustive, d'exemples de protection de fonctions réactives peut être citée :
- les groupements hydroxyle peuvent être protégés par exemple par les radicaux alkyle tels que tert-butyle, triméthylsilyle, tert-butyldiméthylsilyle, méthoxyméthyle, tétrahydropyrannyle, benzyle ou acétyle,
- les groupements amino peuvent être protégés par exemple par les radicaux acétyle, trityle, benzyle, tert-butoxycarbonyle, phtalimido ou d'autres radicaux connus dans la chimie des peptides,
- les groupements acyles tel que le groupement formyle peuvent être protégés par exemple sous forme de cétals ou de thiocétals cycliques ou non cycliques tels que le diméthyl ou diéthylcétal ou l'éthylène dioxycétal, ou le diéthylthiocétal ou l'éthylènedithiocétal,
- les fonctions acide des produits décrits ci-dessus peuvent être, si désiré, amidifiées par une amine primaire ou secondaire par exemple dans du chlorure de méthylène en présence, par exemple, de chlorhydrate de 1-éthyl-3-(diméthylaminopropyl) carbodiimide à la température ambiante :
   - les fonctions acide peuvent être protégées par exemple sous forme d'esters formés avec les esters facilement clivables tels que les esters benzyliques ou ter butyliques ou des esters connus dans la chimie des peptides.

Les réactions auxquelles les produits de formules (I₁), (I₂), (I₃), (I₄), (I₅), (IV₂), (XIV), (XV) et (I'), telles que définies ci-dessus peuvent être soumis, si désiré ou si nécessaire, peuvent être réalisées, par exemple, comme indiqué ci-après.
a) Les produits décrits ci-dessus peuvent, si désiré, faire l'objet, sur les éventuelles fonctions carboxy, de réactions d'estérification qui peuvent être réalisées selon les méthodes usuelles connues de l'homme du métier.
b) Les éventuelles transformations de fonctions ester en fonction acide des produits décrits ci-dessus peuvent être, si désiré, réalisées dans les conditions usuelles connues de l'homme du métier notamment par hydrolyse acide ou alcaline par exemple par de la soude ou de la potasse en milieu alcoolique tel que, par exemple, dans du méthanol ou encore par de l'acide chlorhydrique ou sulfurique.
c) la réaction d'addition sur la fonction ester 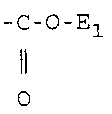 dans laquelle E₁ peut représenter un radical alkyle ou aryle éventuellement substitué et éventuellement protégé en fonction acyle 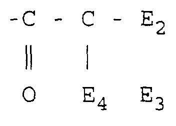 peut être réalisée notamment par action de l'anion carboné 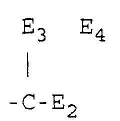 dans lequel E₂, E₃ et E₄, identiques ou différents sont choisis parmi l'atome d'hydrogène, les radicaux alkyl, alkylthioaryle, alkylsulfoxyde, arylsulfoxyde, alkylsulfone, arylsulfone, acyle, carboxy libre, salifié, estérifié ou amidifié, les radicaux alkyle, alkylthio et aryle étant éventuellement substitués et éventuellement protégés ainsi qu'il est indiqué ci-dessus.
   Une telle réaction est réalisée notamment ainsi qu'il est décrit dans la partie expérimentale, ou selon les méthodes usuelles connues de l'homme du métier.
d) Les éventuelles fonctions cyano des produits décrits ci-dessus peuvent être, si désiré, transformées en fonction acide dans les conditions usuelles connues de l'homme du métier par exemple par une double hydrolyse réalisée en milieu acide tel que par exemple dans un mélange d'acide sulfurique, d'acide acétique glacial et d'eau, ces trois composés étant de préférence en proportions égales, ou encore dans un mélange de soude, d'éthanol et d'eau au reflux.
e) la réaction de transformation de fonction acide en fonction amide peut notamment être réalisée par formation d'abord d'un chlorure d'acide selon les conditions usuelles connues de l'homme du métier et par exemple par action de SOCl₂ puis amidification ci-dessus, ou encore par amidification directe de l'acide ci-dessus.
   Notamment on peut obtenir le radical 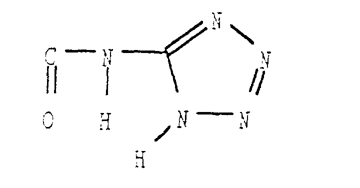 en transformant la fonction acide en chlorure d'acide, notamment par action de SOCl₂ dans un solvant tel que par exemple le toluène, ou le benzène,
   puis en faisant agir l'amine 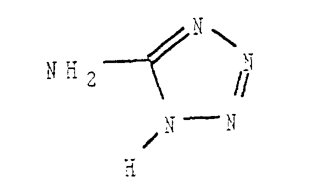
   L'amide ainsi obtenue peut alors si désiré, être transformée en thioamide par action notamment du réactif de LAWESSON dans le toluène,
f) Les éventuelles fonctions carboxy libre ou estérifié des produits décrits ci-dessus peuvent être, si désiré, réduites en fonction alcool par les méthodes connues de l'homme de métier : les éventuelles fonctions carboxy estérifié peuvent être, si désiré, réduites en fonction alcool par les méthodes connues de l'homme du métier et notamment par de l'hydrure de lithium et d'aluminium dans un solvant tel que par exemple le tétrahydrofuranne ou encore le dioxane ou l'éther éthylique.
   Les éventuelles fonctions carboxy libre des produits décrits ci-dessus peuvent être, si désiré, réduites en fonction alcool notamment par de l'hydrure de bore.
g) Les éventuelles fonctions alcoxy telles que notamment méthoxy des produits décrits ci-dessus peuvent être, si désiré, transformées en fonction hydroxyle dans les conditions usuelles connues de l'homme du métier par exemple par du tribromure de bore dans un solvant tel que par exemple le chlorure de méthylène, par du bromhydrate ou chlorhydrate de pyridine ou encore par de l'acide bromhydrique ou chlorhydrique dans de l'eau ou de l'acide trifluoro acétique au reflux.
h) Les éventuelles fonctions alcool des produits décrits ci-dessus peuvent être, si désiré, transformées en fonction aldéhyde ou acide par oxydation dans les conditions usuelles connues de l'homme du métier telles que par exemple par action de l'oxyde de manganèse pour obtenir les aldéhydes ou du réactif de Jones pour accéder aux acides.
i) j) Les réactions de transformation de radical formyle en radical carbamoyle et de radical carbamoyle en radical nitrile, sont réalisées notamment pour R₃ et R₄ selon les conditions usuelles connues de l'homme du métier, telles que par exemple passage par le céto nitrile et déplacement par une amine (Chem. Comm. 1971, p. 733).
k) Les éventuelles fonctions nitrile des produits décrits ci-dessus peuvent être, si désiré, transformées en tétrazolyle dans les conditions usuelles connues de l'homme du métier telles que par exemple par cycloaddition d'un azidure métallique tel que par exemple l'azidure de sodium ou un azidure de trialkylétain sur la fonction nitrile ainsi qu'il est indiqué dans la méthode décrite dans l'article référencé comme suit :
   J. Organometallic Chemistry., 33, 337 (1971) KOZIMA S.& coll. 1) Les éventuels groupements alkylthio ou arylthio des produits décrits ci-dessus peuvent être, si désiré, transformés en les fonctions sulfoxyde ou sulfone correspondantes dans les conditions usuelles connues de l'homme du métier telles que par exemple par les peracides comme par exemple l'acide peracétique ou l'acide métachloroperbenzoïque ou encore par l'ozone, l'oxone, le périodate de sodium dans un solvant tel que par exemple le chlorure de méthylène ou le dioxanne à la température ambiante.

   L'obtention de la fonction sulfoxyde peut être favorisée par un mélange équimolaire du produit renfermant un groupement alkylthio ou arylthio et du réactif tel que notamment un peracide.
   L'obtention de la fonction sulfone peut être favorisée par un mélange du produit renfermant un groupement alkylthio ou arylthio avec un excès du réactif tel que notamment un peracide.
m) Les éventuelles fonctions sulfure, sulfoxyde ou sulfone des produits décrits ci-dessus peuvent être, si désiré, transformées en les fonctions sulfoximine correspondantes dans les conditions usuelles connues de l'homme du métier : des exemples non exhaustifs de préparation de produits renfermant une fonction sulfoximine sont décrites ci-dessous.
   Ainsi par exemple pour la préparation de composés tels que les N-(arylsulfonyl) sulfoximines et par exemple dans le cas où le groupement aryle que représente X' est un radical toluène, la sulfoximine peut être obtenue par action de l'azoture de paratoluènesulfonyle sur le sulfoxyde correspondant soit -S(O)CH₃ de préférence en présence de cuivre ainsi qu'il est indiqué, par exemple, dans la référence suivante : J. A. C. S., 95, pp. 4287 (1973) JOHNSON C.R. & coll.
   Une autre méthode également utilisée consiste à traiter la N-tosylsulfilimine, elle-même préparée à partir du sulfure par action, par exemple, de la chloramine "T", par un agent oxydant comme par exemple, l'hypochlorite de sodium dans des conditions de transfert de phase ainsi qu'il est indiqué, par exemple, dans la référence suivante :
   J. Org. Chem., 49, pp. 2282 (1984) AKUTAGAWA K.& coll.
n) la réaction de transformation de fonction oxo en fonction thioxo peut être réalisée notamment par le réactif de LAWESSON dans les conditions définies ci-dessus.
o) La réaction de transformation de radical 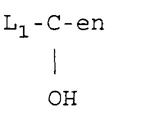 radical 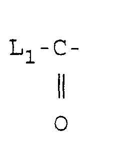 peut notamment être réalisée par un solvant alcool tel que par exemple l'oxyde de manganèse dans le dioxanne.
   La réaction inverse de transformation de radical 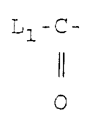 en radical 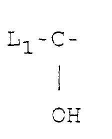 peut notamment être réalisée par le borohydrure de sodium dans l'éthanol.
p) La réaction de transformation de fonction acide en fonction tétrazolylcarboxy peut être réalisée par exemple par transformation préalable de la fonction acide en chlorure d'acide ainsi qu'il est indiqué ci-dessus, puis par action de Cu-C≡N, selon les conditions usuelles connues de l'homme du métier sur le chlorure d'acide ainsi obtenu, on obtient ainsi le radical 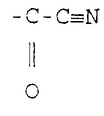 que l'on peut transformer en radical 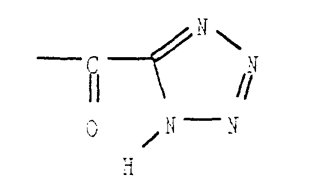 par exemple par action du composé Sn(Bu)₃N₃ dans le toluène,
q) la réaction de transformation de la fonction β céto sulfoxyde en fonction α céto thioester, peut être réalisée par bromation en α du cétosulfoxyde par exemple par action du NBS dans par exemple le chlorure de méthylène puis par une réaction de PUMMERER réalisée dans un mélange d'acide trifluoroacétique et de chlorure de méthylène ou encore un mélange d'acide sulfurique et de dioxanne.
   Notamment, ainsi qu'il est défini ci-dessus en c) et q), on peut réaliser le schéma réactionnel suivant : 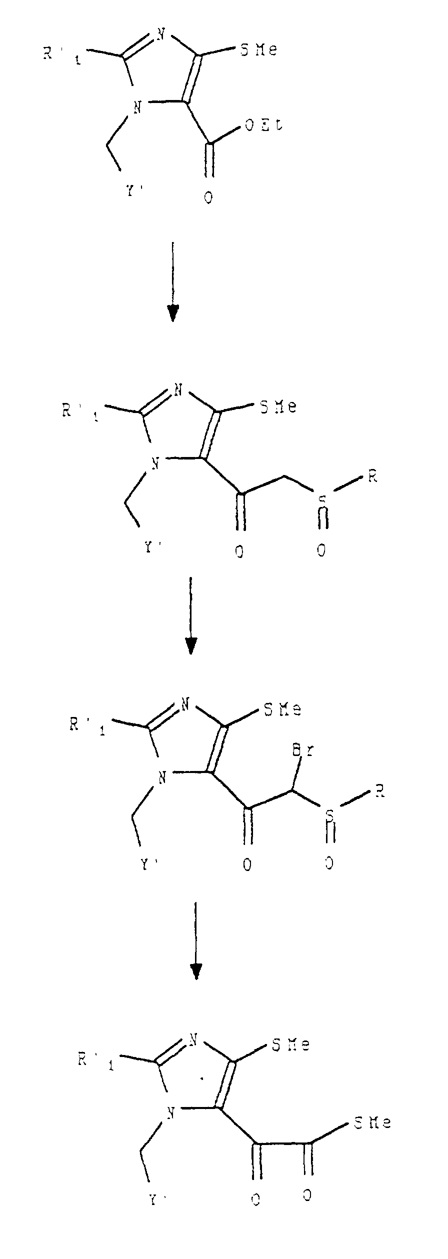 composés dans lesquels R'₁ et Y' ont les significations indiquées ci-dessus, et R représente un radical alkyle ou aryle éventuellement substitués ainsi qu'il est indiqué ci-dessus.
r) la réaction de transformation d'un carbamate en urée et notamment d'un sulfonylcarbamate en sulfonylurée, peut être réalisée par exemple au reflux d'un solvant comme par exemple le toluène en présence de l'amine adéquate.
s) la transformation d'un radical halogéné en radical formyle peut être notamment effectuée par action d'un dérivé organo métallique, par exemple le bromure d'éthyl magnésium, au sein d'un solvant organique,
t) la transformation du radical formyle en radical CH=CH-CO₂alk peut être effectuée par une réaction de type Wittig par condensation d'un sel de phosphonium approprié en présence d'hydrure de sodium ; la transformation en acide est effectuée par hydrolyse, par exemple au moyen d'une base telle que la soude en milieu alcoolique
u) la transformation du radical formyle en radical 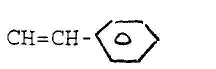 peut être effectuée par réaction de Wittig comme indiqué ci-dessus ; la transformation en radical 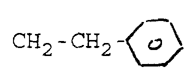 et 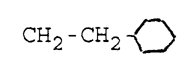 est effectuée par réduction, au moyen d'hydrogène en présence d'un catalyseur, par exemple l'oxyde de platine,
v) la transformation du radical formyle en radical CH₂OH peut être réalisée au moyen d'un agent réducteur, par exemple le borohydrure de sodium dans l'éthanol à la température ambiante ; la transformation en radical CH₂Rs peut être effectuée par action du thiol approprié Rs-SH sur le mésylate intermédiaire préparé au préalable par action du chlorure de mésyle sur l'alcool en présence de la base de Hunig,
w) l'oxydation du substituant S-alk en sulfoxyde peut être effectuée par exemple, par action de l'acide métachloroperbenzoïque ; la transformation du thiol est obtenue par la réaction de PUMMERER par exemple en présence d'anhydride trifluoroacétique ; la transformation du substituant SH en SZ₂ peut être obtenue par action d'un dérivé halogéné Hal-Z₂ par exemple l'iodocyclohexane.
   Il est entendu que les réactions décrites ci-dessus peuvent être effectuées selon les méthodes usuelles connues de l'homme du métier.
x) L'élimination de groupements protecteurs tels que par exemple ceux indiqués ci-dessus peut être effectuée dans les conditions usuelles connues de l'homme de métier notamment par une hydrolyse acide effectuée avec un acide tel que l'acide chlorhydrique, benzène sulfonique ou para-toluène sulfonique, formique ou trifluoroacétique ou encore par une hydrogénation catalytique.
   Le groupement phtalimido peut être éliminé par l'hydrazine.
   On trouvera une liste de différents groupements protecteurs utilisables par exemple dans le brevet BF 2 499 995.
y) Les produits décrits ci-dessus peuvent, si désiré, faire l'objet de réactions de salification par exemple par un acide minéral ou organique ou par une base minérale ou organique selon les méthodes usuelles connues de l'homme du métier.
z) Les éventuelles formes optiquement actives des produits décrits ci-dessus peuvent être préparées par dédoublement des racémiques selon les méthodes usuelles connues de l'homme du métier.

Des illustrations de telles réactions définies ci-dessus sont données dans la préparation des exemples décrits ci-après.

Les composés de formule (I) tels que définis ci-dessus ainsi que leurs sels d'addition avec les acides présentent d'intéressantes propriétés pharmacologiques.

Les produits de formule (I) tels que définis ci-dessus, sont doués de propriétés antagonistes pour les récepteurs à l'endothéline et sont ainsi notamment inhibiteurs des effets de l'endothéline, notamment des effets vasoconstricteurs et hypertenseurs induits par l'endothéline. On note en particulier un effet antiischémique, l'activité vasoconstrictrice de l'endothéline étant abolie.

Les produits de formule (I) sont également capables de s'opposer aux effets stimulants de l'endothéline au niveau de tous les types cellulaires, notamment les cellules musculaires lisses, les cellules neuronales et les cellules osseuses.

Ces propriétés justifient leur application en thérapeutique et l'invention a particulièrement pour objet à titre de médicaments, les produits de formule (I) telle que définie ci-dessus, dans laquelle Y comporte au moins un radical benzofuranyle ou un radical phényle substitué par un radical dioxol et éventuellement substitué par un ou plusieurs substituants choisis parmi les valeurs de R₂ et R₃,
lesdits produits de formule (I) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (I).

L'invention a ainsi pour objet à titre de médicaments, les produits tels que définis par la formule (I) ci-dessus, lesdits produits de formule (I) étant sous toutes les formes isoméres possibles racémiques ou optiquement actives, ainsi que les sels d'addition avec les acides minéraux et organiques ou avec les bases minérales et organiques pharmaceutiquement acceptables desdits produits de formule (I).

L'invention a plus particulièrement pour objet, à titre de médicaments, les produits décrits ci-après dans les exemples et notamment les produits de formule (I) telle que définie ci-dessus, répondant aux formules suivantes :
- acide 1-((6-chloro 1,3-benzodioxol-5-yl) méthyl) 4-((4-méthoxyphényl) méthylthio) 1H-imidazole 5-carboxylique
- acide 2-butyl 1-((6-chlor 1,3-benzodioxol-5-yl) méthyl) 5-((4-méthoxyphényl) thio) 1H-imidazole 4-carboxylique
- acide 1-((6-chloro 1,3-benzodioxol-5-yl) méthyl) 2,4-bis (((4-méthoxyphényl) méthyl) thio) 1H-imidazole 5-carboxylique
- acide 1-((6-chloro 1,3-benzodioxol-5-yl) méthyl) 4-(4-(méthoxyphényl) thio) 2-(propylthio) 1H-imidazole 5-carboxylique,
- acide 1-((6-chloro 1,3-benzodioxol-5-yl) méthyl) 4-((3,4-diméthoxyphényl) thio) 2-propyl 1H-imidazole 5-carboxylique,
- acide 2-butyl 1-((6-chloro 1,3-benzodioxol-5-yl) méthyl) 4-(cyclohexylthio) 1H-imidazole 5-carboxylique,
- acide 2-butyl 4-((3-carboxypropyl) thio) 1-((6-chloro 1,3-benzodioxol-5-yl) méthyl) 1H-imidazole 5-carboxylique,
- acide 4-(((4-(2-carboxyéthyl) phényl) thio) méthyl) 1-((6-chloro 1,3-benzodioxol-5-yl) méthyl) 2-propyl 1H-imidazol 5-carboxylique,
- acide 4-((3-carboxypropyl) thio) 1-1((6-chloro 1,3-benzodioxol-5-yl) méthyl) 2-propyl 1H-imidazol 5-carboxylique,
- acide 4-((7-carboxyheptyl) thio) 1-((6-chloro 1,3-benzodioxol-5-yl) méthyl) 2-propyl 1H-imidazol 5-carboxylique. ainsi que leurs sels d'addition avec les acides minéraux ou organiques ou avec les bases minérales et organiques pharmaceutiquement acceptables.

Les médicaments, objet de l'invention, trouvent, par exemple, leur emploi dans le traitement de tous les spasmes vasculaires, dans le traitement des post-hémorragies cérébrales, dans le traitement des spasmes coronariens, des spasmes vasculaires périphériques ainsi que dans le traitement des insuffisances rénales. Ces médicaments peuvent également être utilisés dans le traitement de l'infarctus du myocarde, de l'insuffisance cardiaque congestive, dans la prévention des resténoses post-angioplastie, dans le traitement de l'athérosclérose, de certains formes d'hypertension comme notamment l'hypertension pulmonaire, ainsi que dans le traitement de l'asthme.

Les médicaments, objet de l'invention, peuvent également trouver une application dans le traitement de l'ostéoporose, de l'hypertension prostatique et en tant que protecteurs neuronaux.

L'invention s'étend aux compositions pharmaceutiques contenant à titre de principe actif l'un au moins des médicaments tels que définis ci-dessus.

Ces compositions pharmaceutiques peuvent être administrées par voie buccale, rectale, par voie parentérale ou par voie locale en application topique sur la peau et les muqueuses ou par injection par voie intraveineuse ou intramusculaire.

Ces compositions peuvent être solides ou liquides et se présenter sous toutes les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 1 à 300 mg par jour chez l'adulte, par voie orale ou de 1 à 100 mg par jour par voie intraveineuse.

Certains produits de départ de formules (II) et (XVI) sont connus et peuvent être préparés par exemple comme indiqué dans le brevet européen EP 168 950.

D'autres produits de départ de formules (II) et (XVI) peuvent en particulier être préparés comme indiqué dans le brevet européen EP 0465368, ou encore dans les exemples décrits ci-après dans la partie expérimentale, notamment aux exemples 1 et 3.

Certains produits de départ de formules (II) et (XVI) sont commerciaux tels que par exemple, les produits de formule (II) suivants :
- le 2-méthoxyméthylimidazole
- le 2-propylimidazole
- le 2-isopropylimidazole
- le 2-éthylimidazole
- le 2-méthylimidazole.

Des exemples de produits commerciaux de formule (XVI) sont donnés dans les brevets EP 0465368 ou EP 0503162.

On peut encore notamment préparer certains produits de formules (II) et (XVI) à partir de produits de formule (II) par exemple en les soumettant à une ou plusieurs des réactions décrites ci-dessus en a) à z), réalisées dans les conditions également décrites ci-dessus.

Certains produits de formules (XVI) peuvent également être obtenus par monohalogénation du produit de formule (II) tel que défini ci-dessus en produit de formule (P₁) : 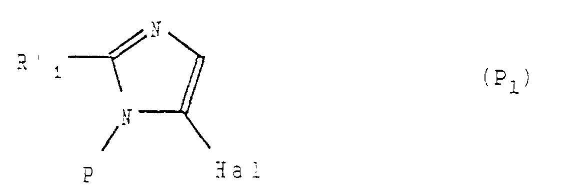 dans lequel R'₁ et P ont les significations indiquées ci-dessus pour le produit de formule (II), produit de formule (P₁) que l'on peut faire réagir, après échange suivant la réaction halogène métal connue de l'homme du métier, avec l'électrophile adapté, suivant les méthodes connues de l'homme du métier et notamment par exemple suivant le même type de réaction décrite ci-dessus pour passer par exemple du composé de formule (XIII) au composé de formule (XIV).
- Certains produits de formule (XVI) peuvent aussi être préparés selon le schéma suivant : 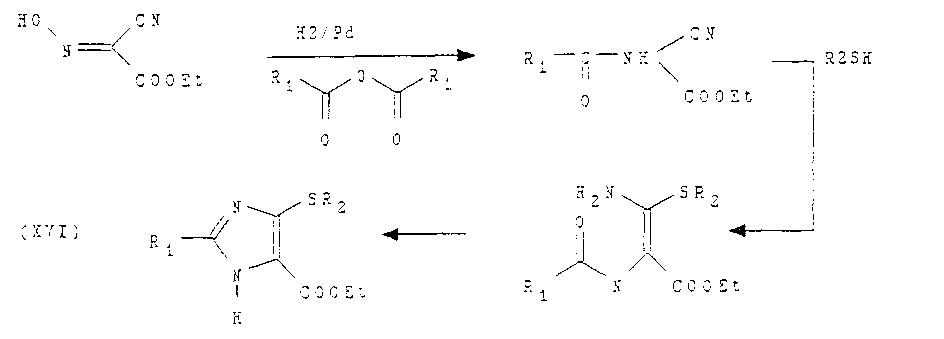
- Certains produits de formule (XVI) peuvent également être préparés selon le schéma suivant : 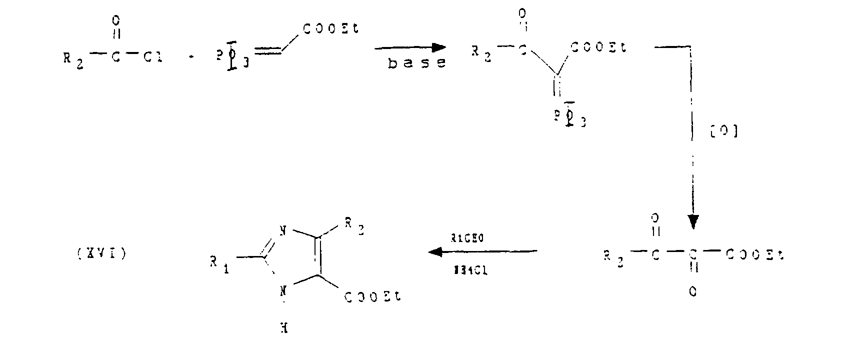
- Certains produits de formule (XVI) peuvent encore être préparés selon le schéma suivant :

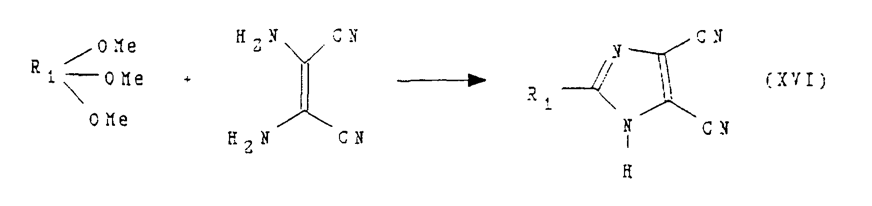

Les composés de départ de formule (VIII) peuvent être disponibles dans le commerce tel que le 2,4,5-tribromoimidazole ou peuvent être préparés selon les méthodes usuelles connues de l'homme du métier.

Les produits de départ de formules (Vₐ), (V_{b}), (VIₐ), (VI_{b}) et (VI_{c}), sont commerciaux tels que notamment :
les produits de formule (Vₐ) ou (XI) suivants :
   - le sec-butyl disulfide
   - l'éthyl disulfide
   - l'isopropyl disulfide
   - le méthyl disulfide
   - le benzyl disulfide
   - le phényl disulfide
   - le propyl disulfide
les produits de formule (V_{b}) ou (XII) suivants :
   - le méthyl méthanethiosulfonate
   - le phényl benzenethiol sulfonate
les produits de formule (VI_{c}) suivants :
   - le méthyl chloroformate
   - le benzyl chloroformate
   - l'isobutyl chloroformate
   - l'éthyl chloroformate
   - le N-propyl chloroformate
les produits de formule (VI_{d}) suivants :
   - le diméthyl carbonate
   - le diéthyl carbonate
les produits de formule (VIₑ) suivants :
   - le di-tert-butyl oxalate
   - le diéthyl oxalate
   - le diméthyl oxalate.

Les produits de départ de formules (X), (X') et (XII') sont commerciaux tels que notamment :
les produits de formule (X) suivants :
   - le benzaldehyde ou butanal
les produits de formule (X') suivants :
   - le chlorure de benzoyle ou de buturyle
les produits de formule (XII') suivants :
   - le chlorure de mesyle
   - le chlorure de tosyle

Un procédé de préparation de certains produits de formule (III) est notamment décrit dans le brevet européen EP 0465368.

Des exemples de préparation de composés de formule (III) sont également décrits dans la littérature et des exemples en sont donnés notamment dans le brevet US 4,880,804 ou par exemple dans la référence Chemistry and Industry 7 september 1987 HOWARD and COLQUHOUN pp. 612-617.

Notamment, le produit de formule (III) qui est le chlorure de 6-chloro pipéronyle est commercial chez Janssen.

La présente invention a enfin pour objet à titre de produits industriels nouveaux, les composés de formules (IV₁), (IV₂), (XIII), (XIV) et (XV) dans lesquels Y' représente le radical phényle substitué par un radical dioxol et éventuellement substitué par un ou plusieurs substituants choisis parmi les valeurs de R₂ et R₃, dans laquelle les éventuelles fonctions réactives sont éventuellement protégées par des groupements protecteurs.

L'invention a ainsi particulièrement pour objet l'utilisation des produits de formule (I) telle que définie ci-dessus, pour la préparation de compositions pharmaceutiques destinées au traitement d'affections résultant d'une stimulation anormale des récepteurs de l'endothéline et notamment destinées au traitement de l'hypertension induite par l'endothéline, de tous les spasmes vasculaires, au traitement: des post-hémorragies cérébrales, des insuffisances rénales, de l'infarctus du myocarde et à la prévention des resténoses post-angioplastie.

Les exemples suivants illustrent l'invention sans toutefois la limiter.

### EXEMPLE préparatif 1 :

### Exemple 1A : 2-butyl-1-((6-chloro-1,3-benzodioxol-5-yl) méthyl)-4-( (4-méthoxyphényl)méthylthio)-1H-imidazole-5-carboxylate d'éthyle

### EXEMPLE 1B : 2-butyl 1-((6-chloro-1,3-benzodioxol-5yl) méthyl)-5-((4-méthoxyphényl)méthylthio)-1H-imidazole-4-carboxylate d'éthyle

### STADE 1 : ((butylcarbonyl) amino) cyanoacétate d'éthyle

On introduit 6,71 g de 2-cyano-2-amino acétate d'éthyle (obtenu ainsi qu'il est indiqué dans Chemistry and Industry 1980, 541-542 par F.I. Logemann G.Shaw) dans 100 ml de chlorure de méthylène. On amène la solution à 0°C et ajoute 4,24 ml de pyridine, puis 6,31 ml de chlorure d'acide en 30 minutes à une cempérature inférieure à 6°C.

On laisse ensuite revenir à température ambiante.

On ajoute du toluène pour éliminer la pyridine en excès puis reprend par 200 ml de chlorure de méthylène et lave à l'eau deux fois puis sèche.

Le solide obtenu est purifié par empâtage à l'étherisopropylique puis filtré. On obtient ainsi 8,40 g de produit attendu (solide blanc) point de fusion 88°C.

### Analyses

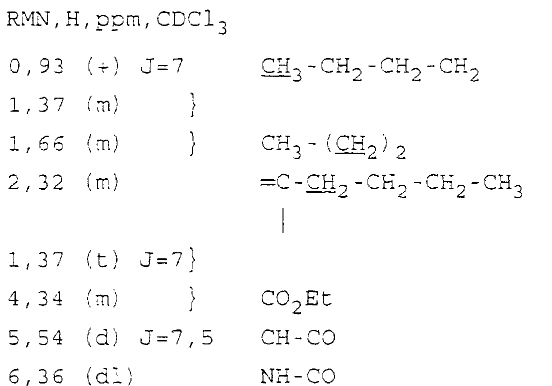

### STADE 2 : 3-amino 2-((butylcarbonyl) amino) 3-(((4-méthoxyphényl) méthyl) thio) propènoate d'éthyle

On introduit 3 g du produit obtenu au stade 1 ci-dessus, 4 ml de paraméthoxybenzylmercaptan, 0,2 ml de triéthylamine et 5 ml d'éthanol.

On agite à température ambiante durant 3-4 jours, distille le solvant, reprend dans l'éther isopropylique, filtre le précipité puis lave abondamment à l'éther isopropylique. On obtient ainsi 4,65 g de produit attendu (solide blanc), Pf = 120°C

### Analyses

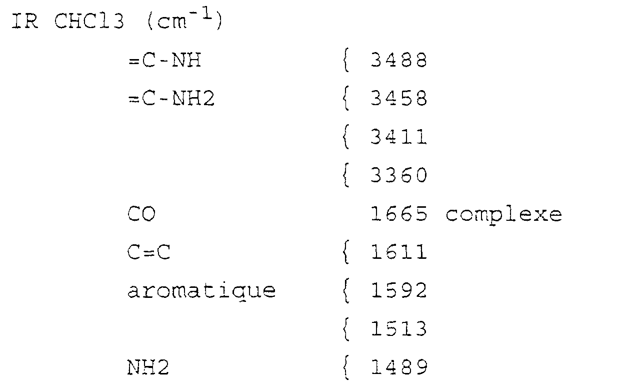

### Microanalyse

| | % calculés | % trouvés |
|---|---|---|
| C | 58,99 | 58,90 |
| H | 7,15 | 7,17 |
| N | 7,64 | 7,63 |
| S | 8,75 | 8,79 |

### STADE 3 :2-butyl-4-((4-méthoxyphényl)-méthylchio)-1H-imidazole-5-carboxylate d'éthyle

On procède à partir de 1,14 g de pentachlorure de phosphore dans 20 ml de chlorure de méthylène, refroidit à 0°C et ajoute 0,73 g de 4-diméthylamino pyridine en solution dans 7 ml de chlorure de méthylène. On agite alors 5 minutes puis ajoute lentement 1 g du produit obtenu au stade 2 ci-dessus en solution dans 10 ml de chlorure de méthylène pendant environ 5 minutes.

Après chromatographie sur silice avec pour éluant (AE/chlorure de méthylène, 10:90), on obtient 686 mg de produit attendu Pf = 105 °C.

### Analyses

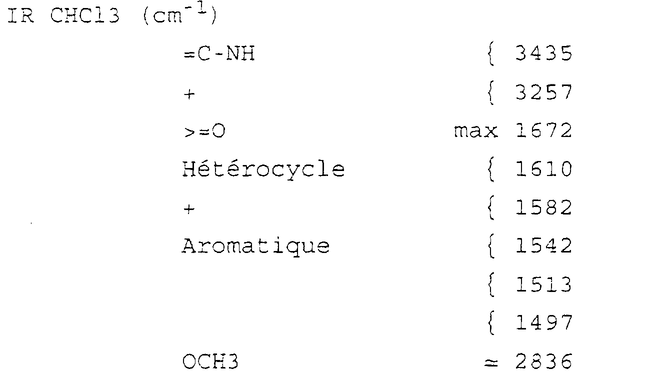

### STADE 4 : 2-butyl-1-((6-chloro-1,3-benzodioxol-5-yl)méthyl)-4-((4-méthoxyphényl)méthylthio)-1H-imidazole-5-carboxylate d'éthyle et 2-butyl-1-((6-chloro-1,3-benzodioxol-5yl) méthyl)-5-((4-méthoxyphényl)méthylthio)-1H-imidazole-4-carboxylate d'éthyle

Dans 14,2 ml de diméthylformamide, à température ambiante, on mélange 495 mg du produit obtenu au stade 3 ci-dessus, 350 mg de chlorure de 6-chloro piperonyl, et 235 mg de bicarbonate de potassium et le milieu réactionnel est laissé pendant environ 48 heures.

On hydrolyse alors avec H2O (chlorure d'ammonium), extrait à l'acétate d'éthyle et lave par 2 x H2O (chlorure d'ammonium), 2 x H2O, 2 x H2O (chlorure de sodium), sèche, filtre et évapore à sec.

Après purification sur colonne de silice avec pour éluant acétate d'éthyle/Cyclohexane (1/4), on obtient 557,6 mg du produit attendu de l'exemple 1A et 60,0 mg du produit attendu de l'exemple 1B.

### Analyses

### 1) Exemple 1A

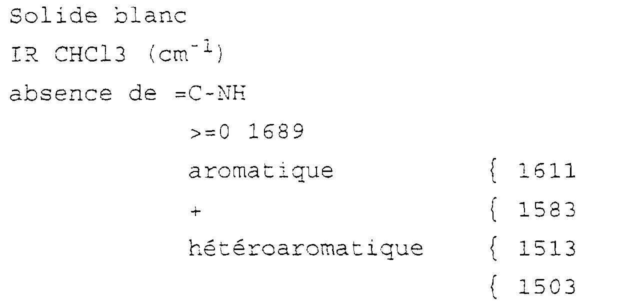

### 2) Exemple 1B

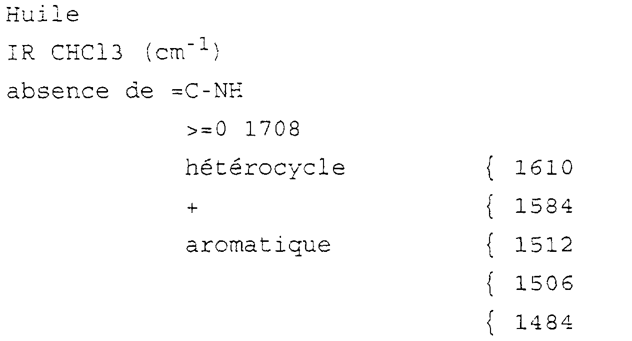

### EXEMPLE 2 : Acide 2-butyl-1-((6-chloro-1,3-benzodioxol-5yl) méthyl)-4-((4-méthoxyphényl)méthylthio)-1H-imidazole-5-carboxylique

Dans 10,4 ml d'éthanol, on met 536 mg du produit de l'exemple 1A puis 1,04 ml de soude 2N, 10 ml de tétrahydrofurenne et chauffe à 50 °C pendant 12 heures.

On ajoute alors 10 ml d'eau (distillée), évapore sous vide pour éliminer le tétrahydrofuranne et l'éthanol, ajoute 20 ml d'eau (distillée), agite bien cette suspension, introduit goutte à goutte 2,08 ml d'acide chlorhydrique (1N) puis laisse 1 heure à température ambiante sous agitation, 30 minutes sous ultrason et 1 heure à 60°C sous agitation.

Après refroidissement à température ambiante, on filtre, lave par de l'eau distillée (3x15 ml) et obtient 460 mg de produit atrendu (solide blanc) (Pf = 158°C).

### Analyses

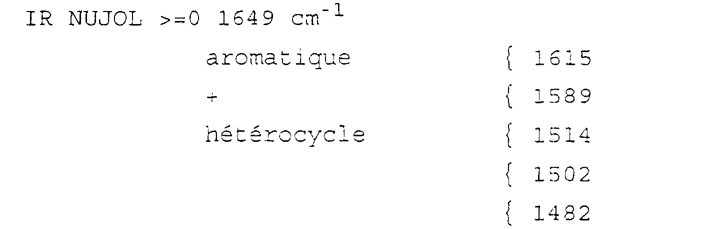

### Microanalyse

| | % calculés | % trouvés |
|---|---|---|
| C | 58,95 | 58,9 |
| H | 5,15 | 5,2 |
| Cl | 7,25 | 7,5 |
| N | 5,72 | 5,6 |
| S | 6,55 | 6,8 |
| O | 16,35 | |

### EXEMPLE 3A : 2-butyl-1-((6-chloro-1,3-benzodioxol-5-yl) méthyl-4-((4-méthoxyphényl)thio)-1H-imidazole-5-carboxylate d'éthyle

### EXEMPLE 3B : 2-butyl-1-((6-chloro-1,3-benzodioxol-5yl) méthyl)-5-((4-méthoxyphényl)thio)-1H-imidazole-4-carboxylate d'éthyle

### STADE 1 : 3-amino 2-((butylcarbonyl) amino) 3-((4-méthoxyphényl) thio) propènoate d'éthyle

On introduit 1 g du produit obtenu au stade 1 de l'exemple préparatif 1, 10 ml d'éthanol 100 %, 0,065 cm³ de triéthylamine et 1,72 ml de 4-méthoxy thiophénol.

On agite à température ambiante durant 3 jours. On porte à sec, reprend par 10 ml d'éther isopropylique, agite durant 30 minutes, essore et rince à l'éther isopropylique. On obtient 1,52 g de produit attendu (solide blanc) (Pf = 122°C).

### Analyses

### Microanalyse

| | % calculés | % trouvés |
|---|---|---|
| C | 57,43 | 58,01 |
| H | 6,86 | 6,97 |
| N | 7,95 | 7,93 |
| S | 9,10 | 9,15 |

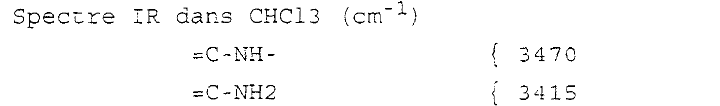

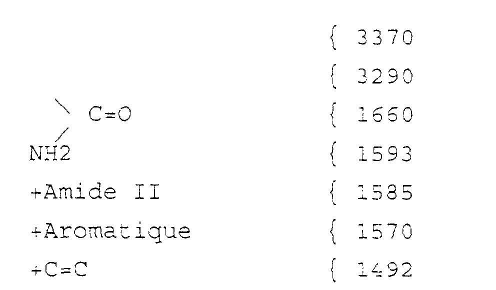

### Spectre UV dans l'éthanol :

Max 235 nm ε = 13900
Max 290 nm ε = 28800

### STADE 2 : 2-butyl-4-((4-méthoxyphényl)thio)-1H-imidazole-5-carboxylate d'éthyle

On introduit 1,75 g de pentachlorure de phosphore et 87,5 ml de chlorure de méthylène/NK20 et refroidit à -70°C puis ajoute en 5 minutes la solution de 1,127 g de 4-diméthylaminopyridine et 11,27 ml de chlorure de méthylène/NK20.

On agite 10 minutes à -70°C puis ajoute en 5 minutes la solution de 1,48 g du produit obtenu au stade 1 ci-dessus et 14,8 ml de chlorure de méthylène/NK20.

On laisse revenir à température ambiante et agite durant 16 heures. On hydrolyse en ajoutant 100 ml de solution saturée d'hydrocarbonate de sodium, agite durant 30 minutes, décante, réextrait par 3 x 50 ml de chlorure de méthylène, lave par 50 ml d'eau et sèche. Après chromatographie sur silice dans chlorure de méthylène-acétate d'éthyle (8/2), on isole 1 g de produit attendu (solide crème) Pf ≃ 96°C.

### Analyses

### Microanalyse

| | % calculés | % trouvés |
|---|---|---|
| C | 61,05 | 60,7 |
| H | 6,63 | 5,8 |
| N | 8,37 | 8,3 |
| S | 9,58 | 9,4 |

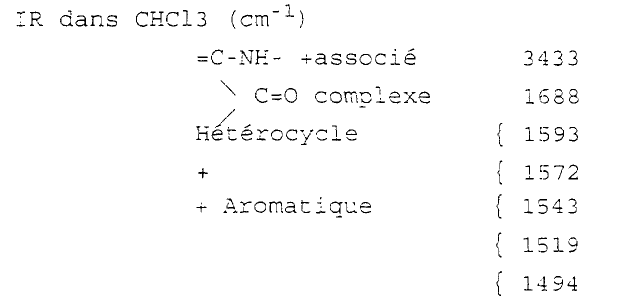

### Spectre UV

- Dans l'éthanol : Infl 233 nm ε = 13100
   (+chlorure de méthylène) Max 251 nm ε = 13800
   Max 238 nm ε = 8800
   Infl 225
- Dans l'éthanol-HCl(0,1N) : Max 242 nm ε = 16200
   Max 273 nm ε = 8700
   Infl 325

### STADE 3 : 2-butyl-1-((6-chloro-1,3-benzodioxol-5-yl)méthyl-4-((4-méthoxyphényl) thio)-1H-imidazole-5-carboxylate d'éthyle et 2-butyl-1-((6-chloro-1,3-benzodioxol-5yl)méthyl)-5-((4-méthoxyphényl)thio)-1H-imidazole-4-carboxylate d'éthyle

On procède comme à l'exemple 1 à partir de 1,6 g du produit obtenu au stade 2 ci-dessus, 24 ml de diméthylformamide, 0,79 g de bicarbonate de sodium et 1,14 g de chlorure de 6-chloropiperonyl que l'on laisse à température ambiante pendant 48 heures.

Après purification par chromatographie sur colonne de silice avec pour éluant chlorure de méthylène-acétate d'éthyle 95-05, on obtient 1,24 g de produit attendu de l'exemple 3A (poudre) (Pf = 100°C) et 1,14 g de produit attendu de l'exemple 3B (huile).

### Analyses

### 1) Exemple 3A

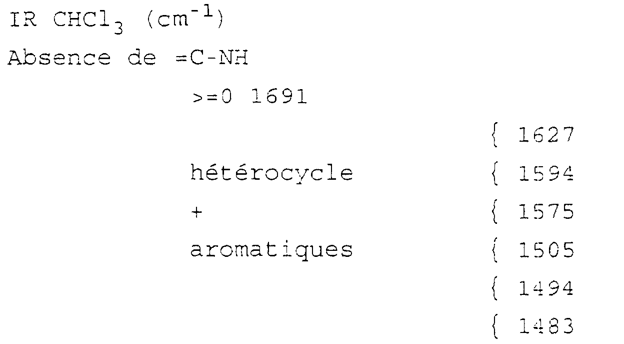

### 2) Exemple 3B

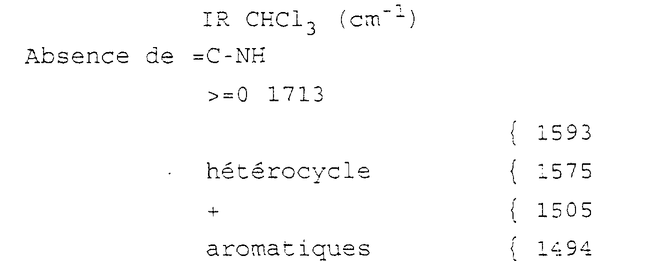

### EXEMPLE 4 : Acide 2-butyl-1-((6-chloro-1,3-benzodioxol-5-yl)méthyl)-4-((4-méthoxyphényl)thio)-1H-imidazole-5-carboxylique

On procède comme à l'exemple 2 à partir de 0,35 g du produit de l'exemple 3A, 3 ml d'éthanol + 1 ml de méthanol puis introduit à température ambiante 0,417 ml puis 0,2 ml de soude 2N.

On laisse 14 heures à température ambiante, amène à sec, reprend par 10 ml d'eau puis 0,62 ml d'acide chlorhydrique 2N.

On obtient ainsi 290 mg de produit attendu (Pf = 186°C).

### Analyses

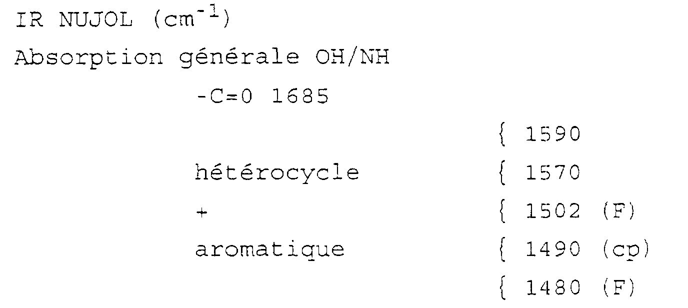

### Spectre UV

dans l'éthanol
max 232 nm ε *=* 18100
max 291 nm ε = 16500
dans acide acétique-NaOH (N/10)
max 241 nm ε = 18900
max 285 nm ε = 11500

### Microanalyse

| | % calculés | % trouvés |
|---|---|---|
| C | 58,11 | 57,9 |
| H | 4,88 | 4,7 |
| N | 5,89 | 5,7 |
| S | 6,75 | 6,9 |
| Cl | 7,46 | 7,6 |

### EXEMPLE 5 : 2-butyl-1-((6-chloro-1,3-benzodioxol-5-yl) méthyl)-4-((4-méthoxyphényl)sulfinyl-1H-imidazole-5-carboxylate d'éthyle

On introduit 0,4 g du produit de l'exemple 3A, 4 ml de chlorure de méthylène/NK₂₀ et goutte à goutte à 0°C une solution de 0,174 g acide métachloroperbenzoïque à 85°C dans 1,7 cm3 de chlorure de méthylène/NK₂₀.

On laisse remonter à température ambiante, laisse sous agitation pendant 4 heures et rajoute alors 0,03 g d'acide métachloroperbenzoïque dans du chlorure de méthylène.

On laisse sous agitation pendant 16 heures, puis verse dans 50 cm³ d'eau, amène à pH 6-7 par addition de 2 ml de solution de bicarbonate de sodium saturée et extrait avec 3x50 ml de chlorure de méthylène.

On sèche, filtre et amène à sec.

Après purification par chromatographie sur colonne de silice avec pour éluant chlorure de méthylène-acétate d'éthyle 80-20, on obtient 0,37 g de produit attendu (huile)

### Analyses

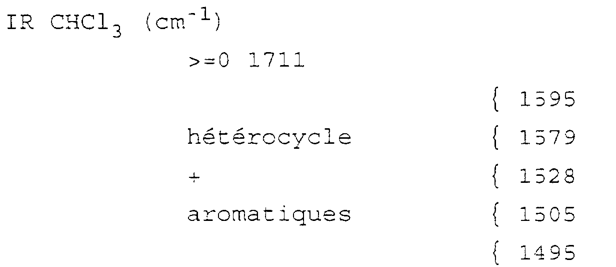

### EXEMPLE 6 : Acide 2-butyl-1-((6-chloro-1,3-benzodioxol-5-yl)méthyl)-4-((4-méthoxyphényl)sulf-1H-imidazole-5-carboxylique

On procède comme à l'exemple 2 à partir de 350 mg du produit de l'exemple 5 dans 3,5 ml d'éthanol. On introduit alors 0,438 ml de soude 2N et laisse sous agitation à température ambiante pendant 4 heures. On anène à sec, reprend le résidu huileux par 5 ml d'eau, filtre, ajoute 0,44 ml d'acide chlorhydrique 2N et laisse sous agitation pendant 1/2 heure. On filtre, lave à l'eau, sèche et obtient 287 mg de produit attendu (Pf = 90°C).

### Analyses

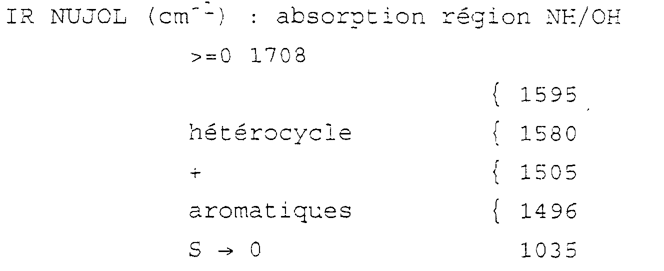

### Spectre UV

dans l'éthanol
max 240 nm ε = 21000
inf 260, 280, 295 nm

### Microanalyse

| | % calculés | % trouvés | |
|---|---|---|---|
| C | 56,26 | 55,9 | 56,0 |
| H | 4,72 | 4,8 | 4,6 |
| N | 5,70 | 5,6 | 5,5 |
| S | 6,53 | | 6,8 |
| Cl | 7,22 | | 7,3 |

### EXEMPLE 9 : 2-butyl-1-((6-chloro-1,3-benzodioxol-5-yl)méthyl) -4- ((4-méthoxyphényl)sulfonyl)-1H-imidazole-5-carboxylate d'éthyle

On introduit 0,44 g du produit de l'exemple 3A dans 4,4 ml de chlorure de méthylène et ajoute goutte à goutte à température ambiante en solution 0,397 g d'acide métachloroperbenzoïque à 85 % et 2 ml de chlorure de méthylène.

On laisse pendant 16 heures sous agitation, à température ambiante, verse dans 50 ml d'eau, amène à pH ≃ 6-7 par addition de solution saturé de bicarbonate de sodium, extrait avec 3 fois 50 ml de chlorure de méthylène, sèche, filtre et amène à sec.

Après purification par chromatographie sur colonne de silice avec pour éluant chlorure de méthylène-acétate d'éthyle/95.05, on obtient 380 g de produit attendu.

### Analyses

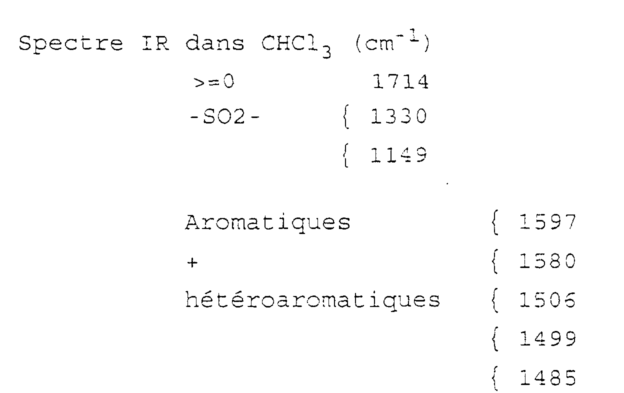

### EXEMPLE 10 : acide 2-butyl-1-((6-chloro-1,3-benzodioxol-5-yl)méthyl)-4-((4-méthoxyphényl)sulfonyl)-1H-imidazole-5-carboxylique

On procède comme à l'exemple 2 à partir de 0,35 g de produit de l'exemple 9, 3,5 ml d'éthanol et 0,43 ml de soude 2N.

On laisse 3 heures à température ambiante sous agitation, amène à sec, reprend par 5 ml d'eau et ajoute 0,44 ml d'acide chlorhydrique 2N puis laisse 2 heures sous agitation, filtre et lave à l'eau.

On obtient ainsi 280 mg de produit attendu (poudre) F = 154°C.

### Analyses

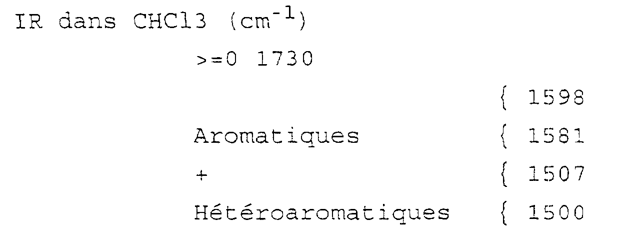

### Spectre UV

- dans éthanol-acide chlorhydrique N/10
   max 245 nm ε = 19800
   infl 257, 270, 279, 294 nm
- Dans éthanol-soude N/10
   max 253 nm ε = 19800
   max 292 nm ε = 4200
   infl 270 nm

### Microanalyse

| | % calculés | % trouvés |
|---|---|---|
| C | 54,49 | 54,4 |
| H | 4,57 | 4,5 |
| N | 5,52 | 5,4 |
| S | 6,32 | 6,5 |
| Cl | 6,99 | 7,06 |

### EXEMPLE préparatif 11 : 1-((6-chloro-1,3-benzodioxol-5-yl)méthyl)-2,4-bis(((4-méthoxyphényl)méthyl)thio)-1H-imidazole-5-carboxaldehyde

### STADE 1 : 1-((6-chlore-1,3-benzodioxol-5-yl)méthyl)-2,4,5-tribromo-1H-imidazole

On introduit 25 g de 2,4,5 tribromoimidazole dans 500 ml de diméthylformamide et ajoute 4,3 g d'hydrure de sodium. L'agitation est maintenue 10 mn à température ambiante. On y ajoute ensuite au milieu réactionnel 18,4 g de chlorure de 6 chloro pipéronyl, puis 25 g d'iodure de sodium et l'agitation est poursuivie pendant 15 mn à température ambiante.

Le milieu réactionnel est finalement versé sur 3 1 d'eau, on essore, lave abondamment par de l'eau, puis successivement par 250 ml d'éthanol, 250 ml d'isopropanol, puis finalement par 250 ml d'éther isopropylique.

On sèche et récupère 31,5 g de produit attendu (solide crème) Pf = 225°C.

### Analyses

IR CHC13 (cm⁻¹)
Absence de =C-NH
Hétérocycle aromatique 1624-1506-1497-1485

### STADE 2 : 1-((6-chloro-1,3-benzodioxol-5-yl)méthyl)-4,5-dibromo-2-(phénylthio)-1H-imidazole

On introduit 4,73 g du produit obtenu ci-dessus au stade 1 dans 100 ml de mélange chlorure de méthylène/éther sulfurique (20/80), à laquelle on ajoute goutte à goutte 3,5 ml de bromure d'éthyle magnésium : on maintient sous agitation pendant 30 mn à température ambiante.

On introduit alors au milieu réactionnel obtenu 2,9 g de phényl benzène thiosulfonate. L'agitation est maincenue pendanc 2 h à température ambiante.

On hydrolyse le milieu par addition d'acide chlorhydrique dilué, après extraction par de l'acétate d'éthyle lavage abondant à l'eau puis séchage, on récupère un solide crème que l'on purifie par empâtage dans 50 ml d'éthanol. On obtient ainsi 3,35 g du produit attendu Pf 165°C.

### Analyses

IR CHC13 (cm⁻¹)
Hétérocycle + aromatique 1585-1508-1484

### Microanalyse

| | % calculés | % trouvés |
|---|---|---|
| C | 40,6 | 40,7 |
| H | 2,2 | 2,1 |
| N | 5,57 | 5,4 |
| S | 6,37 | 6,4 |
| Br | 31,8 | 31,4 |
| Cl | 7,05 | 6,8 |

### STADE 3 : 4-bromo-1-((6-chloro-1,3-benzodioxol-5-yl)méthyl)-2- (phénylthio)-1H-imidazole-5-carboxaldehyde

On introduit 6,4 g du produit obtenu au stade 2 ci-dessus dans 100 ml de tétrahydrofuranne et on ajoute goutte à goutte 9,3 ml de bromure d'éthyle magnésien. On maintient sous agitation pendant 30 mn à température ambiante.

On introduit alors au milieu réactionnel obtenu 5 équivalents de diméthyl formamide.

L'agitation est maintenue pendant 2 h à température ambiante.

On hydrolyse le milieu par addition d'acide chlorhydrique dilué, après extraction de l'acétate d'éthyle, lavage abondant à l'eau puis séchage, on récupère un solide marron que l'on purifie par chromatographie sur silice avec pour éluant chlorure de méthylène. On obtient 5 g de produit attendu (Pf = 155°C) dont 500 mg sont recristallisés dans 25 ml d'éthanol : après essorage puis séchage on récupère 420 mg de produit attendu (solide blanc) (Pf = 155°C).

### Analyses

IR CHC13 (cm⁻¹)
C=0 1669
Aromatique+Hétéroatome 1627-1580-1505-1485

### Microanalyse

| | % calculés | % trouvés |
|---|---|---|
| C | 47,9 | 47,9 |
| H | 2,67 | 2,6 |
| N | 6,2 | 6,1 |
| S | 7,09 | 7,2 |
| Br | 17,68 | 17,7 |
| Cl | 7,84 | 7,7 |

### STADE 4 : 1-((6-chloro-1,3-benzodioxol-5-yl)méthyl)-2,4-bis(((4-méthoxyphényl)méthyl)thio)-1H-imidazole-5-carboxaldehyde

On introduit 620 mg d'hydrure de sodium 50 % dans l'huile dans 50 ml de tétrahydrofuranne, à laquelle on ajoute lentement 1,8 ml de méthoxy-benzeneméthanethiol. L'agitation est maintenue pendant 15 mn à température ambiance.

On ajoute ensuite au thiolate ainsi formé une solution de 3,8 g du produit obtenu au stade 3 ci-dessus dans 25 ml de tétrahydrofuranne. L'agitation est poursuivie dans ces conditions pendant 2 h 30mn.

Le milieu réactionnel est finalement versé sur de la soude 0,1N, après extraction par de l'acétate d'éthyle, lavage abondant à l'eau puis séchage, on récupère une résine brune que l'on purifie par chromatographie sur silice avec pour éluant chlorure de méthylène 80/cyclohexane 20 puis chlorure de méthylène + 20 % d'acétate d'éthyle.

On recueille 2 fractions 1,98 g du produit attendu B et 1,97 g d'un second produit A (Pf = 135°C) qui se forme dans les mêmes conditions opératoires : le 1-((6-chloro-1,3-benzodioxol-5-yl)méthyl)-4-(((4-méthoxyphényl)méthyl)thio)-2-(phénylthio)-1H-imidazole-5-carboxaldehyde

### ANALYSES POUR A

IR CHC13 (cm⁻¹)
C=O 1660
aromatique + Hétérocycle 1611-1580-1513-1505-1485

### Microanalyse

| | % calculés | % trouvés |
|---|---|---|
| C | 59,47 | 59,2 |
| H | 4,03 | 3,8 |
| N | 5,33 | 5,2 |
| S | 12,21 | 12,0 |
| Cl | 6,75 | 6,7 |

### ANALYSES POUR B

IR CHC13 (cm⁻¹)
absence de S phényl
C=O 1655
aromatique Hétérocycle 1612-1585-1512-1508-1485

### EXEMPLE préparatif 12 : 1-((6-chloro-1,3-benzodioxol-5-yl)méthyl)-2,4-bis(((4-méthoxyphényl)méthyl)thio)-1H-imidazole-5-carboxylate de méthyle

On introduit une solution de 1 g du produit de l'exemple préparatif 11 dans un mélange chlorure de méthylène/méthanol (50 ml/ 100 ml), ajoute ensuite successivement 5 g d'oxyde de manganèse, 500 mg de cyanure de sodium et 200 µl d'acide acétique.

L'agitation est alors maintenue pendant 72 heures à température ambiante.

On essore, lave par du chlorure de méthylène, concentre et purifie sur silice avec pour éluant chlorure de méthylène + 20 % d'acétate d'éthyle.

On obtient ainsi 750 mg de produit attendu (résine).

### Analyses

IR CHCl3 (cm⁻¹)
ester 1696-1436
aromatique + hétérocycle 1611-1585-1513-1505-1484

### EXEMPLE 13 : Acide 1-((6-chloro-1,3-benzodioxol-5-yl)méthyl)-2,4-bis(((4-méthoxyphényl)méthyl)thio)-1H-imidazole-5-carboxylique

On procède comme à l'exemple 2 à partir de 580 mg du produit de l'exemple préparatif 12 dans un mélange éthanol/tétrahydrofuranne (20 ml/ 60 ml) à laquelle on ajoute 20 ml de soude N.

L'agitation est maintenue pendant 48 heures à température ambiante.

On acidifie par addition d'acide chlorhydrique 2N, extrait par de l'acétate d'éthyle, lave à l'eau puis sèche et purifie par chromatographie sur silice avec pour éluant chlorure de méthylène + 5 % méthanol.

On purifie de nouveau par empâtage dans 50 ml d'éthanol bouillant, lave par 20 ml d'acétate d'éthyle puis sèche et récupère ainsi 350 mg de produit attendu (solide blanc) Pf = 210 °C.

### Analyses

IR NUJOL (cm⁻¹)
Absorption complexe OH/NH
-C=O 1648
Aromatique hétéroatome 1513-1585-1513-1502

### Microanalyse

| | % calculés | % trouvés |
|---|---|---|
| C | 57,47 | 57,3 |
| H | 4,3 | 4,2 |
| N | 4,79 | 4,7 |
| S | 10,96 | 10,7 |
| Cl | 6,06 | 6,4 |

### EXEMPLE préparatif 14 : 4-brome-1-((6-chlore-1,3-benzodioxol-5-yl)méthyl)-2-(propylthio)-1H-imidazole-5-carboxaldehyde STADE 1 : 1-((6-chloro-1,3-benzodioxol-5-yl)méthyl)-4,5 dibromo 2-(propylthio)-1H-imidazole

On introduit 5 g du produit obtenu du stade 1 de l'exemple préparatif 11 dans 100 ml de tétrahydrofuranne.

Cette solution est refroidie à -78°C puis on y ajoute lentement 7,8 ml de méthyl lithium. L'agitation est maintenue à cette température pendant 10 mn puis on ajoute 4 ml de disulfure de dipropyl. L'agitation est poursuivie pendant 2h30mn (température ambiante).

On hydrolyse par addition de soude diluée, ajoute 100 ml d'éthanol, essore, lave abondamment par de l'eau puis par 150 ml d'éthanol.

On obtient ainsi 1,8 g de produit attendu (solide crème) Pf = 116°C.

### Analyses

IR CHCl3 (cm⁻¹)
Système conjugué 1627-1506-1484

### STADE 2 : 4-bromo-1-((6-chloro-1,3-benzodioxol-5-yl)méthyl)--2-(propylthio)-1H-imidazole-5-carboxaldehyde

On introduit 2,1 g du produit obtenu au stade 1 ci-dessus dans 30 ml de tétrahydrofuranne et ajoute goutte à goutte 10 ml de bromure d'éthyle magnésien. On maintient sous agitation pendant 15 mn à température ambiante.

On introduit alors au milieu réactionnel 2 ml de diméthylformamide. L'agitation est maintenue pendant 2 h à température ambiante.

On hydrolyse le milieu par addition d'acide chiorhydrique dilué, extrait par de l'acétate d'éthyle, lave à l'eau puis sèche et purifie par empâtage dans 2x20 ml d'éthanol.

On obtient ainsi 1,4 g de produit attendu Pf = 145°C.

### Analyses

IR CHC13 (cm⁻¹)
C=O 1663
Aromatique + Hétérocycle 1627-1505-1484

### EXEMPLE préparatif 15 : 1-((6-chloro-1,3-benzodioxol-5-yl)méthyl)-4-(4-méthoxyphényl)thio)-2-(propylthio)-1H-imidazole-5-carboxaldéhyde

On introduit 240 mg d'hydrure de sodium dans 50 % dans l'huile dans 50 ml de tétrahydrofuranne et ajoute lentement 0,6 ml de paraméthoxyphénylthiol et l'agitation est maintenue pendant 15 mn à température ambiante.

On introduit ensuite au thiolate ainsi formé une solution de 1,4 g au produit de l'exemple préparatif 14 dans 20 ml de tétrahydrofuranne et agite pendant 2h. On verse alors sur la soude 0,1N, extrait par de l'acétate d'éthyle, lave à l'eau, sèche et purifie par chromatographie sur silice avec pour éluant chlorure de méthylène. On obtient ainsi 1,32 g de produit attendu.

### Analyses

IR CHC13 (cm⁻¹)
C=O 1657
Aromatique + hétérocycle 1593-1570-1505-1494-1484-dont S-Φ-CCH3

### EXEMPLE 16 : 1-((6-chloro-1,3-benzodioxol-5-yl)méthyl)-4-((4-méthoxyphényl)thio)-2-(propylthio)-1H-imidazole-5-carboxylate de méthyle

On procède comme à l'exemple préparatif 12 à partir de 1,26 g du produit de l'exemple préparatif 15 dans un mélange chlorure de méthylène/méthanol (20 ml/100 ml) et ajoute successivement 6 g d'oxyde de manganèse, 600 mg de cyanure de sodium et 300 µl d'acide acétique.

L'agitation est alors maintenue pendant 24 h à température ambiante.

On obtient ainsi 1,1 g de produit attendu.

### Analyses

IR CHC13 (cm⁻¹)
ester 1697-1436
Aromatique + hétérocycle 1593-1575-1505-1494-1484

### EXEMPLE 17 : Acide 1-((6-chloro-1,3-benzodioxol-5-yl)méthyl)-4-((4-méthoxyphényl)thio)-2-(propylthio)-1H-imidazole-5-carboxylique

On procède comme à l'exemple 13 à partir de 1 g du produit de l'exemple 16 dans un mélange éthanol/tétrahydrofuranne (20 ml/ 20 ml) à laquelle on ajoute 25 ml de soude N.

L'agitation est maintenue pendant 48 h à température ambiante.

On obtient ainsi 270 mg de produit attendu (solide blanc) Pf = 164°C.

### Analyses

IR NUJOL (cm⁻¹)
-C=O 1650
Aromatique Hétéroatome 1592-1573-1508-1489

### Microanalyse

| | % calculés | % trouvés |
|---|---|---|
| C | 53,6 | 53,5 |
| H | 4,3 | 4,2 |
| N | 5,68 | 5,4 |
| S | 13,0 | 12,7 |
| Cl | 7,19 | 7,4 |

### EXEMPLE préparatif 18 : 1-((6-chloro-1,3-benzodioxol-5-yl)méthyl)-4-bromo 2-méthyl -1H-imidazole-5-carboxaldéhyde

### STADE 1 : 1-((6-chloro-1,3-benzodioxol-5-yl)méthyl) 2-méthyl -1H-imidazole

On procède comme au stade 1 de l'exemple préparatif 11 à partir de 12,3 g de 2-méthyl-1H-imidazole, 7,92 g de hydrure de sodium à 50 % huile, 150 ml de N diméthylformamide et 33,8 g de chlorure de 6-chloropipéronyl.

Le produit attendu est obtenu sans être isolé.

### STADE 2 : 1-((6-chloro-1,3-benzodioxol-5-yl)méthyl)-4,5-dibromo 2-méthyl -1H-imidazole

On introduit le produit brut obtenu au stade 1 ci-dessus dans 58,7 g de N bromosuccinimide et 200 ml de chlorure de méthylène au reflux.

On obtient ainsi 16,33 g ce produit attendu Pf = 185°C.

### STADE 3 : 1-((6-chloro-1,3-benzodioxol-5-yl)méthyl)-4-bromo 2-méthyl -1H-imidazole-5-carboxaldéhyde

On procède comme au stade 3 de l'exemple préparatif 11 à partir de 15 g du produit obtenu au stade 2 ci-dessus dans 300 ml de tétrahydrofuranne, 73,4 ml de bromure d'éthyle magnésien en solution dans du tétrahydrofuranne et 28,4 ml de N diméthylformamide.

On obtient ainsi 9,8 g de produit attendu Pf = 141°C

### EXEMPLE préparatif 19 : 1-((6-chloro-1,3-benzodioxol-5-yl)méthyl)-4-((4-méthoxyphényl)thio)-2-méthyl-1H-imidazole-5-carboxaldehyde

On procède comme au stade 4 de l'exemple préparatif 11 à partir de 3 g du produit de l'exemple préparatif 18, 765 mg de soude 50 % huile et 1,96 ml de 4 méthoxythiophénol dans du tétrahydrofuranne.

On obtient ainsi 3,08 g de produit attendu (amorphe).

### EXEMPLE 20 : 1-((6-chloro-1,3-benzodioxol-5-yl)méthyl)-4-((4-méthoxyphényl)thio)-2-méthyl-1H-imidazole-5-carboxylate de méthyle

On procède comme à l'exemple préparatif 12 à partir de 1 g du produit de l'exemple préparatif 19, 5 g d'oxyde de manganèse, 500 mg de cyanure de sodium, 300 µl d'acide acétique, 100 cm³ de méthanol et 20 cm³ de chlorure de méthylène.

On obtient ainsi 410 mg de produit attendu Pf = 152°C.

### EXEMPLE 21 : Acide 1-((6-chlore-1,3-benzodioxol-5-yl)méthyl)-4-((4-méthoxyphényl)thio)-2-méthyl-1H-imidazole-5-carboxylique

On procède comme à l'exemple 13 à partir de 740 mg du produit de l'exemple préparatif 12, 25 ml de soude 1N et 25 ml d'éthanol.

On obtient ainsi 521 mg de produit attendu Pf = 200-210°C.

### EXEMPLE préparatif 29 : 1-((6-chloro 1,3-benzodioxol-5-yl) méthyl) 4-((3,4-diméthoxyphényl) thio-2-(1,3-dioxolan-2-yl) 1H-imidazol-5-carboxaldéhyde.

### STADE A : 1-((6-chloro 1,3-benzodioxol-5-yl) méthyl) 4,5-dibromo 2-(1,3-dioxolan-2-yl) 1H-imidazole.

A 20 g de produit obtenu comme à l'exemple préparatif 11 stade A dans 200 ml de dichlorométhane et 500 ml d'éther, on ajoute 15,6 ml de solution 3M de bromure d'éthylmagnésium dans l'éther et agite 20 minutes à température ambiante. On ajoute 200 ml d'acide chlorhydrique N et extrait à l'acétate d'éthyle. On lave la phase organique à l'eau, sèche et évapore le solvant. On reprend l'aldéhyde intermédiaire obtenu dans 200 ml de toluène, ajoute 20 ml d'éthylène glycol et chauffe au reflux pendant 16 heures. On évapore le solvant, reprend le résidu dans une solution aqueuse saturée en bicarbonate de sodium, extrait à l'acétate d'éthyle, lave à l'eau, sèche et évapore le solvant sous pression réduite. On empâte le résidu dans l'éther isopropylique, filtre, sèche sous pression réduite et recueille 13,5 g de produit attendu.
F = 188°C.

### STADE B : 4-bromo 1-((6-chloro 1,3-benzodioxol-5-yl) méthyl) 2-(1,3-dioxolan-2-yl) 1H-imidazol 5-carboxaldéhyde.

On introduit 10 g du produit obtenu au stade A ci-dessus dans 200 ml de tétrahydrofuranne et ajoute goutte à goutte 10 ml de bromure d'éthyle magnésium en solution 3M dans l'éther On maintient sous agitation pendant 20 minutes à température ambiante. On ajoute alors en milieu réactionnel obtenu 10 ml de diméthylformamide et agite pendant 2 heures à température ambiante. On hydrolyse le milieu par addition de 200 ml d'acide chlorhydrique dilué, extrait à l'acétate d'éthyle, lave à l'eau puis sèche et récupère après empâtage dans l'éther isopropylique, 7,6 g de produit attendu utilisé tel quel pour le stade suivant.

### STADE C : 1-((6-chloro 1,3-benzodioxol-5-yl) méthyl) 4-((3,4-diméthoxyphényl) thio-2-(1,3-dioxolan-2-yl) 1H-imidazol-5-carboxaldéhyde.

On ajoute 1,1 g d'hydrure de sodium dans 3,15 ml de 3,4-diméthoxythiophénol en solution dans 200 ml de diméthylformamide et agite 20 minutes à température ambiante. On ajoute 7,6 g de produit obtenu au stade B, agite 16 heures à température ambiance, évapore le solvant sous pression réduite, reprend le résidu dans 200 ml de solution aqueuse saturée en chlorure d'ammonium et extrait au dichlorométhane. On sèche la phase organique, évapore le solvant, empâte le résidu à l'éther isopropylique, filtre et obtient 5,2 g de produit attendu. F = 149°C.

### EXEMPLE 30 : 1-((6-chloro 1,3-benzodioxol-5-yl) méthyl) 4-((3,4-diméthoxyphényl) thio-2-(1,3-dioxolan-2-yl) 1H-imidazol-5-carboxylate de méthyle.

On introduit une solution de 5,05 g du produit de l'exemple 29 dans un mélange chlorure de méthylène/méthanol (100 ml/500 ml), ajoute ensuite successivement 22 g d'oxyde de manganèse, 2,2 g de cyanure de sodium et 1,5 ml d'acide acétique puis agite pendant 72 heures à température ambiante. On filtre, lave la phase organique à l'eau, sèche, évapore le solvant et obtient après chromatographie sur silice 4,1 g de produit attendu. F = 170°C.

### EXEMPLE 31 : Acide 1-((6-chloro 1,3-benzodioxol-5-yl) méthyl) 4-((3,4-diméthoxyphényl) thio-2-(1,3-dioxolan-2-yl) 1H-imidazol-5-carboxylique.

On introduit 1 g de produit obtenu à l'exemple 30 dans 100 ml d'éthanol et ajoute 50 ml de soude 2N. On agite 16 heures à température ambiante, évapore le solvant sous pression réduite, reprend le résidu dans un mélange d'eau glacée, acidifie par de l'acide chlorhydrique 2N, extrait à l'acétate d'éthyle, sèche et évapore le solvant sous pression réduite. On récupère 650 mg de produit attendu. F = 155°C.

En opérant comme aux exemples 29 à 31 à partir des composés appropriés, on a préparé les produits des exemples suivants :

### EXEMPLE 32 : Acide 1-((6-chloro 1,3-benzodioxol-5-yl) méthyl) 4-((4-méthoxyphényl) thio-2-propyl) 1H-imidazol-5-carboxylique.

F = 174,5°C.

### EXEMPLE 33 : Acide 1-((6-chloro 1,3-benzodioxol-5-yl) méthyl) 4-((4-méthoxyphényl) thio) 2-(1-méthyléthyl) 1H-imidazol-5-carboxylique.

F = 179°C.

### EXEMPLE 34 : Acide 1-((6-chloro 1,3-benzodioxol-5-yl) méthyl) 4-((3,4-diméthoxyphényl) thio) 2-propyl) 1H-imidazol-5-carboxylique.

F = 175°C.

### EXEMPLE 35 : Acide 4-(butylthio) 1-((6-chloro 1,3-benzodioxol-5-yl) méthyl) 2-propyl) 1H-imidazol-5-carboxylique.

F = 151,8°C.

### EXEMPLE 36 : Acide 4-((2-carboxyphényl) thio) 1-((6-chloro 1,3-benzodioxol-5-yl) méthyl) 2-(1-méthyléthyl) 1H-imidazol-5-carboxylique.

F = 203°C.

### EXEMPLE 37 : Acide 1-((6-chloro 1,3-benzodioxol-5-yl) méthyl) 2-(1-méthyléthyl) 4-(phénylthio) 1H-imidazol-5-carboxylique.

rf = 0,2 (CH₂Cl₂-CH₃OH 95-5)

### EXEMPLE 38 : Sel de potassium de l'acide 1-((6-chloro 1,3-benzodioxol-5-yl) méthyl) 4-((3,4-diméthoxyphényl) thio) 2-(1-hydroxy 1-méthyléthyl) 1H-imidazol-5-carboxylique.

L'acide obtenu comme indiqué ci-dessus est dissous dans l'isopropanol à chaud puis on ajoute goutte à goutte une solution de potasse dans l'isopropanol jusqu'à pH basique. Après 4 heures d'agitation à température ambiante, on essore le précipité puis le lave à l'isopropanol à l'éther isopropylique, le sèche et obtient le produit attendu.
rf = 0,70 (AcOEt)

En opérant comme à l'exemple 4 à partir des composés appropriés, on a préparé les produits des exemples suivants :

### EXEMPLE 39 : Acide 2-butyl 1-((6-chloro 1,3-benzodioxol-5-yl) méthyl) 4-(méthylthio) 1H-imidazol-5-carboxylique.

F = 177°C.

### EXEMPLE 40 : Acide 1-((6-chloro 1,3-benzodioxol-5-yl) méthyl) 2-éthyl 4-((4-méthoxyphényl) thio) 1H-imidazol-5-carboxylique.

F = 172°C.

### EXEMPLE 41 : Acide 1-((6-chloro 1,3-benzodioxol-5-yl) méthyl) 4-(méthylthio) 2-propyl 1H-imidazol-5-carboxylique.

F = 196°C.

### EXEMPLE 42 : Acide 4-((1,3-benzodioxol-5-yl) thio) 1-((6-chloro 1,3-benzodioxol-5-yl) méthyl) 2-propyl 1H-imidazol-5-carboxylique.

F = 203 °C.

### EXEMPLE préparatif 43 : 1-((6-chloro 1,3-benzodioxol-5-yl) méthyl) 2-propyl 1H-imidazol-4,5-dicarboxylate d'éthyle.

On dissout 2,54 g de 2-propyl 1H-imidazol 4,5-dicarboxylate d'éthyle préparé comme indiqué dans Bio. Org. Med. Chem. Letters Vol. 4 N°1 (177-182) 1994, dans 50 ml de diméthylformamide et ajoute 2,5 g de carbonate de potassium, 2,5 g de chlorure de 6-chloropipéronyl puis 2,5 g d'iodure de sodium. On chauffe à 80°C la suspension obtenue pendant 1 heure, verse le milieu réactionnel dans l'eau glacée, extrait à l'acétate d'éthyle, lave à l'eau, sèche, élimine le solvant, chromatographie le résidu sur silice (éluant : CH₂Cl₂-AcOEt 80-20) et obtient 4,4 g de produit actendu.
rf = 0,36 (CH₂Cl₂-AcOEt 80-20).

### EXEMPLE préparatif 44 : 1-((6-chloro 1,3-benzodioxol-5-yl) méthyl) 4-formyl 2-propyl 1H-imidazol-5-carboxylate d'éthyle.

On refroidit à -78°C 3,3 g de produit obtenu à l'exemple 43 dans 40 ml de tétrahydrofuranne et ajoute 6,5 ml d'hydrure diisobutylaluminium et agite 2 heures. On verse le milieu réactionnel dans une solution aqueuse saturée en chlorure d'ammonium, extrait à l'acétate d'éthyle, lave à l'eau, sèche, élimine le solvant, chromatographie le résidu sur silice (éluant : CH₂Cl₂-AcOEt 80-20) et obtiens 2,8 g de produit attendu. F = 105°C.

### EXEMPLE 48 : 2-butyl 1-((6-chloro 1,3-benzodioxol-5-yl) méthyl) 4-(cyclohexylthio) 1H-imidazol-5-carboxylate d'éthyle.

### STADE A : 2-butyl 1-((6-chloro 1,3-benzodioxol-2-yl) méthyl) 4-(méthylsulfinyl) 1H-imidazol 5-carboxylate d'éthyle.

On dissout 10 g de l'ester éthylique obtenu à partir du produit de l'exemple 39 dans 500 ml de dichlorométhane, refroidit à 0°C et ajoute 6,6 g d'acide métachloroperbenzoïque et agite 2 heures et demie. On lave le milieu réactionnel avec une solution de bicarbonate de sodium, extrait au dichlorométhylène, lave à l'eau la phase organique, la sèche et évapore le solvant sous pression réduite. On obtient 12 g de produit brut que l'on chromatographie sur silice (éluant : acétate d'éthyle-cyclohexane 7-3 puis dichlorométhaneméthanol 85-15). On obtient 8,49 g de produit attendu.
F = 103°C.

### STADE B : 2-butyl 1-((6-chloro 1,3-benzodioxol-5-yl) méthyl) 4-mercapto 1H-imidazol-5-carboxylate d'éthyle.

On dissout 1 g de produit obtenu au stade A dans 20 ml de dichlorométhane et ajoute 0,66 ml d'anhydride trifluoroacétique. On agite pendant 30 minutes, évapore le solvant sous pression réduite, reprend le résidu dans 10 ml de méthanol et 4 ml de triéthylamine et agite de nouveau 30 minutes à température ambiante, extrait au chloroforme, lave la phase organique avec une solution aqueuse saturée en chlorure d'ammonium, sèche et évapore le solvant sous pression réduite. On recristallise le produit brut dans l'éther et recueille 490 mg de produit attendu. F = 114°C.

### STADE C : 2-butyl 1-((6-chloro 1,3-benzodioxol-5-yl) méthyl) 4-(cyclohexylthio) 1H-imidazol-5-carboxylate d'éthyle.

On ajoute 125 mg d'hydrure de sodium dans une solution comprenant 950 mg de produit obtenu comme au dtade B dans 20 ml de diméthylformamide. On ajoute ensuite 0,33 ml de iodocyclohexane et agite 3 heures à température ambiante. On ajoute 20 ml d'une solution aqueuse saturée en chlorure d'ammonium, extrait au dichlorométhane, lave à l'eau, sèche et évapore le solvant sous pression réduite. Après chromatographie sur silice (éluant : CH₂Cl₂), on obtient 850 mg de produit attendu.
rf = 0,43 (CH₂Cl₂).

### EXEMPLE 49 : Acide 2-butyl 1-((6-chloro 1,3-benzodioxol-5-yl) méthyl) 4-(cyclohexylthio) 1H-imicazol-5-carboxylique.

On mélange 800 mg de produit obtenu à l'exemple 48 dans 50 ml de soude 2N et 50 ml d'éthanol et ajoute du tétrahydrofuranne jusqu'à complète dissolution. On agite 48 heures à température ambiante, évapore les solvants organiques sous pression réduite, acidifie la solution jusqu'à pH 1 à l'aide d'acide chlorhydrique 2N, essore le précipité formé, le lave à l'eau, le sèche à 40°C sous pression réduite et récupère 317 mg de produit attendu après recristallisation dans le méthanol. F = 257°C.

En opérant comme à l'exemple 49 à partir des composés appropriés, on a préparé les produits des exemples suivants :

### EXEMPLE 50 : Acide 2-butyl 4-((2-carboxyéthényl) thio) 1-((6-chloro 1,3-benzodioxol-5-yl) méthyl) 1H-imidazol-5-carboxylique.

F = 212°C.

### EXEMPLE 51 : Acide 2-butyl 1-((6-chloro 1,3-benzodioxol-5-yl) méthyl) 4-((1-méthyléthyl) thio) 1H-imidazol-5-carboxylique.

F = 160°C.

### EXEMPLE 52 : Acide 2-butyl 1-((6-chloro 1,3-benzodioxol-5-yl) méthyl) 4-((4-hydroxybutyl) thio) 1H-imidazol-5-carboxylique.

F = 135°C.

### EXEMPLE 53 : Acide 2-butyl 4-((carboxyméthyl) thio) 1-((6-chloro 1,3-benzodioxol-5-yl) méthyl) 1H-imidazol-5-carboxylique.

F = 165,8°C.

### EXEMPLE 54 : Acide 2-butyl 4-((3-carboxypropyl) thio) 1-((6-chloro 1,3-benzodioxol-5-yl) méthyl) 1H-imidazol-5-carboxylique.

F = 140°C.

### EXEMPLE préparatif 58 : Acide 1-((6-chloro 1,3-benzodioxol-5-yl) méthyl) 4-((4-méthoxyphényl) thio) 2-propyl 1H-imidazol-5-acétique.

On opère comme à l'exemple 49 en utilisant 290 mg de produit obtenu à l'exemple précédent, 10 cm³ de solution aqueuse d'hydroxyde de sodium 2N et 10 cm³ de méthanol. Après recristallisation dans l'acétate d'éthyle, on obtient 193 mg de produit attendu. F = 187°C.

### EXEMPLE préparatif 59 : (E) 1-((6-chloro 1,3-benzodioxol-5-yl) méthyl) 4-(2-phényléthényl) 2-propyl 1H-imidazol-5-carboxylate d'éthyle.

On ajoute 150 mg d'hydrure de sodium dans une suspension comprenant 1,2 g de chlorure de benzyltriphényl phosphonium dans 30 ml de tétrahydrofuranne. On agite 30 minutes, refroidit à +10°C et ajoute 1 g de l'aldéhyde obtenu à l'exemple préparatif 44 en solution dans du tétrahydrofuranne et poursuis l'agitation 2 heures à température ambiante, filtre, lave la phase organique au tétrahydrofuranne, élimine le solvant sous pression réduite et chromatographie le résidu sur silice (éluant : CH₂Cl₂-AcOEt 80-20). on obtient 920 mg de produit attendu.
F = 161°C.

### EXEMPLE préparatif 60 : Acide (E) 1-((6-chloro 1,3-benzodioxol-5-yl) méthyl) 4-(2-phényléthényl) 2-propyl 1H-imidazol-5-carboxylique.

On agite pendant 16 heures à température ambiante 400 mg de produit obtenu à l'exemple 59 en solution dans 50 ml d'éthanol et 50 ml de tétrahydrofuranne et 10 ml de soude 2N. On élimine le solvant sous pression réduite, reprend à l'eau, filtre, acidifie par addition d'acide chlorhydrique 2N, essore le précipité, le lave à l'eau puis à l'éthanol, le sèche à 50°C sous pression réduite et récupère 250 mg de produit attendu. F = 230°C.

### EXEMPLE 61 : Acide 1-((6-chloro 1,3-benzodioxol-5-yl) méthyl) 4-(2-phényléthyl) 2-propyl 1H-imidazol-5-carboxylique.

On met en solution 490 mg de produit obtenu à l'exemple préparatif 58 dans un mélange comprenant 50 ml d'éthanol et 50 ml d'acétate d'éthyle, ajoute 50 mg d'oxyde de platine et hydrogène sous 200 g de pression. On essore le catalyseur, élimine le solvant sous pression réduite, chromatographie le résidu sur silice (éluant : CH₂Cl₂-AcOEt 95-5) et obtient 320 mg de 1-((6-chlore 1,3-benzodioxol-5-yl) méthyl) 4-(2-phényléthyl) 2-propyl 1H-imidazol 5-carboxylate d'éthyle de rf = 0,22 (CH₂Cl₂-AcOEt 95-5) et 80 mg de produit totalement réduit de 1-((6-chloro 1,3-benzodioxol-5-yl) méthyl) 4-(2-cyclohexyléthyl) 2-propyl 1H-imidazol 5-carboxylate d'éthyle de
rf = 0,35.

On dissout 300 mg de l'isomère partiellement réduit dans 40 ml d'éthanol, ajoute 5 ml de soude 2N, agite 16 heures à température ambiante, acidifie par addition d'acide chlorhydrique 2N, élimine le solvant sous pression réduite, essore le précipité, le lave à l'eau, le sèche à 60°C sous pression réduite et recueille après empâtage à l'éther isopropylique, 240 mg de produit attendu. F = 204°C.

En opérant comme indiqué aux exemples préparatifs 59 et 60, à partir des composés appropriés, on a préparé l'ester et l'acide de l'exemple correspondant :

### EXEMPLE 62 : Acide 1-((6-chloro 1,3-benzodioxol-5-yl) méthyl) 4-(2-cyclohexyléthyl) 2-propyl 1H-imidazol-5-carboxylique.

F ≈ 150°C.

### EXEMPLE 63 : 4-(((4-(2-carboxyéthyl) phényl) thio) méthyl) 1-((6-chloro 1,3-benzodioxol-5-yl) méthyl) 2-propyl 1H-imidazol-5-carboxylate d'éthyle.

On dissout 500 mg de produit obtenu à l'exemple 45 dans 50 ml de dichlorométhane et ajoute 0,8 ml de N-éthyl diisopropylamine. On refroidit à 0°C, ajoute lentement 0,15 ml de chlorure de mésyle, agite 1 heure et ajoute 360 mg de 4-(2-carboxyéthyl) phénylthiol et agite 2 heures. On lave à l'eau, sèche, élimine le solvant sous pression réduite, chromatographie le résidu sur silice (éluant : CH₂Cl₂-CH₃OH 95-5) et obtient 570 mg de produit attendu. F = 174°C.

### EXEMPLE 64 : Acide 4-(((4-(2-carboxyéthyl) phényl) thio) méthyl) 1-((6-chloro 1,3-benzodioxol-5-yl) méthyl) 2-propyl 1H-imidazol-5-carboxylique.

On dissout 540 mg de l'ester obtenu à l'exemple 63 dans 20 ml d'éthanol et 10 ml de soude 2N et agite 5 heures à température ambiante. On évapore le solvant sous pression réduite, filtre la phase aqueuse, l'acidifie à l'aide d'acide acétique, essore le précipité, le lave à l'eau, le sèche à 50°C sous pression réduite. On reprend le résidu dans l'éther isopropylique, le sèche et obtient 420 mg de produit attendu.
F = 171°C.

En opérant comme indiqué aux exemples 63 et 64 à partir des composés appropriés, on a préparé les esters et les acides des exemples suivants :

### EXEMPLE 65 : Acide 1-((6-chloro 1,3-benzodioxol-5-yl) méthyl) 4-(((3,4-diméthoxyphényl) thio) méthyl) 2-propyl 1H-imidazol-5-carboxylique.

F = 215°C.

### EXEMPLE 66 : Acide 1-((6-chloro 1,3-benzodioxol-5-yl) méthyl) 4-(((4-(1-méthyléthyl) phényl) thio) méthyl) 2-propyl 1H-imidazol-5-carboxylique.

F = 230°C.

### EXEMPLE 67 : Acide 1-((6-chloro 1,3-benzodioxol-5-yl) méthyl) 4-((4-morpholinyl) méthyl) 2-propyl 1H-imidazol-5-carboxylique.

F = 104°C.

### EXEMPLE 68 : Acide 1-((6-chloro 7-(1,2-dioxo 2-éthoxyéthyl) 1,3-benzodioxol-5-yl) méthyl) 4-((3-diméthoxyphényl) thio) 2-propyl 1H-imidazol 5-carboxylique.

On dissout 350 mg de l'acide obtenu à l'exemple 34 dans 30 ml de tétrahydrofuranne, refroidit à -78°C et ajoute 1,05 ml de n-butyllithium. On agite 5 minutes, ajoute 0,48 ml d'oxolate de diéthyle et agite 1 heure à température ambiante. On hydrolyse par addition d'eau, acidifie par addition d'acide chlorhydriaue 2N, extrait à l'acétate d'éthyle, lave la phase organique à l'eau, sèche, élimine le solvant, chromatographie le résidu sur silice (éluant : CH₂Cl₂-CH₃OH 95-5) et obtient 210 mg de produit attendu.
rf = 0,25 ( CH₂Cl₂-CH₃OH 95-5).

### EXEMPLE 69 : Sel de potassium de l'acide 1-((6-chloro 7-(carboxycarbonyl) 1,3-benzodioxol-5-yl) méthyl) 4-((3,4-diméthoxyphényl) thio) 2-propyl 1H-imidazol 5-carboxylique.

On ajoute 5 ml de soude 2N à 190 mg de produit obtenu à l'exemple 68 en solution dans 10 ml d'éthanol et agite 16 heures à température ambiante. On élimine l'éthanol sous pression réduite, acidifie la phase aqueuse par addition d'acide chlorhydrique 2N, essore le précipité, le lave à l'eau, le sèche sous pression réduite. On reprend le résidu dans 1'éthanol, alcalinise par addition de potasse dans 10 ml d'isopropanol, essore le produit cristallisé et le sèche à 50°C sous pression réduite. On obtient 70 mg de produit attendu. F > 260°C.

En opérant comme à l'exemple 54, au départ des produtis appropriés, on a préparé les produits suivants :

### EXEMPLE 70 : acide 4-((3-carboxypropyl) thio) 1-((6-chlore 1,3-benzodioxol-5-yl) méthyl) 2-propyl 1H-imidazol 5-carboxylique.

F = 154°C.

### EXEMPLE 71 : acide 4-((7-carboxyheptyl) thio) 1-((6-chloro 1,3-benzodioxol-5-yl) méthyl) 2-propyl 1H-imidazol 5-carboxylique.

F = 133°C.

### EXEMPLE 72 de composition pharmaceutique.

On a préparé des comprimés répondant à la formule suivante :

| | |
|---|---|
| Produit de l'exemple 2 | 50 mg |
| Excipient pour un comprimé terminé à | 200 mg |

(détail de l'excipient : lactose, talc, amidon, stéarate de magnésium).

### RESULTATS PHARMACOLOGIQUES :

### ETUDE DE L'ACTIVITE SUR RECEPTEUR B DE L'ENDOTHELINE

On effectue une préparation membranaire à partir de cortex postérieur plus cervelet de rat. Le tissu est broyé au POLYTRON dans un tampon Tris 50 mM pH = 7,4.

Après 30 minutes à 25 °C (B.M.) l'homogénat est centrifugé à 30000 g pendant 15 minutes (2 centrifugations avec reprise intermédiaire dans le tampon Tris pH. 7,4).

Les culots sont remis en suspension dans un tampon d'incubation (Tris 25 mM, pepstatine A 5 microg/ml, aprotinine 3 microg/ml, PMSF 0,1 mM, EDTA 3mM, EGTA 1mM pH 7,4).

On répartit des aliquotes de 2 ml dans des tubes à hémolyse et ajoute de la 125I Endothéline (environ 50000 dpm/tube) et le produit à étudier. (Le produit est d'abord testé à 3*10⁻⁵ M en triple). Lorsque le produit testé déplace de plus de 50 % la radioactivité liée spécifiquement au récepteur, il est testé à nouveau selon une gamme de 7 concentrations afin de déterminer la concentration qui inhibe de 50 % la radioactivité liée spécifiquement au récepteur. On détermine ainsi la concentration inhibitrice 50 %.

La liaison non spécifique est déterminée par addition d'endothéline à 10⁻⁶ M (en triple). On incube à 25 °C pendant 60 minutes, remet au bain-marie à 0 °C, pendant 5 minutes, filtre sous pression réduite, rince au tampon Tris 7,4 et compte la radioactivité en présence du scintillant Triton.

Le résultat est exprimé directement en concentration inhibitrice 50 % (CI50), c'esc-à-dire en concentration de produit étudié exprimée en nM, nécessaire pour déplacer 50 % de la radioactivité spécifique fixée sur le récepteur étudié.

### Résultat :

Les CI50 trouvées pour les produits des exemples sont données dans le tableau I ci-après, en nanomoles.

### Résultats :

**TABLEAU I**

| Produit des exemples | Récepteur B de l'sndothélin CI/50 en nanomoles |
|---|---|
| 10 | 250 |
| 21 | 350 |

### ETUDE DE L'ACTIVITE SUR RECEPTEUR A DE L'ENDOTHELINE

On effectue une préparation membranaire à partir de coeur (ventricules) de rat. Le tissu est broyé au POLYTRON dans un tampon Tris 50 mM pH = 7,4.

Après 30 minutes à 25 °C (B.M.) l'homogénat est centrifugé à 30000 g pendant 15 minutes (2 centrifugations avec reprise intermediaire dans le tampon Tris pH. 7,4).

Les culots sont remis en suspension dans un tampon d'incubation (Tris 25 mM, pepstatine A 5 mlcrog/ml, aprotinine 3 microg/ml, PMSF 0,1 mM, EDTA 3mM, EGTA 1mM pH 7,4).

On répartit des aliquotes de 2 ml dans des tubes à hémolyse et ajoute de la 125I Endothéline (environ 50000 dpm/tube) et le produit à étudier. (Le produit est d'abord testé à 3*10⁻⁵ M en triple). Lorsque le produit testé déplace de plus de 50 % la radioactivité liée spécifiquement au récepteur, il est testé à nouveau selon une gamme de 7 concentrations afin de déterminer la concentration qui inhibe de 50 % la radioactivité liée spécifiquement au récepteur. On détermine ainsi la concentration inhibitrice 50 %.

La liaison non spécifique est déterminée par addition d'endothéline à 10⁻⁶ M (en triple). On incube à 25 °C pendant 60 minutes, remet au bain-marie à 0 °C, pendant 5 minutes, filtre sous pression réduite, rince au tampon Tris 7,4 et compte la radioactivité en présence du scintillant Triton.

Le résultat est exprimé directement en concentration inhibitrice 50 % (CI50), c'est-à-dire en concentration de produit étudié exprimée en nM, nécessaire pour déplacer 50 % de la radioactivité spécifique fixée sur le récepteur étudié.

### Résultat :

Les CI50 trouvées pour les produits des exemples sont données dans le tableau I ci-après, en nanomoles.

### Résultats :

**TABLEAU I**

| Produit des exemples | Récepteur A de l'endothéline CI/50 en nanomoles |
|---|---|
| 10 | 91 |
| 21 | 110 |

## Revendications

1. Produits répondant à la formule (I) : 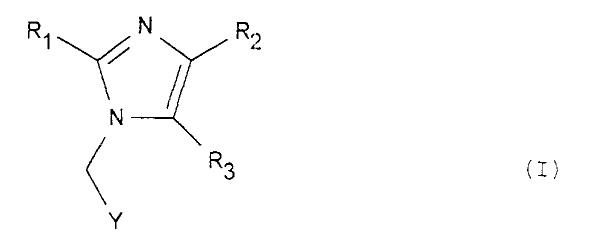 dans laquelle :
R₁ est choisi parmi les radicaux méthyle, éthyle, propyle, butyle linéaires ou ramifiés éventuellement substitués par un radical hydroxy ou alcoxy, et les radicaux alkylthio renfermant jusqu'à 6 atomes de carbone, dioxolanne et dioxane,
R₂ est choisi parmi les radicaux :
a) 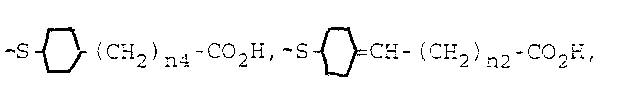 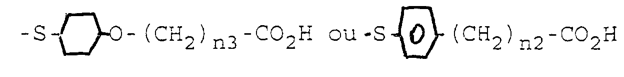 dans lequel n₄ représente le nombre 2 ou 3, n₂ représente un nombre entre 0 et 3 et n₃ représente un nombre entre 1 et 3.
b) les radicaux alkylthio, alkénylthio, cycloalkylthio, cycloalkyle, cycloalkylalkyle, cycloalkylalkényle, phényle, phénoxy, phénylthio, benzyle, benzoyle, phénylthioalkyle, radicaux dans lesquels les radicaux alkyle et alkényle renferment au plus 4 atomes de carbone, les radicaux cycloalkyle et cycloalkényle renferment au plus 6 atomes de carbone et les radicaux alkyle, alkényle, cycloalkyle et phényle sont éventuellement substitués par un ou plusieurs radicaux choisis parmi les radicaux hydroxy, carboxy, alkyle, alkényle et alcoxy, ces trois derniers radicaux étant eux-mêmes éventuellement substitués par un radical carboxy et les radicaux phényle étant de plus éventuellement substitués sur deux carbones adjacents par un radical dioxole,
R₃ est choisi parmi les radicaux carboxy libre ou estérifié, tétrazolyle ou sulfonylurée,
Y représente un radical phényle substitué par deux radicaux formant ensemble un radical dioxole et éventuellement par un ou plusieurs radicaux choisis parmi les atomes d'halogène et les radicaux hydroxy, alcoxy et carboxy,
l'atome de soufre étant éventuellement oxydé sous forme de sulfoxyde ou de sulfone, ainsi que les produits suivants :
- acide 2-butyl 1-((6-chloro 1,3-benzodioxol-5-yl) méthyl) 4-(4-méthoxyphényl) méthylthio) 1H-imidazole 5-carboxylique
- acide 1-((6-chloro 1,3-benzodioxol-5-yl) méthyl) 2,4-bis (((4-méthoxyphényl) méthyl) thio) 1H-imidazole 5-carboxylique
- acide 1-((6-chloro 1,3-benzodioxol-5-yl) méthyl) 4-(2-phényléthyl) 2-propyl 1H-imidazol-5-carboxylique,
lesdits produits de formule (I) et les produits définis ci-dessus étant sous toutes les formes isomères possibles racémiques, énantiomères et diastéréoisomères, ainsi que les sels d'addition avec les acides minéraux et organiques ou avec les bases minérales et organiques desdits produits de formule (I) et des produits définis ci-dessus .

2. Produits tels que définis à la revendication 1 , répondant aux formules suivantes :
- acide 2-butyl 1-((6-chloro 1,3-benzodioxol-5-yl) méthyl) 4-((4-méthoxyphényl) méthylthio) 1H-imidazole 5-carboxylique
- acide 2-butyl 1-((6-chlore 1,3-benzodioxol-5-yl) méthyl) 4-((4-méthoxyphényl) thio) 1H-imidazole 5-carboxylique
- acide 1-((6-chloro 1,3-benzodioxol-5-yl) méthyl) 2,4-bis (((4-méthoxyphényl) méthyl) thio) 1H-imidazole 5-carboxylique
- acide 1-((6-chloro 1,3-benzodioxol-5-yl) méthyl) 4-(4-(méthoxyphényl) thio) 2-(propylthio) 1H-imidazole 5-carboxylique,
- acide 1-((6-chloro 1,3-benzodioxol-5-yl) méthyl) 4-((3,4-diméthoxyphényl) thio) 2-propyl 1H-imidazole -carboxylique,
- acide 2-butyl 1-((6-chloro 1,3-benzodioxol-5-yl) méthyl) 4-(cyclohexylthio) 1H-imidazole 5-carboxylique,
- acide 2-butyl 4- ((3-carboxypropyl) thio) 1-((6-chloro 1,3-benzodioxol-5-yl) méthyl) 1H-imidazole 5-carboxylique,
- acide 4-(((4-(2-carboxyéthyl) phényl) thio) méthyl) 1-((6-chloro 1,3-benzodioxol-5-yl) méthyl) 2-propyl 1H-imidazol 5-carboxylique,
- acide 4-(3-carboxypropyl) thio) 1-((6-chloro 1,3-benzodioxol-5-yl) méthyl) 2-propyl 1H-imidazol 5-carboxylique,
- acide 4-((7-carboxyheptyl) thio) 1-((6-chloro 1,3-benzodioxol-5-yl) méthyl) 2-propyl 1H-imidazol 5-carboxylique.
- acide 1-((6-chloro 1,3-benzodioxol-5-yl) méthyl) 4-(2-phényléthyl) 2-propyl 1H-imidazol-5-carboxylique.

3. A titre de médicaments, les produits tels que définis aux revendications 1 et 2, ainsi que les sels d'addition avec les acides minéraux et organiques ou avec les bases minérales et organiques pharmaceutiquement acceptables de ces produits.

4. Les compositions pharmaceutiques contenant à titre de principe actif, l'un au moins des médicaments tels que définis à la revendication 3.

5. Utilisation des produits de formule (I) telle que définie aux revendications 1 et 2, pour la préparation de compositions pharmaceutiques destinées au traitement d'affections résultant d'une stimulation anormale des récepteurs de l'endothéline et notamment destinées au traitement de l'hypertension induite par l'endothéline, de tous les spasmes vasculaires, au traitement des post-hémorragies cérébrales, des insuffisances rénales, de l'infarctus du myocarde, à la prévention des resténoses post-angioplastie.

6. Procédé de préparation des produits, tels que définis aux revendications 1 et 2, **caractérisé en ce que** :
soit l'on soumet un composé de formule (II) : 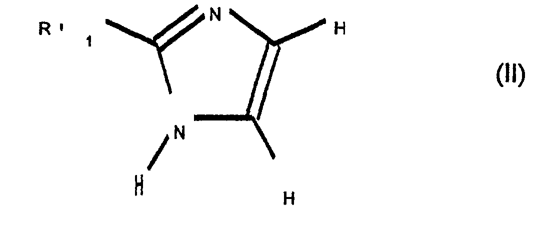 dans laquelle R'₁ a la signification indiquée à la revendication 1 pour R₁, dans laquelle les éventuelles fonctions réactives sont éventuellement protégées par des groupements protecteurs
à une réaction avec un composé de formule (III) : 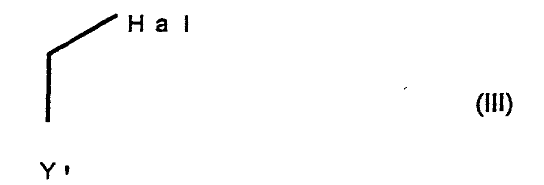 dans laquelle Hal représente un atome d'halogène, et
Y' a la signification indiquée à la revendication 1 pour Y,
dans laquelle les éventuelles fonctions réactives sont éventuellement protégées par des groupements protecteurs,
pour obtenir le produit de formule (IV₁) : 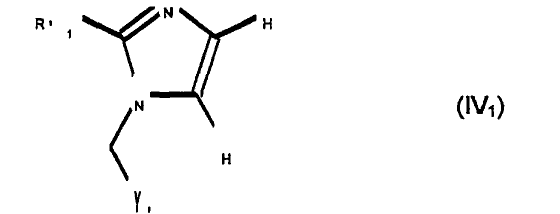 dans laquelle R'₁ et Y' ont les significations indiquées ci-dessus, produit de formule (IV₁) que l'on peut soumettre à une réaction d'halogénation, pour obtenir le produit de formule (IV₂) : 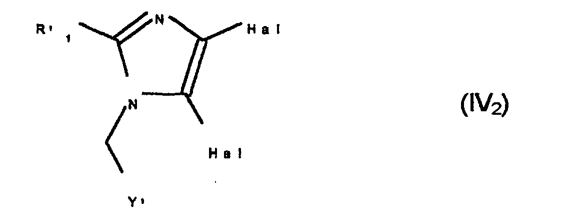 dans laquelle R'₁, Hal et Y' ont les significations indiquées ci-dessus,
soit l'on soumet un composé de formule (VIII) : 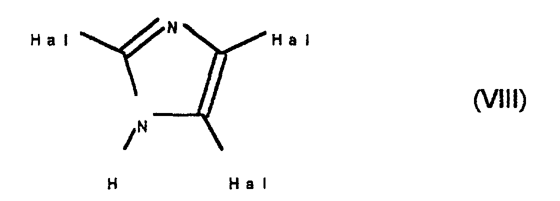 dans laquelle Hal a la signification indiquée ci-dessus,
soit à une réaction avec le composé de formule (III) telle que définie ci-dessus, soit à l'action d'un groupement protecteur P, pour obtenir un produit de formule (IX) : 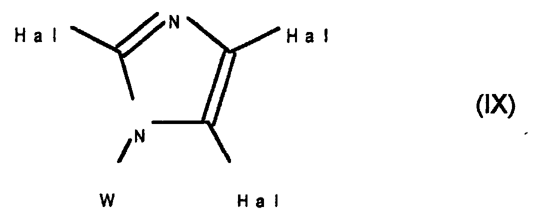 dans laquelle Hal a la signification indiquée ci-dessus et W représente soit -CH₂-Y' avec Y' tel que défini ci-dessus,
soit P qui représente un groupement protecteur de l'atome d'azote, produit de formule (IX) que l'on soumet à une réaction d'échange halogène-métal puis à une réaction avec un électrophile de formule (X), (X'), (XI), (XII) ou (XII') :
L₁-CHO (X)
L₁-CHO-Cl (X')
(-S-L₁) ₂ (XI)
L₁SO₂-S-L'₁ (XII)
L₁SO₂-Cl (XII')
dans lesquelles L₁ et L'₁ identiques ou différents, représentent un radical alkyle ou alkényle tels que définis ci-dessus, pour obtenir un produit de formule (XIII) : 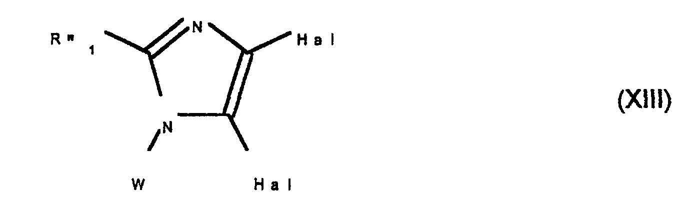 dans laquelle Hal et W ont les significations indiquées ci-dessus, R"₁ représente un radical alkyl-carbonyle, alkyl-hydroxyalkyle ou alkylthio dans lesquels le radical alkyle a la signification indiquée ci-dessus et dans lesquels les éventuelles fonctions réactives sont éventuellement protégées par des groupements protecteurs, produits de formules (IV₂) et (XIII) que l'on peut soumettre à une réaction d'échange halogène-métal sur l'un des atomes d'halogène puis à une réaction avec CO₂ ou DMF ou un composé électrophile de formule (Vₐ), (V_{b}), (VIₐ), (VI_{b}) ou (VI_{c}) :
(-S-Z₁)₂ (Vₐ) ou MeSO₂SZ₁ (V_{b})
ou 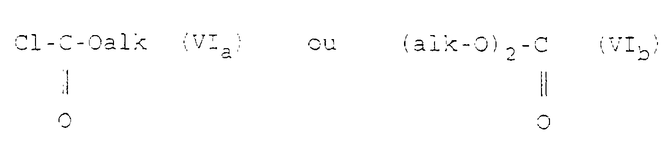 ou 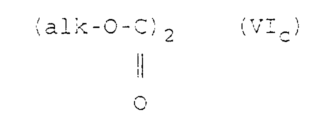 dans lesquelles Z₁ représente un radical alkyle, alkényle ou phényle, éventuellement substitués, dans lesquelles les éventuelles fonctions réactives sont éventuellement protégées par des groupements protecteurs, et alk représente un radical alkyle renfermant au plus 4 atomes de carbone,
pour obtenir respectivement le composé de formule (I₁) : 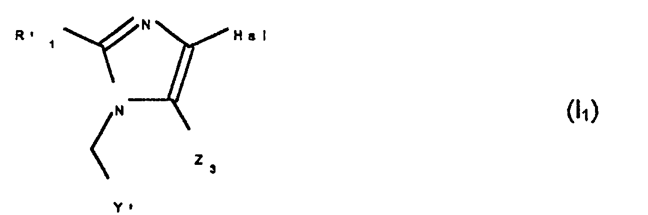 ou le composé de formule (XIV) 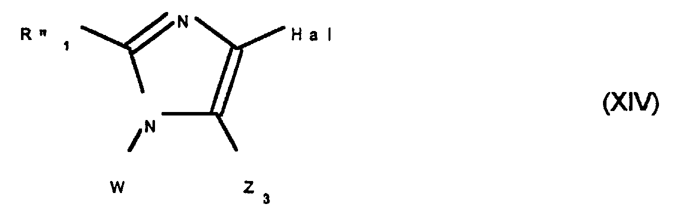 dans lesquelles R'₁, R"₁, Hal, Y' et W ont les significations indiquées ci-dessus et Z₃ représente le radical carboxy, formyle, S-Z₁ tel que défini ci-dessus, ou le radical K-O-alk
dans lequel K représente le radical 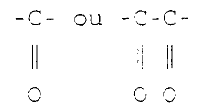 en alk a la signification indiquée ci-dessus,
composés de formule (I₁) ou (XIV) que lorsque Z₃ représente un radical formyle ou carboxy estérifié, l'on peut soumettre à une réaction avec un composé de formule (VII) :
Z₂-S-M (VII)
dans laquelle S représente un atome de soufre, M représente un métal tel que sodium, potassium ou cuivre et Z₂ représente un radical alkyle, alkényle ou phényle, éventuellement substitué, dans lesquelles les éventuelles fonctions réactives sont éventuellement protégées par des groupements protecteurs pour obtenir respectivement le composé de formule (I₂) : 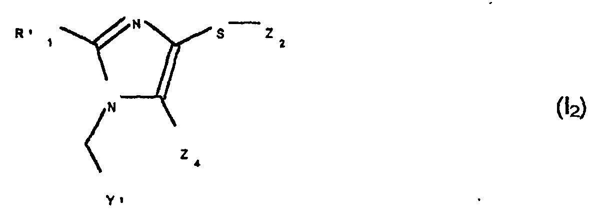 ou le composé de formule (XV) : 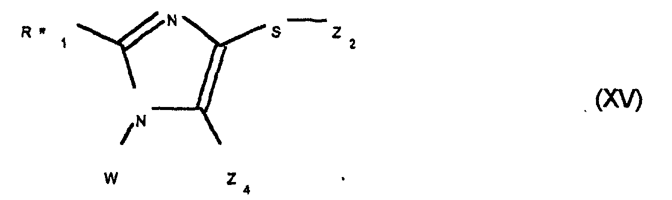 dans lesquelles R'₁, R"₁, Y', Z₂ et W ont les significations indiquées ci-dessus, et Z₄ représente le radical formyle ou carboxy estérifié, produits de formules (I₂) et (XV) que lorsque Z₄ représente le radical formyle, l'on peut soumettre à une réaction d'oxydation ou de réduction pour obtenir respectivement un produit de formule (I₃) : 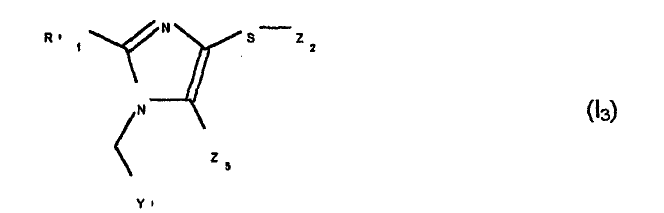 ou un produit de formule (I₄) : 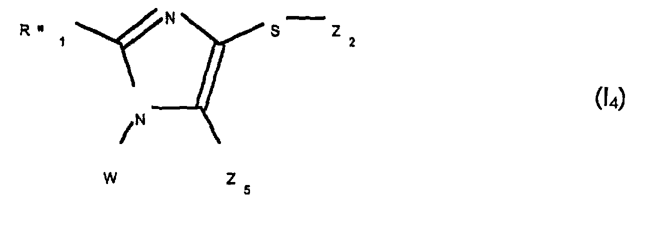 dans lesquelles R'₁, R"₁, Z₂, Y' et W ont les significations indiquées ci-dessus, et Z₅ représente le radical CH₂OH ou le radical carboxy libre ou estérifié par un radical alkyle linéaire ou ramifié renfermant au plus 6 atomes de carbone, produits de formule (XV) ou (I₄) que, dans le cas où W représente P tel que défini ci-dessus et après libération de la fonction amine bloquée par P tel que défini ci-dessus, l'on fait réagir avec le composé de formule (III) telle que définie ci-dessus, pour obtenir un produit de formule (I₅) : 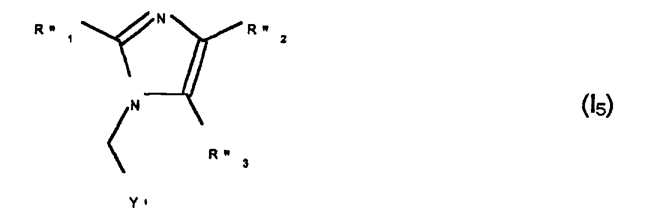 dans laquelle R"₁ et Y' ont les significations indiquées ci-dessus, et R"₂ et R"₃ représentent indifféremment l'un Z₄ ou Z₅ tels que définis ci-dessus et l'autre S-Z₂, tel que défini ci-dessus,
soit l'on soumet un composé de formule (XVI) : 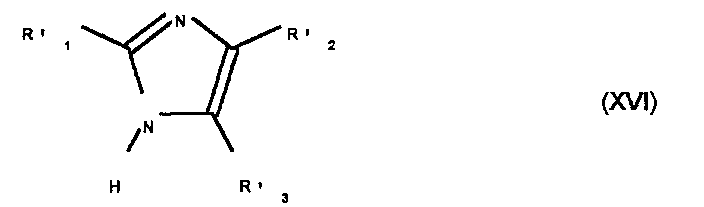 dans laquelle R'₁ a la signification indiquée ci-dessus et R'₂ et R'₃ ont les significations indiquées ci-dessus respectivement pour R₂ et R₃ dans lesquelles les éventuelles fonctions réactives sont éventuellement protégées par des groupements protecteurs, à une réaction avec le composé de formule (III) telle que définie ci-dessus, pour obtenir un produit de formule (I') : 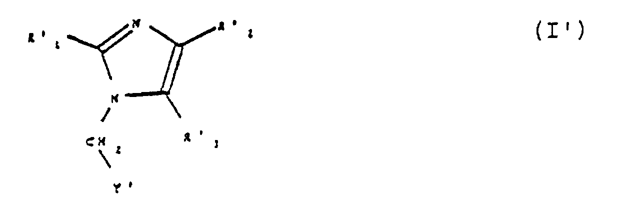 dans laquelle R'₁, R'₂, R'₃ et Y' ont les définitions indiquées ci-dessus,
soit l'on soumet un composé de formule (XVII) : 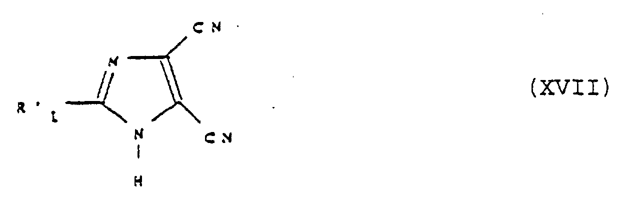 dans laquelle R'₁ a la signification indiquée précédemment, à une réaction d'oxydation pour obtenir le produit de formule (XVIII) : 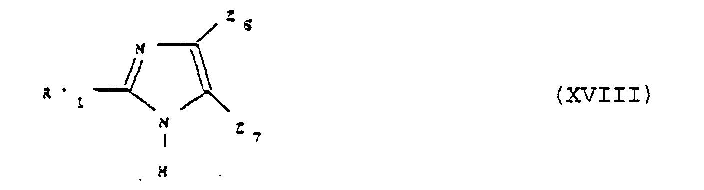 dans laquelle R'₁ a la signification indiquée précédemment et l'un ou les deux de Z₆ et Z₇ représentent un radical carboxy libre ou estérifié et le cas échéant, l'autre de Z₆ et Z₇ conservant la valeur CN, produit de formule (XVIII) que l'on fait réagir avec le composé de formule (III) telle que définie ci-dessus pour obtenir le produit de formule (I₆) : 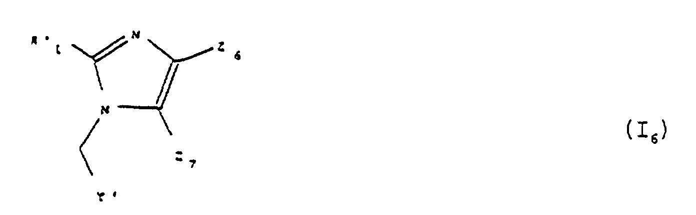 dans laquelle R'1, Y', Z₆ et Z₇ ont les significations indiquées précédemment,
produits de formules (I₁), (I₂), (I₃), (I₄), (XIV), (XV), (I₅), (I₆) et (I') qui peuvent être des produits tels que définis aux revendications 1 et 2 et que, pour obtenir des ou d'autres produits tels que définis aux revendications 1 et 2, l'on peut soumettre, si désiré et si nécessaire, à l'une ou plusieurs des réactions de transformations suivantes, dans un ordre quelconque :
a) une réaction d'estérification de fonction acide,
b) une réaction de saponification de fonction ester en fonction acide,
c) une réaction de transformation de fonction ester en fonction acyle,
d) une réaction de transformation de la fonction cyano en fonction acide,
e) une réaction de transformation de fonction acide en fonction amide, puis éventuellement en fonction thioamide,
f) une réaction de réduction de la fonction carboxy en fonction alcool,
g) une réaction de transformation de fonction alcoxy er fonction hydroxyle, ou encore de fonction hydroxyle en fonction alcoxy,
h) une réaction d'oxydation de fonction alcool en fonction aldéhyde, acide ou cétone,
i) une réaction de transformation du radical formyle en radical carbamoyle,
j) une réaction de transformation du radical carbamoyle en radical nitrile,
k) une réaction de transformation de radical nitrile en tétrazolyle,
l) une réaction d'oxydation de groupement alkylthio ou arylthio en sulfoxyde ou sulfone correspondant,
m) une réaction de transformation de fonction sulfure, sulfoxyde ou sulfone en fonction sulfoximine correspondante,
n) une réaction de transformation de fonction oxo en fonction thioxo,
o) une réaction de transformation du radical 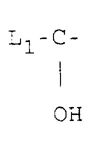 en radical 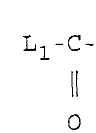 puis si nécessaire de nouveau en radical 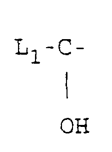 avec L₁ tel que défini ci-dessus,
p) une réaction de transformation de fonction acide en fonction 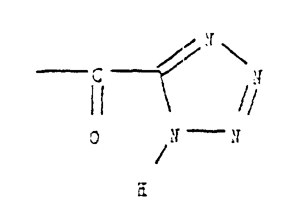
q) une réaction de transformation de la fonction β-céto-sulfoxyde en fonction α-céto thio ester,
r) une réaction de transformation d'un carbamate en urée et notamment d'un sulfonylcarbamate en sulfonylurée,
s) une réaction de transformation d'une fonction halogénée en fonction formyle ou carboxy estérifié,
t) une réaction de transformation d'un radical formyle en fonction CH₂-CO₂alk ou CH=CH-CO₂alk dans laquelle alk représente un radical alkyle renfermant de 1 à 4 atomes de carbone, puis le cas échéant, transformation en acide correspondant,
u) une réaction de transformation d'un radical formyle en radical 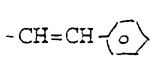 puis le cas échéant en 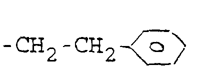 et 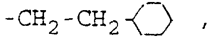
v) une réaction de transformation d'un radical formyle en radical CH₂-OH ou 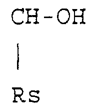 puis le cas échéant en CH₂-Rs ou transformation de 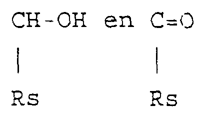 pour obtenir les radicaux alkyle ou acyle éventuellement substitués tels que définis ci-dessus pour R'₂ et R'₃,
w) une réaction d'oxydation du radical S-alk en 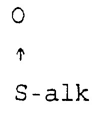 puis transformation en fonction SH et le cas échéant en S-Z₂ dans lequel alk et Z₂ ont la signification indiquée précédemment,
x) une réaction d'élimination des groupements protecteurs que peuvent porter les fonctions réactives protégées,
y) une réaction de salification par un acide minéral ou organique ou par une base pour obtenir le sel correspondant,
z) une réaction de dédoublement des formes racémiques en produits dédoublés,
lesdits produits ainsi obtenus étant sous toutes les formes isomères possibles racémiques, énantiomères et diastéréoisomères.

7. Intermédiaire de formule (IV₁) : 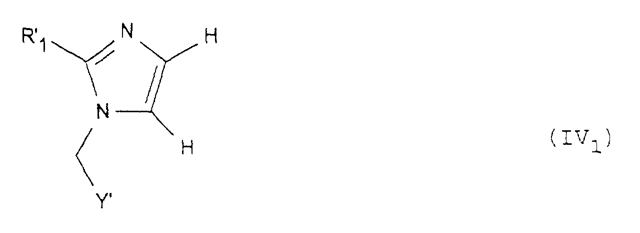 dans laquelle R'₁ et Y' ont les significations données pour
R₁ et Y dans la revendication 1 .

8. Intermédiaire de formule (IV₂) : 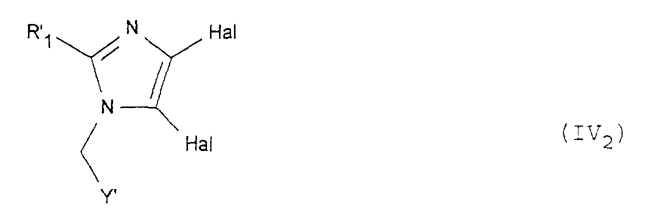 dans laquelle : R'₁ et Y' ont les significations données pour R₁ et Y dans la revendication 1, et Hal signifie halogène .

9. Intermédiaire de formule (XIII) : 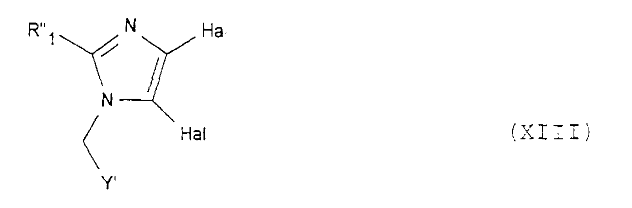 dans laquelle Y' a la signification donnée pour Y dans la revendication 1, Hal signifie halogène et R"₁ représente un radical alkyl-carbonyle, alkyl-hydroxyalkyle ou alkylthio, où le radical alkyl renferme au plus 3 atomes de carbone.

10. Intermédiaire de formule (XIV) : 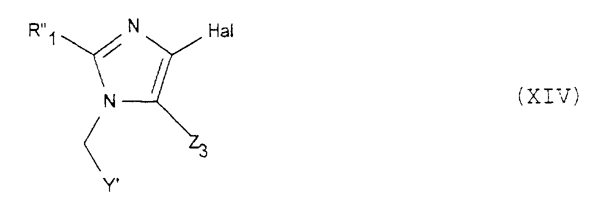 dans laquelle Y' a la signification donnée pour Y dans la revendication 1, Hal signifie halogène, R"₁ représente un radical alkyl-carbonyle, alkyl-hydroxyalkyle ou alkylthio, où le radical alkyl renferme au plus 3 atomes de carbone, Z₃ signifie un radical carboxy, formyle, ou le radical K-0-alk où alk représente un alkyle renfermant au plus 4 atomes de carbone et où K représente -CO- ou -CO-CO-.

11. Intermédiaire de formule (XV) : 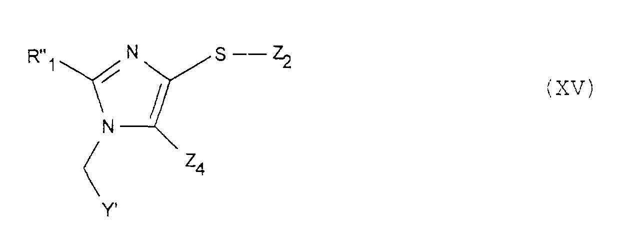 dans laquelle Y' a la signification donnée pour Y dans la revendication 1, R"₁ représente un radical alkyl-carbonyle, alkyl-hydroxyalkyle ou alkylthio, où le radical alkyl renferme au plus 3 atomes de carbone, Z₄ représente le radical formyle ou carboxy estérifié ; Z₂ représente un radical alkyle, alkényle ou phényle, éventuellement substitués par un ou plusieurs radicaux choisis parmi les radicaux hydroxy, carboxy, alkyle, alkényle et alcoxy, ces trois derniers radicaux étant eux-mêmes éventuellement substitués par un radical carboxy et les radicaux phényle étant de plus éventuellement substitués sur deux carbones adjacents par un radical dioxole.

12. Utilisation des produits de formule (I) telle que définie à la revendication 1 pour la préparation de compositions pharmaceutiques destinées au traitement d'affections résultant d'une stimulation anormale des récepteurs à l'endothéline.

## Claims

1. Products corresponding to formula (I): 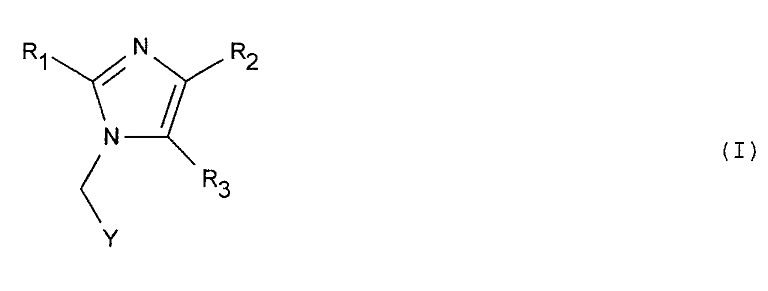 in which:
R₁ is chosen from the linear or branched methyl, ethyl, propyl, butyl radicals optionally substituted by a hydroxy or alkoxy radical, and the alkylthio radicals containing up to 6 carbon atoms, dioxolane and dioxane,
R₂ is chosen from the:
a) 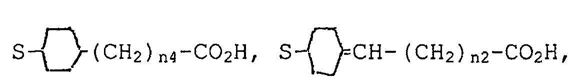 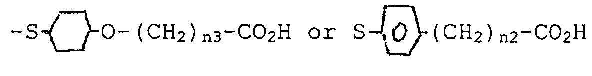 radicals in which n₄ represents the number 2 or 3, n₂ represents an integer between 0 and 3 and n₃ represents an integer between 1 and 3.
b) the alkylthio, alkenylthio, cycloalkylthio, cycloalkyl, cycloalkylalkyl, cycloalkylalkenyl, phenyl, phenoxy, phenylthio, benzyl, benzoyl, phenylthioalkyl radicals, radicals in which the alkyl and alkenyl radicals contain at most 4 carbon atoms, the cycloalkyl and cycloalkenyl radicals contain at most 6 carbon atoms and the alkyl, alkenyl, cycloalkyl and phenyl radicals are optionally substituted by one or more radicals chosen from the hydroxy, carboxy, alkyl, alkenyl and alkoxy radicals, these last three radicals being themselves optionally substituted by a carboxy radical and the phenyl radicals being in addition optionally substituted on two adjacent carbons by a dioxole radical,
R₃ is chosen from the free or esterified carboxy, tetrazolyl or sulphonylurea radicals,
Y represents a phenyl radical substituted by two radicals together forming a dioxole radical and optionally by one or more radicals chosen from the halogen atoms and the hydroxy, alkoxy and carboxy radicals,
the sulphur atom being optionally oxidized in the form of sulphoxide or sulphone, as well as the following products:
- 2-butyl 1-((6-chloro 1,3-benzodioxol-5-yl) methyl) 4-((4-methoxyphenyl) methylthio) 1H-imidazole 5-carboxylic acid
- 1-((6-chloro 1,3-benzodioxol-5-yl) methyl) 2,4-bis (((4-methoxyphenyl) methyl) thio) 1H-imidazole 5-carboxylic acid
- 1-((6-chloro 1,3-benzodioxol-5-yl) methyl) 4-(2-phenylethyl) 2-propyl 1H-imidazol-5-carboxylic acid,
said products of formula (I) and the products defined above being in all possible racemic, enantiomeric and
diastereoisomeric isomer forms, as well as the addition salts with mineral and organic acids or with mineral and organic bases of said products of formula (I) and of the products defined above.

2. Products as defined in claim 1, corresponding to the following formulae:
- 2-butyl 1-((6-chloro 1,3-benzodioxol-5-yl) methyl) 4-((4-methoxyphenyl) methylthio) 1H-imidazole 5-carboxylic acid
- 2-butyl 1-((6-chloro 1,3-benzodioxol-5-yl) methyl) 4-((4-methoxyphenyl) thio) 1H-imidazole 5-carboxylic acid
- 1-((6-chloro 1,3-benzodioxol-5-yl) methyl) 2,4-bis (((4-methoxyphenyl) methyl) thio) 1H-imidazole 5-carboxylic acid
- 1-((6-chloro 1,3-benzodioxol-5-yl) methyl) 4-(4-(methoxyphenyl) thio) 2-(propylthio) 1H-imidazole 5-carboxylic acid,
- 1-((6-chloro 1,3-benzodioxol-5-yl) methyl) 4-((3,4-dimethoxyphenyl) thio) 2-propyl 1H-imidazole-carboxylic acid,
- 2-butyl 1-((6-chloro 1,3-benzodioxol-5-yl) methyl) 4-(cyclohexylthio) 1H-imidazole 5-carboxylic acid,
- 2-butyl 4-((3-carboxypropyl) thio) 1-((6-chloro 1,3-benzodioxol-5-yl) methyl) 1H-imidazole 5-carboxylic acid,
- 4-(((4-(2-carboxyethyl) phenyl) thio) methyl) 1-((6-chloro 1,3-benzodioxol-5-yl) methyl) 2-propyl 1H-imidazol 5-carboxylic acid,
- 4-((3-carboxypropyl) thio) 1-((6-chloro 1,3-benzodioxol-5-yl) methyl) 2-propyl 1H-imidazol 5-carboxylic acid,
- 4-((7-carboxyheptyl) thio) 1-((6-chloro 1,3-benzodioxol-5-yl) methyl) 2-propyl 1H-imidazol 5-carboxylic acid.
- 1-((6-chloro 1,3-benzodioxol-5-yl) methyl) 4-(2-phenylethyl) 2-propyl 1H-imidazol-5-carboxylic acid.

3. As medicaments, the products as defined in claims 1 and 2, as well as the addition salts with pharmaceutically acceptable mineral and organic acids or with mineral and organic bases of these products.

4. Pharmaceutical compositions containing as active ingredient, at least one of the medicaments as defined in claim 3.

5. Use of the products of formula (I) as defined in claims 1 and 2, for the preparation of pharmaceutical compositions intended for the treatment of illnesses resulting from an abnormal stimulation of the endothelin receptors and in particular intended for the treatment of hypertension induced by endothelin, all vascular spasms, in post cerebral haemorrhage treatment, renal insufficiency, myocardial infarctus, in the prevention of post-angioplasty recurrence of stenosis.

6. Process for the preparation of products, as defined in claims 1 and 2, **characterized in that**:
either a compound of formula (II): 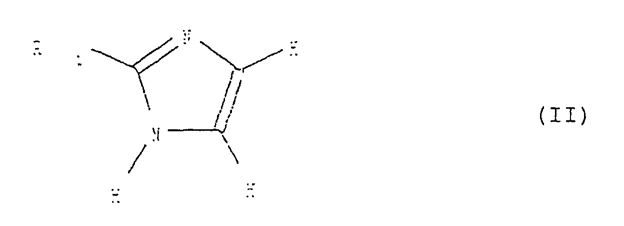 in which R'₁ has the meaning indicated in claim 1 for R₁, in which the optional reactive functions are optionally protected by protective groups,
is subjected to a reaction with a compound of formula (III): 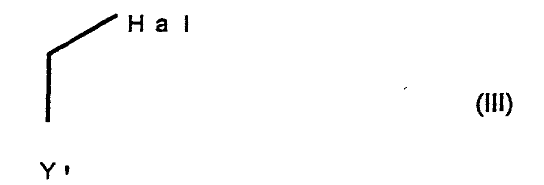 in which Hal represents a halogen atom, and
Y' has the meaning indicated in claim 1 for Y, in which the optional reactive functions are optionally protected by protective groups,
in order to obtain the product of formula (IV₁) : 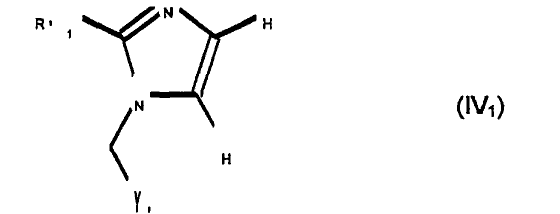 in which R'₁ and Y' have the meanings indicated above, which product of formula (IV₁) can be subjected to a halogenation reaction, in order to obtain the product of formula (IV₂): 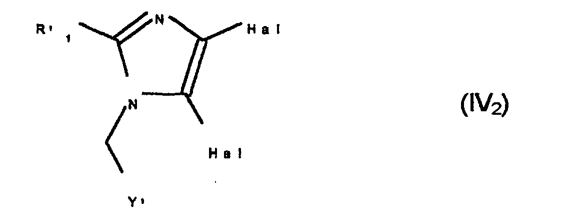 in which R'₁, Hal and Y' have the meanings indicated above,
or a compound of formula (VIII): 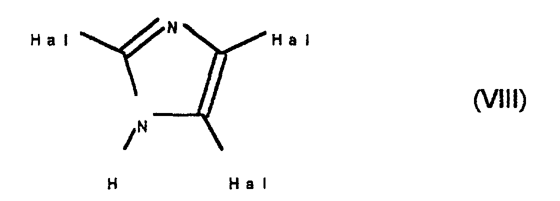 in which Hal has the meaning indicated above,
is subjected either to a reaction with the compound of formula (III) as defined above, or to the action of a protective group P, in order to obtain a product of formula (IX): 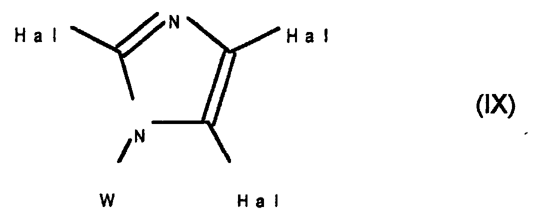 in which Hal has the meaning indicated above and W represents either -CH₂-Y' with Y' as defined above, or P which represents a protective group of the nitrogen atom, which product of formula (IX) is subjected to a halogen-metal exchange reaction then to a reaction with an electrophile of formula (X), (X'), (XI), (XII) or (XII'):
L₁-CHO (X)
L₁-CHO-Cl (X')
(-S-L₁)₂ (XI)
L₁SO₂-S-L'₁ (XII)
L₁SO₂-Cl (XII')
in which L₁ and L'₁ identical or different, represent an alkyl or alkenyl radical as defined above, in order to obtain a product of formula (XIII): 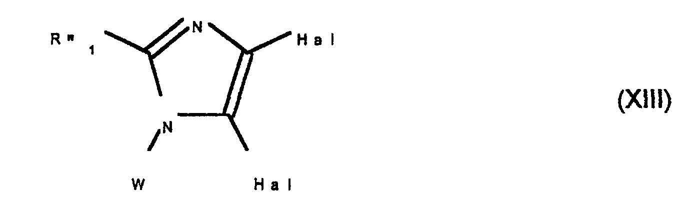 in which Hal and W have the meanings indicated above, R"₁ represents an alkyl-carbonyl, alkyl-hydroxyalkyl or alkylthio radical in which the alkyl radical has the meaning indicated above and in which the optional reactive functions are optionally protected by protective groups, which products of formulae (IV₂) and (XIII) can be subjected to a halogen-metal exchange reaction on one of the halogen atoms then to a reaction with CO₂ or DMF or an electrophilic compound of formula (Vₐ) , (V_{b}), (VIₐ), (VI_{b}) or (VI_{c}) :
(-S-Z₁)₂ (Vₐ) or MeSO₂SZ₁ (V_{b})
or 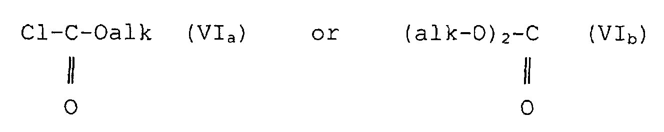 or 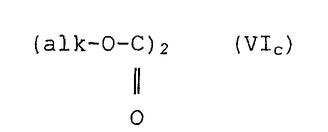 in which Z₁ represents an optionally substituted alkyl, alkenyl or phenyl radical, in which the optional reactive functions are optionally protected by protective groups, and alk represents an alkyl radical containing at most 4 carbon atoms, in order to obtain respectively the compound of formula (I₁): 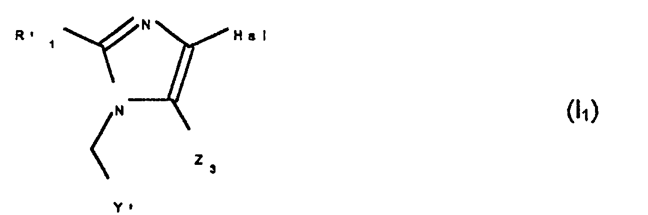 or the compound of formula (XIV): 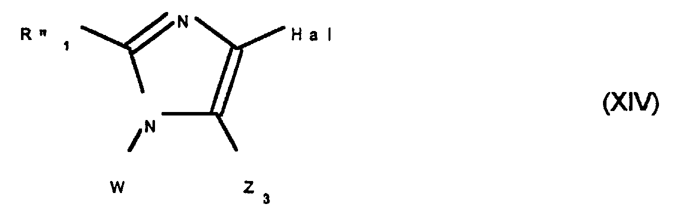 in which R'₁, R"₁, Hal, Y' and W have the meanings indicated above and Z₃ represents the carboxy, formyl, S-Z₁ radical as defined above, or the K-O-alk radical in which K represents the 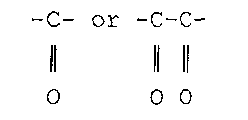 radical and alk has the meaning indicated above,
which compounds of formula (I₁) or (XIV) when Z₃ represents a formyl or esterified carboxy radical, can be subjected to a reaction with a compound of formula (VII):
Z₂-S-M (VII)
in which S represents a sulphur atom, M represents a metal such as sodium, potassium or copper and Z₂ represents an optionally substituted alkyl, alkenyl or phenyl radical, in which the optional reactive functions are optionally protected by protective groups in order to obtain respectively the compound of formula (I₂): 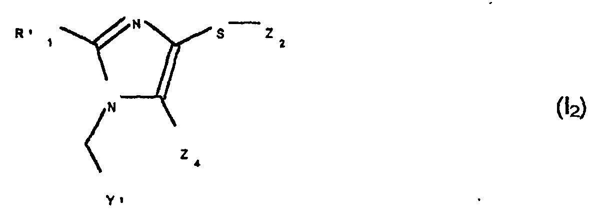 or the compound of formula (XV): 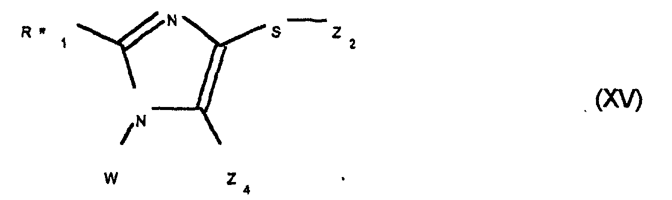 in which R'₁, R"₁, Y', Z₂ and W have the meanings indicated above, and Z₄ represents the formyl or esterified carboxy radical, which products of formulae (I₂) and (XV) when Z₄ represents the formyl radical, can be subjected to an oxidation or reduction reaction in order to obtain respectively a product of formula (I₃) : 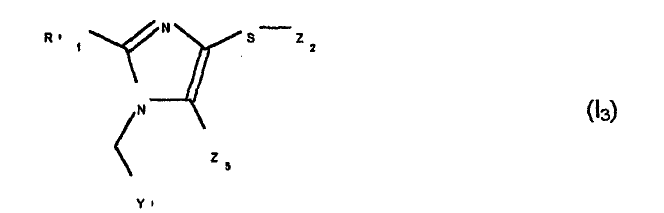 or a product of formula (I₄) : 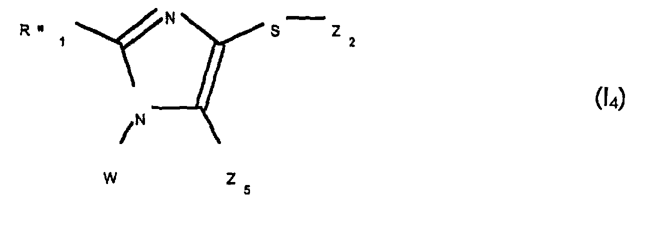 in which R'₁, R"₁, Z₂, Y' and W have the meanings indicated above, and Z₅ represents the CH₂OH radical or the carboxy radical free or esterified by a linear or branched alkyl radical containing at most 6 carbon atoms,
which products of formula (XV) or (I₄), in the case where W represents P as defined above and after release of the amine function blocked by P as defined above, is reacted with the compound of formula (III) as defined above, in order to obtain a product of formula (I₅) : 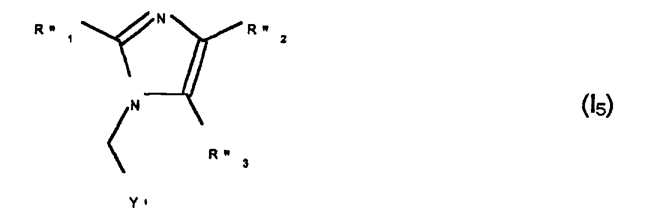 in which R"₁ and Y' have the meanings indicated above, and R"₂ and R"₃ indifferently represent either Z₄ or Z₅ as defined above or S-Z₂, as defined above,
or a compound of formula (XVI): 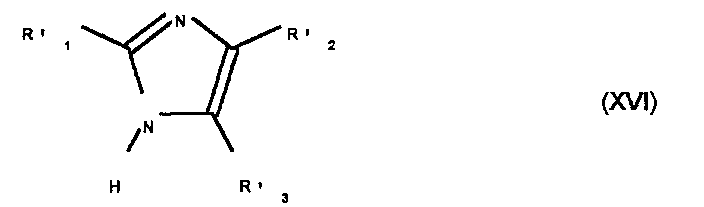 in which R'₁ has the meaning indicated above and R'₂ and R'₃ have the meanings indicated above respectively for R₂ and R₃ in which the optional reactive functions are optionally protected by protective groups, is subjected to a reaction with the compound of formula (III) as defined above, in order to obtain a product of formula (I') : 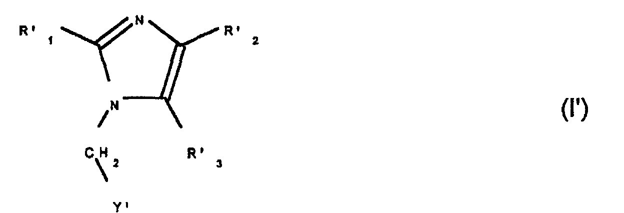 in which R'₁, R'₂, R'₃ and Y' have the definitions indicated above,
or a compound of formula (XVII): 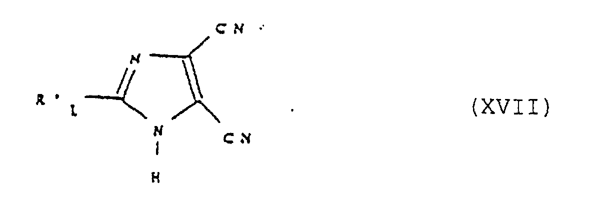 in which R'₁ has the meaning indicated previously, is subjected to an oxidation reaction in order to obtain the product of formula (XVIII): 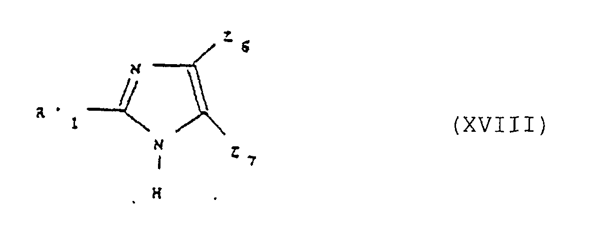 in which R'₁ has the meaning indicated previously and one or both of Z₆ and Z₇ represent a free or esterified carboxy radical and if appropriate, both Z₆ and Z₇ retaining the CN value, which product of formula (XVIII) is reacted with the compound of formula (III) as defined above in order to obtain the product of formula (I₆) : 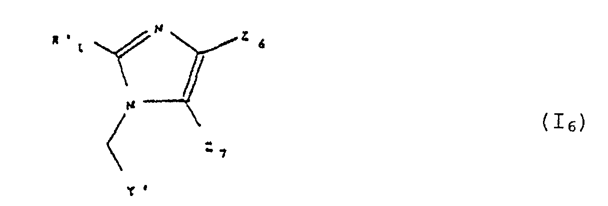 in which R'₁, Y', Z₆ and Z₇ have the meanings indicated previously,
which products of formulae (I₁), (I₂), (I₃), (I₄), (XIV), (XV), (I₅), (I₆) and (I') can be products as defined in claims 1 and 2 and which, in order to obtain the products or other products as defined in claims 1 and 2, can be subjected, if required and if necessary, to one or more of the following transformation reactions, in any order:
a) an esterification reaction of the acid function,
b) a saponification reaction of the ester function to an acid function,
c) a conversion reaction of the ester function to an acyl function,
d) a conversion reaction of the cyano function to an acid function,
e) a conversion reaction of the acid function to an amide function, then optionally to a thioamide function,
f) a reduction reaction of the carboxy function to an alcohol function,
g) a conversion reaction of the alkoxy function to a hydroxyl function, or also of the hydroxyl function to an alkoxy function,
h) an oxidation reaction of the alcohol function to an aldehyde, acid or ketone function,
i) a conversion reaction of the formyl radical to a carbamoyl radical,
j) a conversion reaction of the carbamoyl radical to a nitrile radical,
k) a conversion reaction of the nitrile radical to tetrazolyl,
l) an oxidation reaction of the alkylthio or arylthio group to a corresponding sulphoxide or sulphone,
m) a conversion reaction of the sulphide, sulphoxide or sulphone function to a corresponding sulphoximine function,
n) a conversion reaction of the oxo function to a thioxo function,
o) a conversion reaction of the 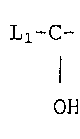 radical to an 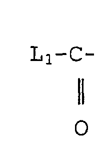 radical then if necessary again to an 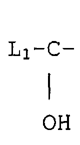 radical with L₁ as defined above,
p) a conversion reaction of the acid function to a 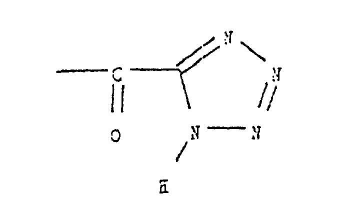 function
q) a conversion reaction of the β-keto-sulphoxide function to an α-keto thio ester function,
r) a conversion reaction of the carbamate to a urea and in particular of sulphonylcarbamate to a sulphonylurea,
s) a conversion reaction of the halogenated function to a formyl or esterified carboxy function,
t) a conversion reaction of the formyl radical to a CH₂-CO₂alk or CH=CH-CO₂alk function in which alk represents an alkyl radical containing from 1 to 4 carbon atoms, then if appropriate, conversion to a corresponding acid,
u) a conversion reaction of a formyl radical to a 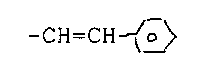 radical then optionally to a 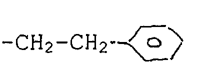 and 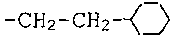 radical,
v) a conversion reaction of a formyl radical to a CH₂-OH or 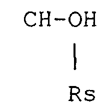 radical then if appropriate to a CH₂-Rs radical or conversion of the 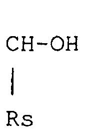 radical to 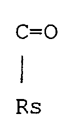 in order to obtain the optionally substituted alkyl or acyl radicals as defined above for R'₂ and R'₃,
w) an oxidation reaction of the 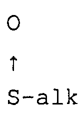 to an S-alk radical then conversion to an SH function and if appropriate to an S-Z₂ function in which alk and Z₂ have the meaning indicated previously,
x) an elimination reaction of the protective groups which can be carried by the protected reactive functions,
y) a salification reaction by a mineral or organic acid or by a base in order to obtain the corresponding salt,
z) a resolution reaction of the racemic forms to resolved products,
said products thus obtained being in all possible, racemic, enantiomeric and diastereoisomeric isomer forms.

7. Intermediate of formula (IV₁) : 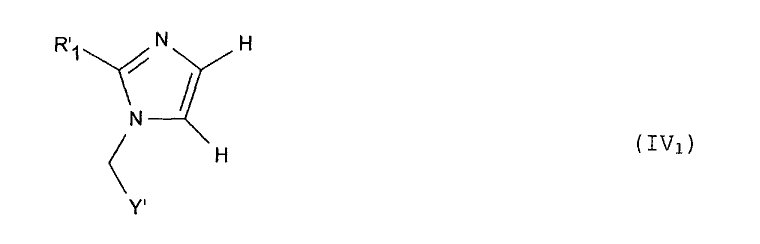 in which R'₁ and Y' have the meanings given for R₁ and Y in claim 1.

8. Intermediate of formula (IV₂): 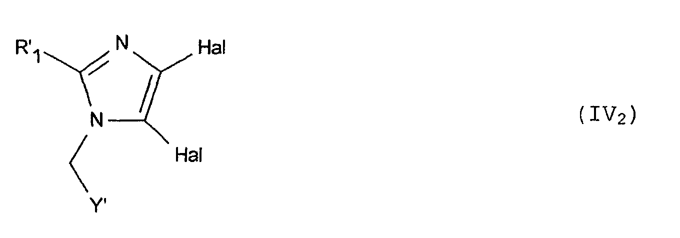 in which: R'₁ and Y' have the meanings given for R₁ and Y in claim 1, and Hal signifies halogen.

9. Intermediate of formula (XIII): 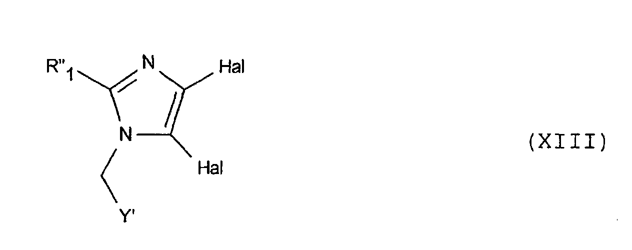 in which Y' has the meaning given for Y in claim 1, Hal signifies halogen and R"₁ represents an alkyl-carbonyl, alkyl-hydroxyalkyl or alkylthio radical, where the alkyl radical contains at most 3 carbon atoms.

10. Intermediate of formula (XIV): 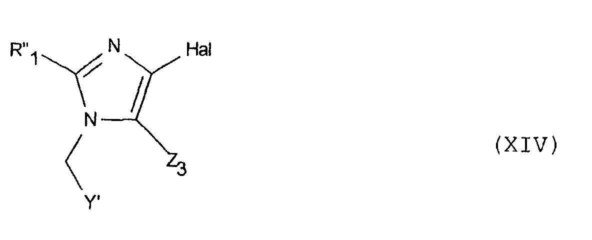 in which Y' has the meaning given for Y in claim 1, Hal signifies halogen, R"₁ represents an alkyl-carbonyl, alkyl-hydroxyalkyl or alkylthio radical, where the alkyl radical contains at most 3 carbon atoms, Z₃ signifies a carboxy, formyl, or the K-0-alk radical where alk represents an alkyl containing at most 4 carbon atoms and where K represents -CO- or -CO-CO-.

11. Intermediate of formula (XV): 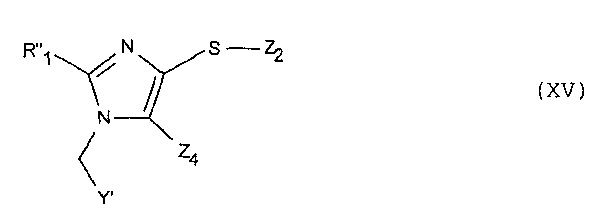 in which Y' has the meaning given for Y in claim 1, R"₁ represents an alkyl-carbonyl, alkyl-hydroxyalkyl or alkylthio radical, where the alkyl radical contains at most 3 carbon atoms, Z₄ represents the formyl or esterified carboxy radical; Z₂ represents an alkyl, alkenyl or phenyl radical, optionally substituted by one or more radicals chosen from the hydroxy, carboxy, alkyl, alkenyl and alkoxy radicals, these last three radicals being themselves optionally substituted by a carboxy radical and the phenyl radicals being in addition optionally substituted on two adjacent carbons by a dioxole radical.

12. Use of the products of formula (I) as defined in claim 1 for the preparation of pharmaceutical compositions intended for the treatment of illnesses resulting from an abnormal stimulation of the endothelin receptors.

## Patentansprüche

1. Produkte, die der Formel (I) entsprechen: 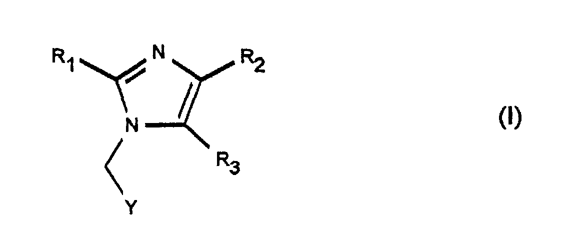 worin:
R₁ ausgewählt ist unter den linearen oder verzweigten Methyl-, Ethyl-, Propyl-, Butylresten, gegebenenfalls substituiert mit einem Hydroxy- oder Alkoxyrest, und den Resten Alkylthio mit bis zu 6 Kohlenstoffatomen, Dioxolan und Dioxan,
R₂ ausgewählt ist unter den Resten:
a) 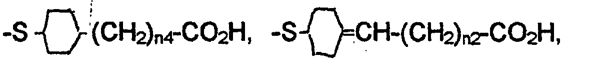 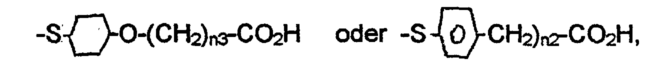 worin n₄ die Zahl 2 oder 3 darstellt, n₂ eine Zahl zwischen 0 und 3 darstellt und n₃ eine Zahl zwischen 1 und 3 darstellt,
b) den Resten Alkylthio, Alkenylthio, Cycloalkylthio, Cycloalkyl, Cycloalkylalkyl, Cycloalkylalkenyl, Phenyl, Phenoxy, Phenylthio, Benzyl, Benzoyl, Phenylthioalkyl, Reste, worin die Alkyl- und Alkenylreste höchstens 4 Kohlenstoffatome enthalten, die Cycloalkyl- und Cycloalkenylreste höchstens 6 Kohlenstoffatome enthalten und die Alkyl-, Alkenyl-, Cycloalkyl- und Phenylreste gegebenenfalls mit einem oder mehreren Resten substituiert sind, die ausgewählt sind unter den Resten Hydroxy, Carboxy, Alkyl, Alkenyl und Alkoxy, wobei diese drei letzten Reste selbst gegebenenfalls mit einem Carboxyrest substituiert sind und die Phenylreste außerdem gegebenenfalls an zwei benachbarten Kohlenstoffen mit einem Dioxolrest substituiert sind,
R₃ ausgewählt ist unter den freien oder veresterten Carboxyresten, Tetrazolyl oder Sulfonylharnstoff,
Y einen Phenylrest darstellt, der substituiert ist mit zwei Resten, die zusammen einen Dioxolrest bilden, und gegebenenfalls mit einem oder mehreren Resten, die ausgewählt sind unter den Halogenatomen und den Resten Hydroxy, Alkoxy und Carboxy,
wobei das Schwefelatom gegebenenfalls in Form von Sulfoxid oder Sulfon oxidiert ist,
sowie die folgenden Produkte:
- 2-Butyl-1-((6-chlor-1,3-benzodioxol-5-yl)methyl)-4-((4-methoxyphenyl)methylthio)-1H-imidazol-5-carbonsäure,
- 1-((6-Chlor-1,3-benzodioxol-5-yl)methyl)-2,4-bis-(((4-methoxyphenyl)methyl)thio)-1H-imidazol-5-carbonsäure,
- 1-((6-Chlor-1,3-benzodioxol-5-yl)methyl)-4-(2-phenylethyl)-2-propyl)-1H-imidazol-5-carbonsäure,
wobei besagte Produkte der Formel (I) und die oben definierten Produkte in allen möglichen racemischen, enantiomeren und diastereoisomeren Isomerformen vorliegen, sowie die Additionssalze besagter Produkte der Formel (I) und der oben definierten Produkte mit den mineralischen und organischen Säuren oder mit den mineralischen und organischen Basen.

2. Produkte, wie in Anspruch 1 definiert, die den folgenden Formeln entsprechen:
- 2-Butyl-1-((6-chlor-1,3-benzodioxol-5-yl)methyl)-4-((4-methoxyphenyl)methylthio)-1H-imidazol-5-carbonsäure,
- 2-Butyl-1-((6-chlor-1,3-benzodioxol-5-yl)methyl)-4-((4-methoxyphenyl)thio)-1Himidazol-5-carbonsäure,
- 1-((6-Chlor-1,3-benzodioxol-5-yl)methyl)-2,4-bis-(((4-methoxyphenyl)methyl)thio)-1H-imidazol-5-carbonsäure,
- 1-((6-Chlor-1,3-benzodioxol-5-yl)methyl)-4-(4-(methoxyphenyl)thio)-2-(propylthio)-1H-imidazol-5-carbonsäure,
- 1-((6-Chlor-1,3-benzodioxol-5-yl)methyl)-4-((3,4-dimethoxyphenyl)thio)-2-propyl-1H-imidazol-5-carbonsäure,
- 2-Butyl-1-((6-chlor-1,3-benzodioxol-5-yl)methyl)-4-(cyclohexylthio)-1H-imidazol-5-carbonsäure,
- 2-Butyl-4-((3-carboxypropyl)thio)-1-((6-chlor-1,3-benzodioxol-5-yl)methyl)-1Himidazol-5-carbonsäure,
- 4-(((4-(2-Carboxyethyl)phenyl)thio)methyl)-1-((6-chlor-1,3-benzodioxol-5-yl)-methyl)-2-propyl-1H-imidazol-5-carbonsäure,
- 4-((3-Carboxypropyl)thio)-1-((6-chlor-1,3-benzodioxol-5-yl)methyl)-2-propyl-1Himidazol-5-carbonsäure,
- 4-((7-Carboxyheptyl)thio)-1-((6-chlor-1,3-benzodioxol-5-yl)methyl)-2-propyl-1Himidazol-5-carbonsäure,
- 1-((6-Chlor-1,3-benzodioxol-5-yl)methyl)-4-(2-phenylethyl)-2-propyl-1H-imidazol-5-carbonsäure.

3. Als Medikamente die Produkte, wie in den Ansprüchen 1 und 2 definiert, sowie die Additionssalze dieser Produkte mit den pharmazeutisch annehmbaren mineralischen und organischen Säuren oder mit den pharmazeutisch annehmbaren mineralischen und organischen Basen.

4. Die pharmazeutischen Zusammensetzungen, die als Wirkstoff wenigstens eins der Medikamente, wie in Anspruch 3 definiert, enthalten.

5. Verwendung der Produkte der Formel (I), wie in den Ansprüchen 1 und 2 definiert, für die Herstellung von pharmazeutischen Zusammensetzungen, die zur Behandlung von Erkrankungen bestimmt sind, die aus einer anormalen Stimulierung der Endothelinrezeptoren resultieren, und insbesondere bestimmt sind zur Behandlung von Bluthochdruck, der von Endothelin bewirkt wird, aller Gefäßspasmen, zur Behandlung nach Gehirnblutungen, von Niereninsuffizienzen, des Myokardinfarkts, zur Vorbeugung von wiederkehrenden Verengungen nach Angioplastie.

6. Verfahren zur Herstellung der Produkte, wie in den Ansprüchen 1 und 2 definiert, **dadurch gekennzeichnet, dass**:
man entweder eine Verbindung der Formel (II): 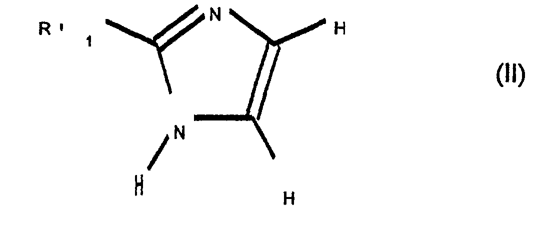 worin R'₁ die in Anspruch 1 für R₁ angegebene Bedeutung hat, worin die eventuellen reaktiven Funktionen gegebenenfalls durch Schutzgruppen geschützt sind,
einer Reaktion mit einer Verbindung der Formel (III): 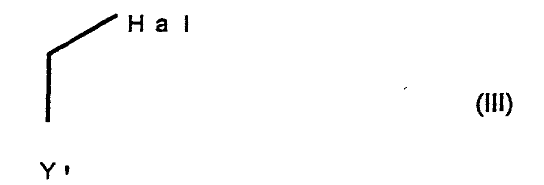 worin Hal ein Halogenatom darstellt und Y' die in Anspruch 1 für Y angegebene Bedeutung hat, worin die eventuellen reaktiven Funktionen gegebenenfalls durch Schutzgruppen geschützt sind,
unterzieht, um das Produkt der Formel (IV₁): 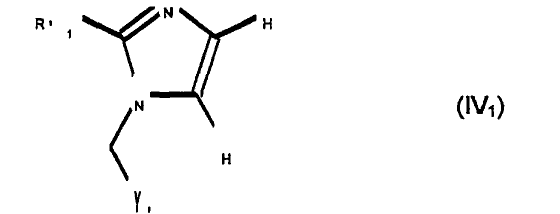 zu erhalten, worin R'₁ und Y' die oben angegebenen Bedeutungen haben,
Produkt der Formel (IV₁), das man einer Halogenierungsreaktion unterziehen kann, um das Produkt der Formel (IV₂): 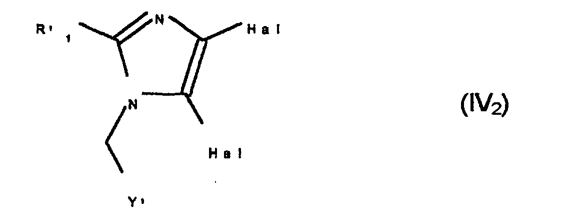 zu erhalten, worin R'₁, Hal und Y' die oben angegebenen Bedeutungen haben,
oder man eine Verbindung der Formel (VIII): 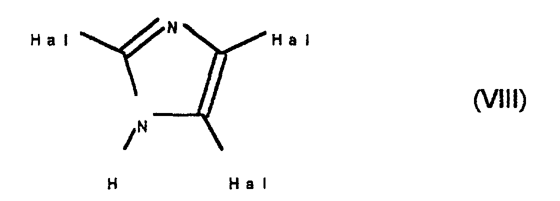 worin Hal die oben angegebene Bedeutung hat,
entweder einer Reaktion mit der Verbindung der Formel (III), wie oben definiert, oder der Einwirkung einer Schutzgruppe P unterzieht, um ein Produkt der Formel (IX): 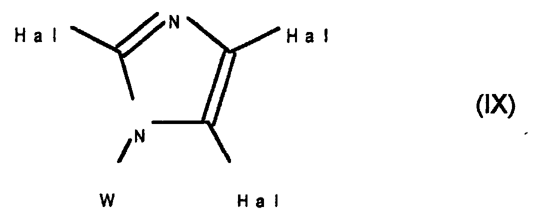 zu erhalten, worin Hal die oben angegebene Bedeutung hat und W entweder -CH₂-Y' mit Y', wie oben definiert, oder P darstellt, das eine Schutzgruppe des Stickstoffatoms darstellt,
Produkt der Formel (IX), das man einer Reaktion zum Austausch Halogen-Metall, dann einer Reaktion mit einem Elektrophil der Formel (X), (X'), (XI), (XII) oder (Xll'):
L₁-CHO (X)
L₁-CHO-CI (X')
(-S-L₁)₂ (XI)
L₁SO₂-S-L'₁ (XII)
L₁SO₂-Cl (XII')
worin L₁ und L'₁, die gleich oder verschieden sind, einen Alkyl- oder Alkenylrest, wie oben definiert, darstellen, unterzieht, um ein Produkt der Formel (XIII): 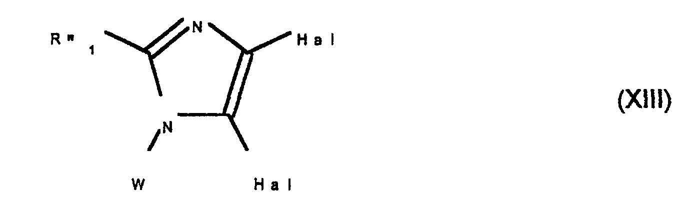 zu erhalten, worin Hal und W die oben angegebenen Bedeutungen haben, R"₁ einen Rest Alkyl-Carbonyl, Alkyl-Hydroxyalkyl oder Alkylthio, worin der Alkylrest die oben angegebene Bedeutung hat, darstellt und worin die eventuellen reaktiven Funktionen gegebenenfalls durch Schutzgruppen geschützt sind,
Produkte der Formeln (IV₂) und (XIII), die man einer Reaktion zum Austausch Halogen-Metall an einem der Halogenatome, dann einer Reaktion mit CO₂ oder DMF oder einer elektrophilen Verbindung der Formel (Vₐ), (V_{b}), (VIₐ), (Vl_{b}) oder (Vl_{c}):
(-S-Z₁)₂ (Vₐ) oder MeSO₂SZ₁ (V_{b})
oder 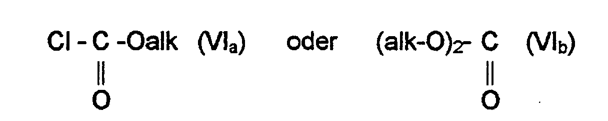 oder 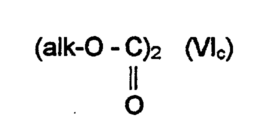 worin Z₁ einen Alkyl-, Alkenyl- oder Phenylrest darstellt, gegebenenfalls substituiert, worin die eventuellen reaktiven Funktionen gegebenenfalls durch Schutzgruppen geschützt sind, und alk einen Alkylrest mit höchstens 4 Kohlenstoffatomen darstellt,
unterziehen kann, um die Verbindung der Formel (I₁): 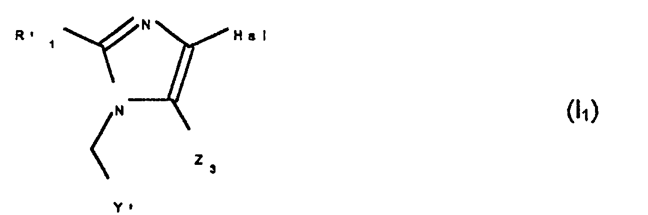 beziehungsweise die Verbindung der Formel (XIV): 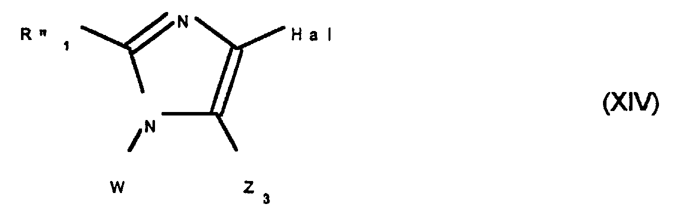 zu erhalten, worin R'₁, R"₁, Hal, Y' und W die oben angegebenen Bedeutungen haben und Z₃ den Rest Carboxy, Formyl, S-Z₁, wie oben definiert, oder den Rest K-O-alk darstellt, worin K den Rest 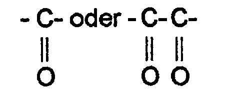 darstellt und alk die oben angegebene Bedeutung hat,
Verbindungen der Formel (I₁) oder (XIV), die man, wenn Z₃ einen Formyl- oder veresterten Carboxyrest darstellt, einer Reaktion mit einer Verbindung der Formel (VII):
Z₂-S-M (VII)
worin S ein Schwefelatom darstellt, M ein Metall, wie Natrium, Kalium oder Kupfer darstellt und Z₂ einen gegebenenfalls substituierten Alkyl-, Alkenyl- oder Phenylrest darstellt, worin die eventuellen reaktiven Funktionen gegebenenfalls durch Schutzgruppen geschützt sind, unterziehen kann, um die Verbindung der Formel (I₂): 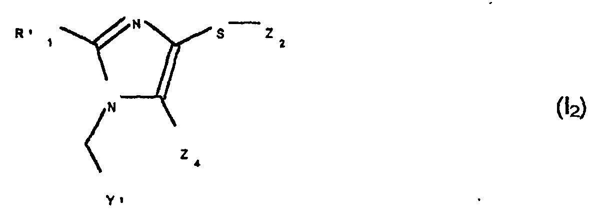 beziehungsweise die Verbindung der Formel (XV): 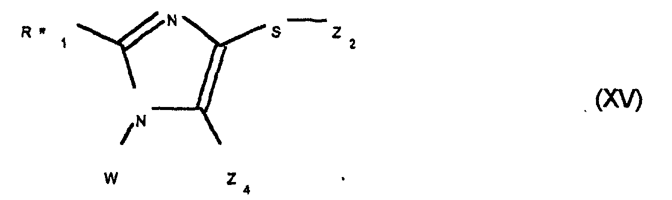 zu erhalten, worin R'₁, R"₁, Y', Z₂ und W die oben angegebenen Bedeutungen haben und Z₄ den Formyl- oder veresterten Carboxyrest darstellt,
Produkte der Formeln (I₂) und (XV), die man, wenn Z₄ den Formylrest darstellt, einer Oxidations- oder Reduktionsreaktion unterziehen kann, um ein Produkt der Formel (I₃): 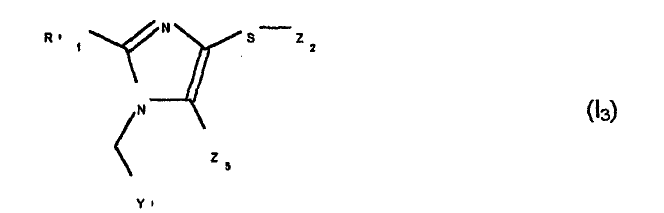 beziehungsweise ein Produkt der Formel (I₄): 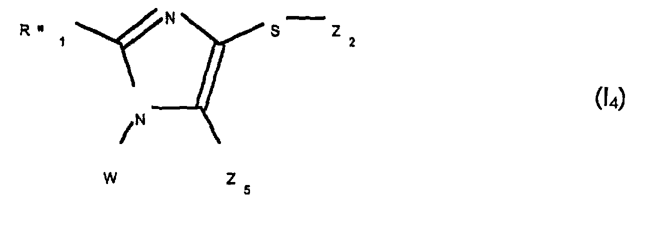 zu erhalten, worin R'₁, R"₁, Z₂, Y' und W die oben angegebenen Bedeutungen haben und Z₅ den Rest CH₂OH oder den freien oder mit einem linearen oder verzweigten Alkylrest mit höchstens 6 Kohlenstoffatomen veresterten Carboxyrest darstellt,
Produkte der Formel (XV) oder (I₄), die man in dem Fall, wo W P, wie oben definiert, darstellt und nach Freisetzung der durch P, wie oben definiert, blockierten Aminfunktion, mit der Verbindung der Formel (III), wie oben definiert, umsetzt, um ein Produkt der Formel (I₅): 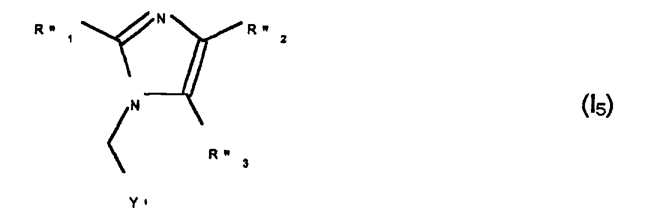 zu erhalten, worin R"₁ und Y' die oben angegebenen Bedeutungen haben und von R"₂ und R"₃ gleichermaßen das eine Z₄ oder Z₅, wie oben definiert, und das andere S-Z₂, wie oben definiert, darstellt,
oder man eine Verbindung der Formel (XVI): 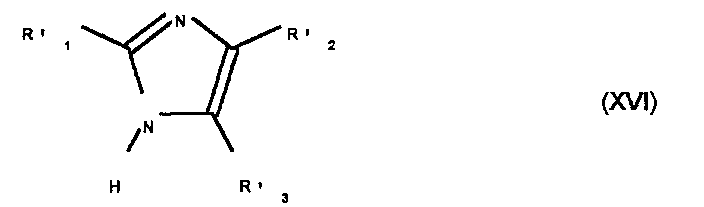 worin R'₁ die oben angegebene Bedeutung hat und R'₂ und R'₃ die oben für R₂ beziehungsweise R₃ angegebenen Bedeutungen haben, worin die eventuellen reaktiven Funktionen gegebenenfalls durch Schutzgruppen geschützt sind, einer Reaktion mit der Verbindung der Formel (III), wie oben definiert, unterzieht, um ein Produkt der Formel (I'): 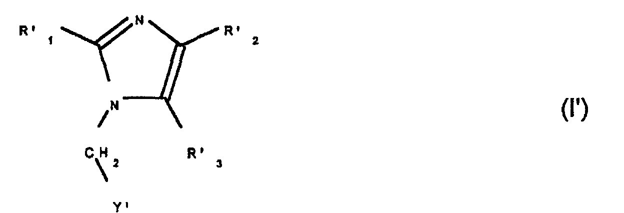 zu erhalten, worin R'₁, R'₂, R'₃ und Y' die oben angegebenen Bedeutungen haben,
oder man eine Verbindung der Formel (XVII): 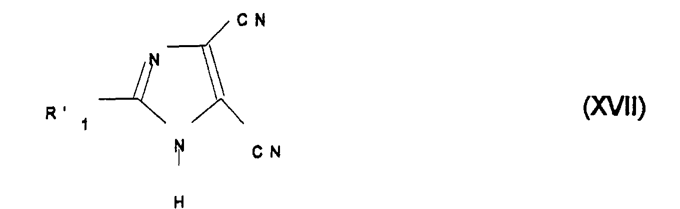 worin R'₁ die zuvor angegebene Bedeutung hat, einer Oxidationsreaktion unterzieht, um das Produkt der Formel (XVIII): 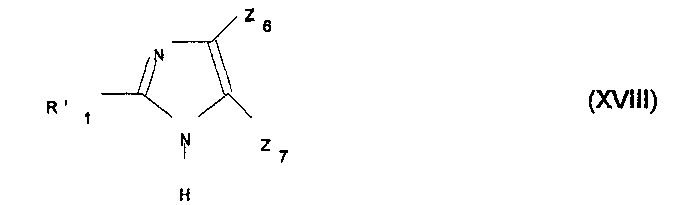 zu erhalten, worin R'₁ die zuvor angegebene Bedeutung hat und eins oder beide von Z₆ und Z₇ einen freien oder veresterten Carboxyrest darstellen und wobei gegebenenfalls das andere von Z₆ und Z₇ den Wert CN behält,
Produkt der Formel (XVIII), das man mit der Verbindung der Formel (III), wie oben definiert, umsetzt, um das Produkt der Formel (I₆): 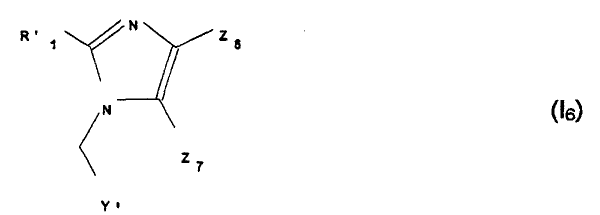 zu erhalten, worin R'₁, Y', Z₆ und Z₇ die zuvor angegebenen Bedeutungen haben,
Produkte der Formeln (I₁), (I₂), (I₃), (I₄), (XIV), (XV), (I₅), (I₆) und (I'), die Produkte, wie in den Ansprüchen 1 und 2 definiert, sein können und die man, um Produkte oder andere Produkte, wie in den Ansprüchen 1 und 2 definiert, zu erhalten, wenn gewünscht und wenn notwendig, einer oder mehreren der folgenden Umwandlungsreaktionen in einer beliebigen Reihenfolge unterziehen kann:
a) einer Reaktion zur Veresterung einer Säurefunktion,
b) einer Reaktion zur Verseifung einer Esterfunktion zu einer Säurefunktion,
c) einer Reaktion zur Umwandlung einer Esterfunktion in eine Acylfunktion,
d) einer Reaktion zur Umwandlung der Cyanofunktion in eine Säurefunktion,
e) einer Reaktion zur Umwandlung einer Säurefunktion in eine Amidfunktion, dann gegebenenfalls in eine Thioamidfunktion,
f) einer Reaktion zur Reduktion der Carboxyfunktion in eine Alkoholfunktion,
g) einer Reaktion zur Umwandlung einer Alkoxyfunktion in eine Hydroxylfunktion oder auch einer Hydroxylfunktion in eine Alkoxyfunktion,
h) einer Reaktion zur Oxidation einer Alkoholfunktion in eine Aldehyd-, Säure- oder Ketonfunktion,
i) einer Reaktion zur Umwandlung des Formylrests in einen Carbamoylrest,
j) einer Reaktion zur Umwandlung des Carbamoylrests in einen Nitrilrest,
k) einer Reaktion zur Umwandlung eines Nitrilrests in Tetrazolyl,
l) einer Reaktion zur Oxidation einer Alkylthio- oder Arylthiogruppe in das entsprechende Sulfoxid oder Sulfon,
m) einer Reaktion zur Umwandlung einer Sulfid-, Sulfoxid- oder Sulfonfunktion in die entsprechende Sulfoximinfunktion,
n) einer Reaktion zur Umwandlung einer Oxofunktion in eine Thioxofunktion,
o) einer Reaktion zur Umwandlung des Rests 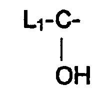 in einen Rest 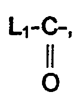 dann, wenn notwendig, von neuem in einen Rest 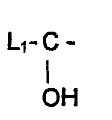 mit L₁, wie oben definiert,
p) einer Reaktion zur Umwandlung einer Säurefunktion in eine Funktion 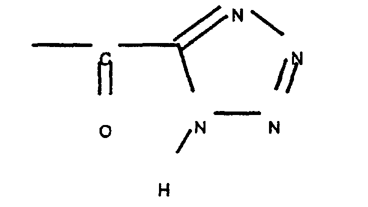
q) einer Reaktion zur Umwandlung der β-Keto-sutfoxid-Funktion in eine α-Ketothioester-Funktion,
r) einer Reaktion zur Umwandlung eines Carbamats in Harnstoff und insbesondere eines Sulfonylcarbamats in Sulfonylharnstoff,
s) einer Reaktion zur Umwandlung einer Halogenfunktion in eine Formyl- oder veresterte Carboxyfunktion,
t) einer Reaktion zur Umwandlung eines Formylrests in eine Funktion CH₂-CO₂alk oder CH=CH-CO₂alk, worin alk einen Alkylrest mit 1 bis 4 Kohlenstoffatomen darstellt, dann gegebenenfalls Umwandlung in die entsprechende Säure,
u) einer Reaktion zur Umwandlung eines Formylrests in einen Rest 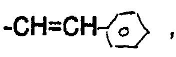 dann gegebenenfalls in 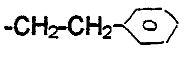 und 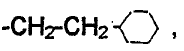
v) einer Reaktion zur Umwandlung eines Formylrests in einen Rest CH₂-OH oder 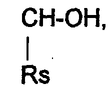 dann gegebenenfalls in CH₂-Rs oder
Umwandlung von 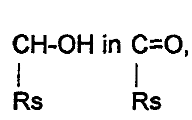 um die Alkyl- oder Acylreste, die gegebenenfalls substituiert sind, wie oben für R'₂ und R'₃ definiert, zu erhalten,
w) einer Reaktion zur Oxidation des Rests S-alk zu 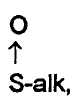 dann Umwandlung in eine SH-Funktion und gegebenenfalls in S-Z₂, worin alk und Z₂ die zuvor angegebene Bedeutung haben,
x) einer Reaktion zur Entfernung der Schutzgruppen, die die geschützten reaktiven Funktionen tragen können,
y) einer Reaktion zur Salzbildung durch eine mineralische oder organische Säure oder durch eine Base, um das entsprechende Salz zu erhalten,
z) einer Reaktion zur Spaltung der racemischen Formen in aufgetrennte Produkte,
wobei besagte so erhaltene Produkte in allen möglichen racemischen, enantiomeren und diastereoisomeren Isomerformen vorliegen.

7. Zwischenprodukt der Formel (IV₁): 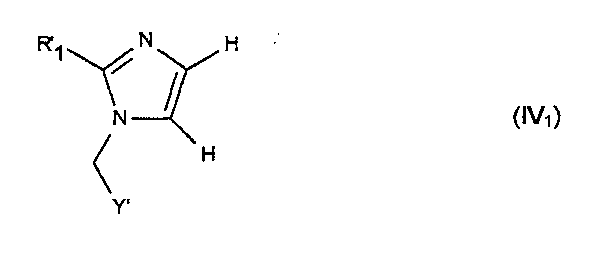 worin R'₁ und Y' die für R₁ und Y in Anspruch 1 angegebenen Bedeutungen haben.

8. Zwischenprodukt der Formel (IV₂): 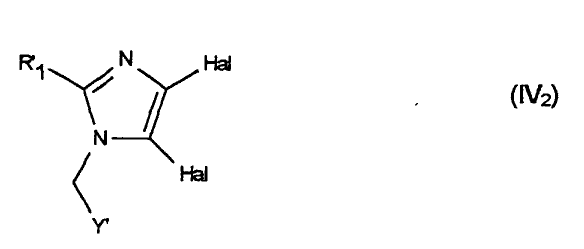 worin R'₁ und Y' die für R₁ und Y in Anspruch 1 angegebenen Bedeutungen haben und Hal Halogen bedeutet.

9. Zwischenprodukt der Formel (Xlll): 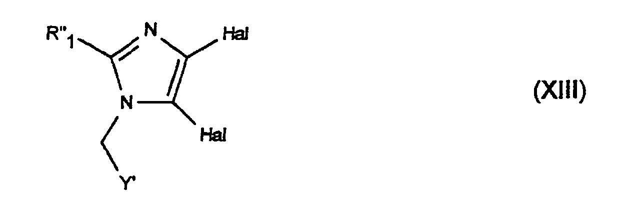 worin Y' die für Y in Anspruch 1 angegebene Bedeutung hat, Hal Halogen bedeutet und R"₁ einen Rest Alkyl-Carbonyl, Alkyl-Hydroxyalkyl oder Alkylthio, worin der Alkylrest höchstens 3 Kohlenstoffatome enthält, darstellt.

10. Zwischenprodukt der Formel (XIV): 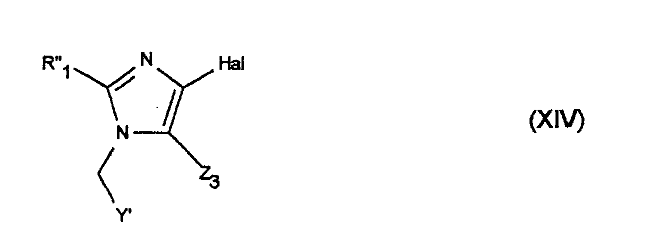 worin Y' die für Y in Anspruch 1 angegebene Bedeutung hat, Hal Halogen bedeutet, R"₁ einen Rest Alkyl-Carbonyl, Alkyl-Hydroxyalkyl oder Alkylthio, worin der Alkylrest höchstens 3 Kohlenstoffatome enthält, darstellt, Z₃ einen Rest Carboxy, Formyl oder den Rest K-O-alk, worin alk ein Alkyl mit höchstens 4 Kohlenstoffatomen darstellt und worin K -CO- oder -CO-CO- darstellt, bedeutet.

11. Zwischenprodukt der Formel (XV): 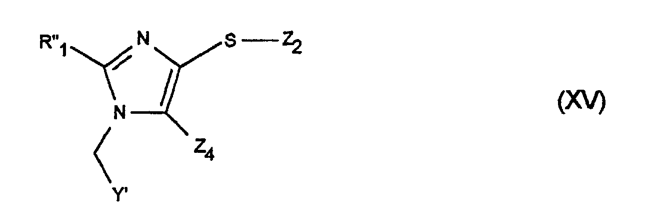 worin Y' die für Y in Anspruch 1 angegebene Bedeutung hat, R"₁ einen Rest Atkyl-Carbonyl, Alkyl-Hydroxyalkyl oder Alkylthio, worin der Alkylrest höchstens 3 Kohlenstoffatome enthält, darstellt, Z₄ den Formyl- oder veresterten Carboxyrest darstellt; Z₂ einen Alkyl-, Alkenyl- oder Phenylrest darstellt, gegebenenfalls substituiert mit einem oder mehreren Resten, die ausgewählt sind unter den Resten Hydroxy, Carboxy, Alkyl, Alkenyl und Alkoxy, wobei diese drei letzten Reste selbst gegebenenfalls mit einem Carboxyrest substituiert sind und die Phenylreste außerdem gegebenenfalls an zwei benachbarten Kohlenstoffen mit einem Dioxolrest substituiert sind.

12. Verwendung der Produkte der Formel (I), wie in Anspruch 1 definiert, für die Herstellung von pharmazeutischen Zusammensetzungen, die zur Behandlung von Erkrankungen bestimmt sind, die aus einer anormalen Stimulierung der Endothelinrezeptoren resultieren.
